(19) Europäisches Patentamt
European Patent Office
Office européen des brevets

(11) EP 4 316 527 A1

(12) **EUROPEAN PATENT APPLICATION**
published in accordance with Art. 153(4) EPC

(43) Date of publication:
**07.02.2024 Bulletin 2024/06**

(21) Application number: **22781285.6**

(22) Date of filing: **31.03.2022**

(51) International Patent Classification (IPC):
***A61K 47/68*** *(2017.01)* ***A61K 39/395*** *(2006.01)*
***A61P 35/00*** *(2006.01)* ***C12N 9/24*** *(2006.01)*
***C12N 15/56*** *(2006.01)* ***C12P 19/44*** *(2006.01)*
***C07K 16/30*** *(2006.01)*

(52) Cooperative Patent Classification (CPC):
**A61K 39/395; A61K 47/68; A61P 35/00; C12N 9/24; C12P 19/44;** C07K 16/30

(86) International application number:
**PCT/JP2022/016786**

(87) International publication number:
**WO 2022/211075 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2021 JP 2021061693**

(71) Applicant: **MicroBiopharm Japan Co., Ltd.**
**Chuo-ku**
**Tokyo 104-0031 (JP)**

(72) Inventors:
- **HIBINO Kazuhiro**
  **Tokyo 104-0031 (JP)**
- **NAGAHARA Takashi**
  **Tokyo 104-0031 (JP)**
- **KANAMORI Satoshi**
  **Tokyo 104-0031 (JP)**
- **BABA Yutaro**
  **Tokyo 104-0031 (JP)**
- **NARUMI Hideki**
  **Tokyo 104-0031 (JP)**
- **OHARA Keiichiro**
  **Tokyo 104-0031 (JP)**
- **NISHIMURA Yutaka**
  **Tokyo 104-0031 (JP)**
- **KAWASAKI Mayu**
  **Shizuoka-shi, Shizuoka 422-8526 (JP)**
- **NAKANO Shogo**
  **Shizuoka-shi, Shizuoka 422-8526 (JP)**
- **ITO Sohei**
  **Shizuoka-shi, Shizuoka 422-8526 (JP)**

(74) Representative: **Vossius & Partner**
**Patentanwälte Rechtsanwälte mbB**
**Siebertstraße 3**
**81675 München (DE)**

# (54) METHOD FOR MANUFACTURING ANTIBODY-DRUG CONJUGATE AND ENZYME USED FOR SAME

(57) A simpler ADC production method is provided. A method for producing an antibody-drug conjugate comprising the following step: the step of conjugating a glycan-homogenized antibody and a glycosylated drug, wherein the glycosylated drug consists of a drug bound to a glycan having an oxazolinated reducing end via a linker. The glycan-homogenized antibody and the glycosylated drug can be conjugated with an endo-β-N-acetylglucosaminidase mutant enzyme.

[Fig.4]

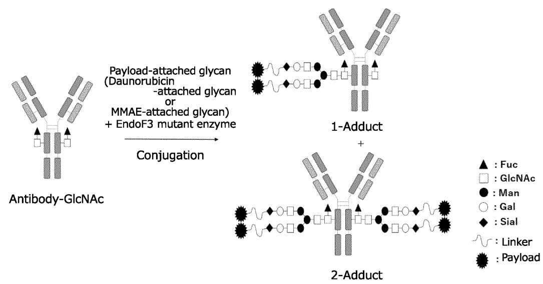


EP 4 316 527 A1

## Description

Technical Field

The present invention relates to a method for producing an antibody-drug conjugate. The present invention also relates to a mutant enzyme of endo-β-N-acetylglucosaminidase used in the production method.

Background Art

Antibody drug conjugates (henceforth referred to as ADCs) are drugs conjugated to antibodies that specifically bind to a target. For example, an ADC consisting of an antibody targeting cancer cells conjugated with a payload having potent cytotoxic activity can selectively deliver a drug (payload) to cancer cells, and thereby effectively kill cancer cells with greatly reduced systemic toxicity as a side effect compared with the use of the payload alone. A typical ADC is T-DM1 (Kadcyla (registered trademark)), which is the anti-HER2 antibody trastuzumab conjugated with a tubulin inhibitor, and it is approved as a drug (Non-patent documents 1 to 3). Concerning T-DM1, payloads are randomly bound to Lys residues on the antibody, and therefore it is produced as a mixture of conjugates heterogeneous for binding positions and binding numbers of the payloads on the antibody. Similar ADCs include gemtuzumab ozogamicin (generic name Mylotarg, Non-patent document 4), brentuximab vedotin (generic name Adcetris, Non-patent document 5), and so forth.

Recently, it has been reported that the homogeneity of ADCs affects pharmacokinetics, drug release rate, and drug efficacy (Non-patent document 6). In addition, from the viewpoint of ADC quality control, regioselective payload binding methods have been actively developed to produce ADCs with high homogeneity. One of such methods is a method for binding payloads to antibody glycans using an enzyme. The ADC production processes using this method reported so far comprise: (1) homogenization of glycans by enzymatic cleavage of heterogeneous antibody glycans (hydrolysis step), (2) enzymatic binding of glycan-homogenized antibodies and another glycan (glycosyltransfer step), and (3) binding of azide group on the other glycan and a payload (chemical reaction step).

In the above method, a family of enzymes called endo-β-N-acetylglucosaminidases is used in the hydrolysis and glycosyltransfer steps in order to convert glycans (Non-patent document 7). Endo-β-N-acetylglucosaminidases are isolated from a variety of species, and are used in different ways depending on the type of antibody glycan. Typical endo-β-N-acetylglucosaminidases used in the hydrolysis step include EndoA, EndoD, EndoM, EndoH, EndoF2, EndoF3, EndoE, EndoS, EndoS2, and so forth.

It has been reported that, among these, hydrolysis abilities of EndoS D233Q, i.e., EndoS in which the 233rd Asp is replaced with Gin (Patent document 1 and Non-patent document 8), EndoS2 D184M or EndoS2 D184Q, i.e., EndoS in which the 184th Asp is replaced with Met or Gln (Patent document 2 and Non-patent document 9), and EndoF3 D165A or EndoF3 D165Q, i.e., EndoF3 in which the 165th Asp is replaced with Ala or Gln (Patent document 3 and Non-patent document 10), are suppressed. It has also been reported that these mutant enzymes promote glycosyltransfer between glycan-homogenized antibodies (GlcNAc antibodies) and a substrate with oxazolinated glycan reducing end.

Furthermore, it has been reported that a homogeneous ADC was produced by transferring a substrate glycan having oxazolinated reducing end and azide group to a GlcNAc antibody using EndoS D233Q, and subsequent click reaction of the azide group of the resulting conjugate with MMAE, which is one type of payload (Non-patent documents 11 and 12).

Prior Art References

Patent documents

Patent document 1: WO2013/120066A1
Patent document 2: WO2017/124084A1
Patent document 3: US2012/0226024A1


Non-patent documents

Non-patent document 1: Nat. Biotechnol., 23, 1073-1078 (2005)
Non-patent document 2: Cancer Sci., 100, 1566-1572 (2009)
Non-patent document 3: Nat. Biotechnol., 23, 1137-1146 (2005)

Non-patent document 4: Clin. Cancer Res., 7, 1490-1496 (2001)
Non-patent document 5: MAbs, 4, 458-465 (2012)
Non-patent document 6: Nat. Biotechnol., 26, 952-932 (2008)
Non-patent document 7: Chem. Soc. Rev., 46, 5128-5146 (2017)
Non-patent document 8: J. Am. Chem. Soc., 134, 12308-12318 (2012)
Non-patent document 9: J. Biol. Chem., 291, 16508-16518 (2016)
Non-patent document 10: J. Biol. Chem., 291, 9356-9370 (2016)
Non-patent document 11: Nat. Protoc., 6, 1183-1191 (2011)
Non-patent document 12: Bioconjug. Chem., 30, 1343-1355 (2019)

## Summary of the Invention

### Object to be achieved by the invention

The ADC production processes using sugar chain-homogenized antibody and enzyme reported so far require three steps: the steps of hydrolysis of antibody glycan, glycosyltransfer to glycan-homogenized antibody, and linkage of payload to antibody-glycan. If ADC purification in each step is also taken into consideration, increase in ADC production cost and decrease in the performance and function of the antibody due to purification are anticipated, and therefore a simpler production method is desired.

In general, enzymatic reactions are substrate-specific. Therefore, if it is attempted to conjugate a substrate glycan modified with a payload with a glycan-homogenized antibody in a single step using a glycosyltransferase, a significant decrease in the reaction efficiency of the enzyme is expected. In fact, when the inventors of the present invention attempted to conjugate peptide- or drug-modified glycans to antibodies with various glycosyltransferases, the reaction efficiency was significantly reduced compared with that for linking simple glycans to antibodies.

An object of the present invention is to provide an enzyme that can efficiently conjugate a glycan modified with a payload to a glycan-homogenized antibody, and to provide a novel production method of ADC that allows such conjugation of an antibody and a payload, which conventionally required two steps, in a single step by using such an enzyme.

### Means for achieving the object

The present invention provides the followings.

[1] A method for producing an antibody-drug conjugate comprising the following step:
the step of conjugating a glycan-homogenized antibody and a glycosylated drug, wherein:
the glycosylated drug consists of a drug bound to a glycan having an oxazolinated reducing end via a linker.
[2] The production method according to 1, wherein the glycan-homogenized antibody and the glycosylated drug are conjugated by using an endo-β-N-acetylglucosaminidase mutant enzyme.
[3] The production method according to 2, wherein the endo-β-N-acetylglucosaminidase mutant enzyme is any one of the following proteins:

(1) a protein consisting of the amino acid sequence of SEQ ID NO: 1, or the amino acid sequence of SEQ ID NO: 1 in which the 165th amino acid is replaced with an amino acid other than aspartic acid (D);
(2) a protein consisting of an amino acid sequence derived from the amino acid sequence of the protein described in (1) by substitution, deletion, insertion, and/or addition of one or more amino acids, provided that the 165th amino acid is an amino acid other than D, and having a conjugation activity;
(3) a protein consisting of an amino acid sequence having an identity of at least 90% to the amino acid sequence of the protein described in (1), provided that the 165th amino acid is an amino acid other than D, and having a conjugation activity;
(4) a protein consisting of the amino acid sequence of SEQ ID NO: 11, or the amino acid sequence of SEQ ID NO: 11 in which the 233rd amino acid is replaced with an amino acid other than D;
(5) a protein consisting of an amino acid sequence derived from the amino acid sequence of the protein described in (4) by substitution, deletion, insertion, and/or addition of one or more amino acids, provided that the 233rd amino acid is an amino acid other than D, and having a conjugation activity;
(6) a protein consisting of an amino acid sequence having an identity of at least 90% to the amino acid sequence of the protein described in (4), provided that the 233rd amino acid is an amino acid other than D, and having a conjugation activity; and
(7) a protein consisting of the amino acid sequence of SEQ ID NO: 24, or the amino acid sequence of SEQ ID

NO: 24 in which the 165th amino acid is replaced with an amino acid other than D.

[4] The production method according to any one of 1 to 3, wherein the linker contains a structure that can be cleaved in vivo.
[5] The production method according to any one of 1 to 4, wherein the linker has a structure containing a peptide.
[6] The production method according to any one of 1 to 5, wherein the linker has a structure represented by the general formula -E-D-C-B-A-, A binds to the glycan, and E binds to the drug.
[in the formula,

A, B, C, and E are each independently selected from the group consisting of an alkyl group, ester group, carbamoyl group, alkoxyalkyl group, imine group, hydrazone group, azo group, sulfone group, aromatic group, and single bond; and
D is a peptide consisting of 2 to 6 amino acid residues].

[7] The production method according to 6, wherein:

A is $-(CH_2CH_2O)_{m1}-(CH_2)_{m2}-$, $-(CH_2)_{m2}-$, or a single bond;
B is selected from the group consisting of

[Formula 1]

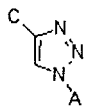
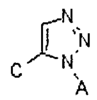
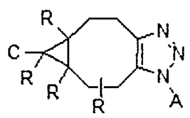
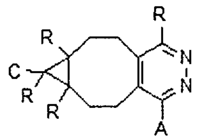
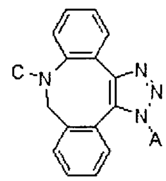
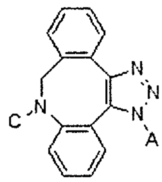

[in the formula, R is independently hydrogen atom, alkyl having 1 to 4 carbon atoms, alkoxy having 1 to 4 carbon atoms, -CN, -OH, $-CF_3$, or NRR];
C is $-C(O)-(CH_2)_{n1}-C(O)-$, $-C(O)-(CH_2)_{n1}-C(O)-NH-(CH_2CH_2O)_{n2}-(CH_2)_{n3}-C(O)-$, $-C(O)-(CH_2)_{n1}-C(O)-NH-(CH_2)_{n3}-C(O)-$, or a single bond,
E is -aminobenzylalcohol-C(O)-, $NH-(CH_2)_o-$, or a single bond,
m1 is an integer of from 1 to 12
m2 is an integer of from 1 to 6,
n1 is an integer of from 1 to 6,
n2 is an integer of from 1 to 12,
n3 is an integer of from 1 to 6, and
o is an integer of from 1 to 6].

[8] The production method according to 7, wherein B is either of

[Formula 2]

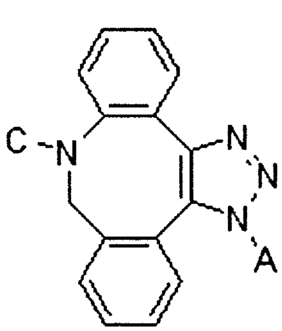
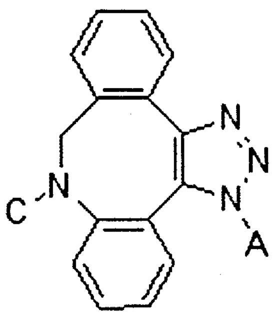

[9] The production method according to any one of 1 to 8, wherein the drug is any selected from the group consisting

of anthracyclines, auristatins, maytansines, camptothecins, pyrrolobenzodiazepine dimers, calicheamicins, and duocarmycins.

[10] An enzyme agent for use in a reaction of a glycan-homogenized antibody with a glycosylated drug, which contains any one of the following proteins:

(1) a protein consisting of the amino acid sequence of SEQ ID NO: 1, or the amino acid sequence of SEQ ID NO: 1 in which the 165th amino acid is replaced with an amino acid other than aspartic acid (D);
(2) a protein consisting of an amino acid sequence derived from the amino acid sequence of the protein described in (1) by substitution, deletion, insertion, and/or addition of one or more amino acids, provided that the 165th amino acid is an amino acid other than D, and having a conjugation activity;
(3) a protein consisting of an amino acid sequence having an identity of at least 90% to the amino acid sequence of the protein described in (1), provided that the 165th amino acid is an amino acid other than D, and having a conjugation activity;
(4) a protein consisting of the amino acid sequence of SEQ ID NO: 11, or the amino acid sequence of SEQ ID NO: 11 in which the 233rd amino acid is replaced with an amino acid other than D;
(5) a protein consisting of an amino acid sequence derived from the amino acid sequence of the protein described in (4) by substitution, deletion, insertion, and/or addition of one or more amino acids, provided that the 233rd amino acid is an amino acid other than D, and having a conjugation activity;
(6) a protein consisting of an amino acid sequence having an identity of at least 90% to the amino acid sequence of the protein described in (4), provided that the 233rd amino acid is an amino acid other than D, and having a conjugation activity; and
(7) a protein consisting of the amino acid sequence of SEQ ID NO: 24, or the amino acid sequence of SEQ ID NO: 24 in which the 165th amino acid is replaced with an amino acid other than D.

[11] Use of a polynucleotide encoding any one of the following proteins in the production of an enzyme for use in a reaction of a glycan-homogenized antibody with a glycosylated drug:

(1) a protein consisting of the amino acid sequence of SEQ ID NO: 1, or the amino acid sequence of SEQ ID NO: 1 in which the 165th amino acid is replaced with an amino acid other than aspartic acid (D);
(2) a protein consisting of an amino acid sequence derived from the amino acid sequence of the protein described in (1) by substitution, deletion, insertion, and/or addition of one or more amino acids, provided that the 165th amino acid is an amino acid other than D, and having a conjugation activity;
(3) a protein consisting of an amino acid sequence having an identity of at least 90% to the amino acid sequence of the protein described in (1), provided that the 165th amino acid is an amino acid other than D, and having a conjugation activity;
(4) a protein consisting of the amino acid sequence of SEQ ID NO: 11, or the amino acid sequence of SEQ ID NO: 11 in which the 233rd amino acid is replaced with an amino acid other than D;
(5) a protein consisting of an amino acid sequence derived from the amino acid sequence of the protein described in (4) by substitution, deletion, insertion, and/or addition of one or more amino acids, provided that the 233rd amino acid is an amino acid other than D, and having a conjugation activity;
(6) a protein consisting of an amino acid sequence having an identity of at least 90% to the amino acid sequence of the protein described in (4), provided that the 233rd amino acid is an amino acid other than D, and having a conjugation activity; and
(7) a protein consisting of the amino acid sequence of SEQ ID NO: 24, or the amino acid sequence of SEQ ID NO: 24 in which the 165th amino acid is replaced with an amino acid other than D.

Brief Description of the Drawings

[Fig. 1] Map of pRSFDuet-1 (Novagen)
[Fig. 2-1] Map of pRSFDuet-1-MBP-EndoF3 D165Q
[Fig. 2-2] Map of pRSFDuet-1-EndoS D233Q
[Fig. 3] Fig. 3 is a schematic diagram of antibody-GlcNAc obtained by hydrolyzing the N297-linked glycan of the EndoS used as the antibody.
[Fig. 4] Fig. 4 is a schematic diagram of conjugation using an EndoF3 mutant enzyme.
[Fig. 5] Fig. 5 is an HPLC chromatogram of ADC obtained by conjugation of antibody-GlcNAc and a daunorubicin-attached glycan using an EndoF3 mutant enzyme.

[Fig. 6] Fig. 6 is an HPLC chromatogram of ADC obtained by conjugation of antibody-GlcNAc and an MMAE-attached glycan using an EndoF3 mutant enzyme.

[Fig. 7] Fig. 7 is an MS chromatogram of ADC obtained by conjugation of antibody-GlcNAc and a daunorubicin-attached glycan using an EndoF3 mutant enzyme.

[Fig. 8] Fig. 8 is an MS chromatogram of ADC obtained by conjugation of antibody-GlcNAc and an MMAE-attached glycan using an EndoF3 mutant enzyme.

[Fig. 9] Amino acid sequence of EndoF3_D165Q (SEQ ID NO: 1), optimized nucleotide sequence of EndoF3_D165Q (SEQ ID NO: 2), and amino acid sequence of the maltose binding protein (SEQ ID NO: 5)

[Fig. 10] Full-length nucleotide sequence of pRSFDuet-1-MBP-EndoF3 D165Q (SEQ ID NO: 10)

[Fig. 11] Amino acid sequence of EndoS_D233Q (SEQ ID NO: 11), and optimized nucleotide sequence of EndoS _D233Q (SEQ ID NO: 12)

[Fig. 12] Full length nucleotide sequence of pRSFDuet-1-EndoS D233Q (SEQ ID NO: 19)

[Fig. 13] AP3-attached glycan conjugate (HPLC)

[Fig. 14] AP3-attached glycan conjugate (MS).

[Fig. 15] Exatecan-attached glycan conjugate (HPLC).

[Fig. 16] Exatecan-attached glycan conjugate (MS).

[Fig. 17] MMAE-PEG3-attached glycan conjugate and MMAE-PEG4-attached glycan conjugate (HPLC)

[Fig. 18] MMAE-PEG3-attached glycan conjugate (MS)

[Fig. 19] MMAE-PEG4-attached glycan conjugate (MS)

[Fig. 20] MMAE-GGFG-attached glycan conjugate (HPLC)

[Fig. 21] MMAE-GGFG-attached glycan conjugate (MS)

[Fig. 22] Map of pRSFDuet-1-MBP-WP1328

[Fig. 23] Nucleotide sequence of WP1328 (SEQ ID NO: 20), and amino acid sequence of WP1328 (SEQ ID NO: 24)

[Fig. 24] Total nucleotide sequence of pRSFDuet-1-MBP-WP1328 (SEQ ID NO: 23)

## Modes for Carrying out the Invention

The present invention relates to a method for producing an antibody drug conjugate (hereinafter referred to as ADC) comprising the following step. The production method of the present invention comprises the step of conjugating a glycan-homogenized antibody and a glycosylated drug. The step of conjugating a glycan-homogenized antibody and a glycosylated drug can be performed by a synthetic method or an enzymatic method.

[Enzyme]

When the conjugation step is performed by an enzymatic method, an endo-β-N-acetylglucosaminidase mutant enzyme can be used. Concerning the present invention, the term endo-β-N-acetylglucosaminidase mutant enzyme refers to an endo-β-N-acetylglucosaminidase mutant enzyme showing suppressed hydrolysis ability. Examples of typical endo-β-N-acetylglucosaminidases include EndoA, EndoD, EndoM, EndoH, EndoF2, EndoF3, EndoE, EndoS, and EndoS2.

In a preferred embodiment, among the endo-β-N-acetylglucosaminidase mutant enzymes, a mutant enzyme that is EndoS of which 233rd asparagine (D) is replaced, a mutant enzyme that is EndoS2 of which 184th asparagine (D) is replaced, or a mutant enzyme that is EndoF3 of which 165th asparagine (D) is replaced, preferably a mutant enzyme that is EndoS of which 233rd asparagine (D) is replaced, or a mutant enzyme that is EndoF3 of which 165th asparagine (D) is replaced is used.

An example of more preferred endo-β-N-acetylglucosaminidase mutant enzyme is a mutant enzyme comprising any one of the following proteins.

(1) a protein consisting of the amino acid sequence of SEQ ID NO: 1, or the amino acid sequence of SEQ ID NO: 1 in which the 165th amino acid is replaced with an amino acid other than aspartic acid (D);

(2) a protein consisting of an amino acid sequence derived from the amino acid sequence of the protein described in (1) by substitution, deletion, insertion, and/or addition of one or more amino acids, provided that the 165th amino acid is an amino acid other than D, and having a conjugation activity;

(3) a protein consisting of an amino acid sequence having an identity of at least 90% to the amino acid sequence of the protein described in (1), provided that the 165th amino acid is an amino acid other than D, and having a conjugation activity;

(4) a protein consisting of the amino acid sequence of SEQ ID NO: 11, or the amino acid sequence of SEQ ID NO: 11 in which the 233rd amino acid is replaced with an amino acid other than D;

(5) a protein consisting of an amino acid sequence derived from the amino acid sequence of the protein described

in (4) by substitution, deletion, insertion, and/or addition of one or more amino acids, provided that the 233rd amino acid is an amino acid other than D, and having a conjugation activity;

(6) a protein consisting of an amino acid sequence having an identity of at least 90% to the amino acid sequence of the protein described in (4), provided that the 233rd amino acid is an amino acid other than D, and having a conjugation activity; and

(7) a protein consisting of the amino acid sequence of SEQ ID NO: 24, or the amino acid sequence of SEQ ID NO: 24 in which the 165th amino acid is replaced with an amino acid other than D.

Another example of the endo-β-N-acetylglucosaminidase mutant enzyme is a mutant enzyme comprising any one of the following proteins:

(1) a protein consisting of the amino acid sequence of SEQ ID NO: 1, or the amino acid sequence of SEQ ID NO: 1 in which the 165th glutamine (Q) is replaced with alanine (A);

(2) a protein consisting of an amino acid sequence derived from the amino acid sequence of the protein described in (1) by substitution, deletion, insertion, and/or addition of one or more amino acids, provided that the 165th amino acid is Q or A, and having a conjugation activity;

(3) a protein consisting of an amino acid sequence having an identity to the amino acid sequence of the protein described in (1), provided that the 165th amino acid is Q or A, and having a conjugation activity;

(4) a protein consisting of the amino acid sequence of SEQ ID NO: 11;

(5) a protein consisting of an amino acid sequence derived from the amino acid sequence of the protein described in (4) by substitution, deletion, insertion, and/or addition of one or more amino acids, provided that the 233rd amino acid is Q, and having a conjugation activity;

(6) a protein consisting of an amino acid sequence having an identity to the amino acid sequence of the protein described in (4), provided that the 233rd amino acid is Q, and having a conjugation activity; and

(7) a protein consisting of the amino acid sequence of SEQ ID NO: 24, or the amino acid sequence of SEQ ID NO: 24 in which the 165th amino acid is replaced with an amino acid other than aspartic acid (D).

In the sequence listing, the amino acid sequence of EndoF3 D165Q, corresponding to EndoF3 in which the 165th amino acid is replaced with glutamine (Q), is shown as SEQ ID NO: 1. The amino acid sequence of EndoS D233Q, corresponding to EndoS in which the 233rd amino acid is replaced with glutamine (Q) is also shown as SEQ ID NO: 11. In the following descriptions, the present invention may be explained by referring to the cases using EndoF3 D165Q or EndoS D233Q as the endo-β-N-acetylglucosaminidase mutant enzyme, but those skilled in the art can also understand the cases using other mutant enzymes by appropriately applying the explanations.

The endo-β-N-acetylglucosaminidase mutant enzyme used for the conjugation may be the mutant enzyme described above into which a point mutation is further introduced. Examples of such an enzyme include a mutant enzyme consisting of a protein consisting of the amino acid sequence of SEQ ID NO: 1, or the amino acid sequence of SEQ ID NO: 1 in which the 165th glutamine (Q) is replaced with alanine (A), and having a mutation corresponding to at least one selected from the group consisting of K81T, M97W, Y, F, V, G, S, or Q, V129G, Q250I, N270R, L or T, and Y282W. Particularly preferred examples include EndoF3 D165Q/M97G, which is a protein consisting of the amino acid sequence of SEQ ID NO: 1, or the amino acid sequence of SEQ ID NO: 1 in which the 165th glutamine (Q) is replaced with alanine (A), wherein the 97th M is replaced with G.

Concerning the present invention, the term conjugation activity used for the endo-β-N-acetylglucosaminidase mutant enzyme means such an activity that when the conjugation reaction between a glycan-homogenized antibody and a glycosylated drug is performed with the enzyme, at least 20% of drug-added antibodies (adducts) can be generated, unless otherwise specified (100 (%) - Remaining ratio (%) is at least 20%). The ratio (%) mentioned here can be calculated on the basis of the peak area of the HPLC chromatogram for the conjugation reaction solution. If more than one type of adducts are formed, for example, if one drug adduct (1-adduct) and two drugs adduct (2-adduct) are formed, all adducts should be considered. Specifically, the remaining ratio can be calculated in accordance with the following equation.

$$\text{Remaining ratio (\%)} = [\text{Peak area value of unreacted antibody}]/[(\text{Peak area value of unreacted antibody}) + (\text{Peak area value of adduct*})] \times 100$$

*When multiple types of adducts are generated, sum of peak area values of respective adducts are used.

1-Adduct conversion rate (%) = [Peak area value of 1-adduct]/[(Peak area value of unreacted antibody) + (Peak area value of 1-adduct) + (Peak area value of 2-adduct)] x 100

2-Adduct conversion rate (%) = [Peak area value of 2-adduct]/[(Peak area value of unreacted antibody) + (Peak area value of 1-adduct) + (Peak area value of 2-adduct)] x 100

The present invention also provides a polynucleotide encoding an endo-β-N-acetylglucosaminidase mutant enzyme that can be used in the method for producing an antibody-drug conjugate. Namely, the present invention provides: a polynucleotide encoding any one of the following proteins:

(1) a protein consisting of the amino acid sequence of SEQ ID NO: 1, or the amino acid sequence of SEQ ID NO: 1 in which the 165th amino acid is replaced with an amino acid other than aspartic acid (D);
(2) a protein consisting of an amino acid sequence derived from the amino acid sequence of the protein described in (1) by substitution, deletion, insertion, and/or addition of one or more amino acids, provided that the 165th amino acid is an amino acid other than D, and having a conjugation activity;
(3) a protein consisting of an amino acid sequence having an identity of at least 90% to the amino acid sequence of the protein described in (1), provided that the 165th amino acid is an amino acid other than D, and having a conjugation activity;
(4) a protein consisting of the amino acid sequence of SEQ ID NO: 11, or the amino acid sequence of SEQ ID NO: 11 in which the 233rd amino acid is replaced with an amino acid other than D;
(5) a protein consisting of an amino acid sequence derived from the amino acid sequence of the protein described in (4) by substitution, deletion, insertion, and/or addition of one or more amino acids, provided that the 233rd amino acid is an amino acid other than D, and having a conjugation activity;
(6) a protein consisting of an amino acid sequence having an identity of at least 90% to the amino acid sequence of the protein described in (4), provided that the 233rd amino acid is an amino acid other than D, and having a conjugation activity; and
(7) a protein consisting of the amino acid sequence of SEQ ID NO: 24, or the amino acid sequence of SEQ ID NO: 24 in which the 165th amino acid is replaced with an amino acid other than D.

Another example of the polynucleotide encoding an endo-β-N-acetylglucosaminidase mutant enzyme that can be used in the method for producing an antibody-drug conjugate is a polynucleotide that encodes any one of the following proteins:

(1) a protein consisting of the amino acid sequence of SEQ ID NO: 1, or the amino acid sequence of SEQ ID NO: 1 in which the 165th glutamine (Q) is replaced with alanine (A);
(2) a protein consisting of an amino acid sequence derived from the amino acid sequence of the protein described in (1) by substitution, deletion, insertion, and/or addition of one or more amino acids, provided that the 165th amino acid is Q or A, and having a conjugation activity;
(3) a protein consisting of an amino acid sequence having an identity of at least 90% to the amino acid sequence of the protein described in (1), provided that the 165th amino acid is Q or A, and having a conjugation activity;
(4) a protein consisting of the amino acid sequence of SEQ ID NO: 11;
(5) a protein consisting of an amino acid sequence derived from the amino acid sequence of the protein described in (4) by substitution, deletion, insertion, and/or addition of one or more amino acids, provided that the 233rd amino acid is Q, and having a conjugation activity;
(6) a protein consisting of an amino acid sequence having an identity of at least 90% to the amino acid sequence of the protein described in (4), provided that the 233rd amino acid is Q, and having a conjugation activity; and
(7) a protein consisting of the amino acid sequence of SEQ ID NO: 24, or the amino acid sequence of SEQ ID NO: 24 in which the 165th amino acid is replaced with an amino acid other than aspartic acid (D).

[Antibody]

The antibody used in the present invention may be any of a mouse antibody, chimeric antibody, humanized

antibody, and human antibody, but a humanized or human antibody is preferred. The antibody used may also be modified or created by the Potelligent technology (removal of fucose from the Fc region of IgG).

An example of the antibody is an anti-HER2 antibody. Anti-HER2 antibodies are recombinant anti-HER2 monoclonal antibodies, and include a chimeric antibody with a human constant region, humanized antibody in which the CDR (complementarity determining region) of a human antibody is replaced into the CDR of an anti-HER2 antibody of a non-human animal (CDR transplanted antibody), and human antibody that is an expression product of a human-derived antibody gene, but a humanized antibody and human antibody are preferred. Fully human monoclonal antibodies can be produced by immunizing mice into which most of the human immunoglobulin heavy and light chain loci have been introduced by transgenesis. A preferred anti-HER2 antibody is trastuzumab (Herceptin (registered trademark)), which is a humanized IgG1 antibody.

The present invention can be applied to antibodies used as antibody drugs. Specific examples include ibritumomabtiuxetan, iodine131, catumaxomab, blinatumomab, muromonab-CD3, abciximab, rituximab, basiliximab, infliximab, cetuximab, brentuximab, siltuximab, dinutuximab, obiltoxaximab, daclizumab, palivizumab, gemtuzumab, alemtuzumab, omalizumab, efalizumab, bevacizumab, natalizumab, tocilizumab, ranibizumab, eculizumab, certolizumab pegol, mogamulizumab, pertuzumab, obinutuzumab, vedolizumab, pembrolizumab, idarucizumab, mepolizumab, elotuzumab, daratumumab, ixekizumab, reslizumab, adalimumab, panitumumab, golimumab, ustekinumab, canakinumab, ofatumumab, denosumab, ipilimumab, belimumab, raxibacumab, ramucirumab, nivolumab, secukinumab, evolocumab, alirocumab, and necitumumab.

An example of the class of antibodies used in the production method of the present invention is IgG, and an example of the subclass is IgG1.

[Drug]

In the present invention, anticancer agents can be used as the drug. Examples of anticancer agents include known anticancer agents such as tubulin synthesis inhibitors (anticancer plant alkaloids), topoisomerase inhibitors, alkylating agents that are inhibitors of nucleic acid synthesis, antimetabolites, antibiotic anticancer agents, hormonal agents, platinum agents, and non-specific anti-malignant tumor agents, as well as anticancer agents to be newly developed. Specifically, there can be mentioned tubulin synthesis inhibitors (anticancer plant alkaloids) such as auristatin, vincristine sulfate, vinblastine sulfate, vindesine sulfate, docetaxel hydrate, paclitaxel, vinorelbine tartrate, and maytansinoids; topoisomerase inhibitors such as camptothecin, topotecan, etoposide, irinotecan hydrochloride and nogitecan hydrochloride; alkylating agents such as cyclophosphamide, ifosfamide, melphalan, thiotepa, busulfan, carboquone, dacarbazine, nimustine hydrochloride and ranimustine; antimetabolites such as methotrexate, mercaptopurine, 6-mercaptopurine riboside, fluorouracil (5-FU), tegafur, tegafur-uracil, carmofur, doxyfluoridine, cytarabine ocfosfate, hydroxycarbamide, cytarabine, gemcitabine hydrochloride, fludarabine phosphate, enocitabine, and leucovorin; anticancer antibiotics such as doxorubicin hydrochloride, idarubicin hydrochloride, epirubicin hydrochloride, pirarubicin hydrochloride, daunorubicin hydrochloride, aclarubicin hydrochloride, mitomycin C, actinomycin D, bleomycin, peplomycin sulfate, neocartinostatin, and zinostatin stimalamar; hormonal agents such as estramustine sodium phosphate, flutamide, bicalutamide, goserelin acetate, leuprorelin acetate, tamoxifen citrate, fadrozole hydrochloride hydrate, anastrozole, mepithiostan, epithiostanol, and medroxyprogesterone acetate; platinum preparations such as cisplatin, carboplatin and nedaplatin; nonspecific anti-malignant tumor agents such as Krestin, lentinan, schizophyllan, and ubenimex; mitoxantrone hydrochloride, procarbazine hydrochloride, pentostatin, sobuzoxane, tretinoin, L-asparaginase, asegratone, mitotane, sodium porphymer, and so forth.

In a preferred embodiment, the drug is any selected from the group consisting of anthracyclines, auristatins, maytansines, camptothecins, pyrrolobenzodiazepine dimers, calicheamicins, and duocarmycins.

Examples of anthracyclines include daunorubicin (daunomycin), doxorubicin (adriamycin), PNU-159682, and pirarubicin.

Examples of auristatins include auristatin, which is a tubulin synthesis inhibitor and an extremely potent antitumor agent. Auristatins include monomethyl auristatin E (MMAE), monomethyl auristatin D (MMAD), monomethyl auristatin F (MMAF), and so forth. Among these, monomethyl auristatin E (MMAE) is preferred.

Examples of maytansines include ansamitosin P3 (AP3), DM1, and DM4.

Examples of camptothecins include exatecan, SN-38, and DXd.

Other specific examples include PBD-dimer, calicheamicin, and duocarmycin.

[Linker]

In the glycosylated drug used in the present invention, the drug is bound to a glycan having an oxazolinated reducing end via a linker. In a preferred embodiment, the linker contains a spacer (-C-B-A- described below) and a cleavable portion (-E-D-).

The spacer is a moiety that connects the glycan and a binding product of the linker (in a narrow sense, -E-D- as described below) and the drug. Examples of the binding group include an amide, ester, alkoxyalkyl, carbamoyl, sulfonamide, sulfonate, phosphonamide, phosphonate, aldoimine, ketoimine, oxime, click binding site formed from alkyne and azide etc., and so forth.

Typical click binding sites are listed in the following references: Chem, Rev., 113, 4905-4979 (2013); Method Mol. Biol., 2078, 83-97 (2020); Chem. Soc. Rev., 48, 4361-4374 (2019); and Protein Cell, Jan., 9(1), 33-46 (2018)). Preferred examples of such binding sites include 1,2,3-triazole moiety formed from alkyne and azide, and tetracyclic moiety containing 1,2,3-triazole formed from DBCO and azide. An even more preferred example is tetracyclic moiety containing 1,2,3-triazole formed from azide.

A common technique for improving water solubility of the linker for the purpose of reducing ADC aggregation is to introduce PEG groups. In the production method of the present invention, for example, PEG groups are introduced into the spacer. Such PEG can have a linear or branched chain, and is preferably a linear PEG. The PEG chain length is not particularly limited, and examples of PEG include PEG2 (the number represents the number of repeats), PEG3, PEG4, and PEG5 to PEG12.

The cleavable portion is a moiety cleavable under specific conditions that do not cause denaturation or degradation of the protein. In other words, it contains a structure that can be cleaved in vivo. The specific conditions that do not cause denaturation and degradation of the protein are conditions of degradation caused by one or more substances selected from the group consisting of acidic substances, basic substances, reducing agents, oxidizing agents, and enzymes (References: Bioorg. Med. Chem., 20 571-582 (2012); J. Control. Release, 99, 423-434 (2004); Bioconjugate Chem., 28, 1906-1915 (2017)). Examples of such a cleavable portion include disulfide bond, acetal bond, ketal bond, aldoimine bond, ketoimine bond, oxime bond, ester bond, amide bond, carbamoyl bond, carbonate bond, alkoxyalkyl bond, sulfone-containing bond, and phosphonic acid-containing bond. Preferred examples of these cleavable portions include peptides consisting of 2 to 6 amino acid residues. Examples thereof include Val-Cit, Gly-Gly-Phe-Gly (GGFG), Val-Ala, Lys-Phe (KF), and Gly-Gly-Val-Gly (GGVG).

In a preferred embodiment, the linker has a structure represented by the general formula -E-D-C-B-A-, where A is bound to the glycan and E is bound to the drug.

In the formula,

A, B, C, and E are each independently selected from the group consisting of an alkyl group, ester group, carbamoyl group, alkoxyalkyl group, imine group, hydrazone group, azo group, sulfone group, aromatic group, and single bond; and

D is a peptide consisting of 2 to 6 amino acid residues.

In the formula,
A, B, C, and E may also be independently selected from the group consisting of $-(CH_2)_{1-12}-$, $-(CH_2)_{1-12}-O-C(O)-(CH_2)_{0-12}$, -O-C(O)-NH-, $-(CH_2CH_2O)_{1-12}-(CH_2)_{1-12}-$, - C(=NR$^1$)- or $-(CH_2)_{1-12}-N(R^1)-$ ($R^1$ is hydrogen atom or $-(CH_2)_{1-6}$), -$R^2$C=N-N$R^3$- or - $R^2$C=N-O$R^3$- ($R^2$ and $R^3$ are independently hydrogen atom or $-(CH_2)_{1-6}$), -N=N-, $-SO_3-$, a divalent aromatic hydrocarbon group (group formed by removing two hydrogen atoms from an aromatic hydrocarbon ring, such as phenylene, indenylene, naphthylene, fluorenylene, phenanthrenylene, anthrylene, and pyrenylene), and a single bond.

A can be $-(CH_2CH_2O)_{m1}-(CH_2)_{m2}-$, $-(CH_2)_{m2}-$, or a single bond.

B may be any selected from the group consisting of the following groups:

[Formula 3]

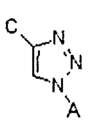 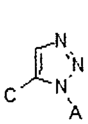 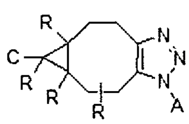 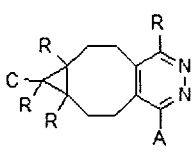 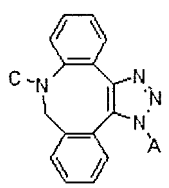 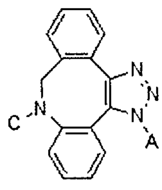

In the formulas, R is independently hydrogen atom, alkyl having 1 to 4 carbon atoms, alkoxy having 1 to 4 carbon atoms, -CN, -OH, $-CF_3$, or NRR.

C is $-C(O)-(CH_2)_{n1}-C(O)-$, $-C(O)-(CH_2)_{n1}-C(O)-NH-(CH_2CH_2O)n2-(CH_2)_{n3}-C(O)-$, $-C(O)-(CH_2)_{n1}-C(O)-NH-(CH_2)_{n3}-C(O)-$, or a single bond,
E is -aminobenzylalcohol-C(O)-, $-NH-(CH_2)_o-$, or a single bond

m1 is an integer of from 1 to 12,
m2 is an integer of from 1 to 6,
n1 is an integer of from 1 to 6,
n2 is an integer of from 1 to 12,
n3 is an integer of from 1 to 6, and
o is an integer of from 1 to 6.

B may be any of the following groups:

[Formula 4]

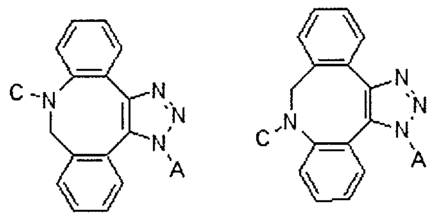

In a particularly preferred embodiment, the linker consists of the following structure.

[Formula 5]

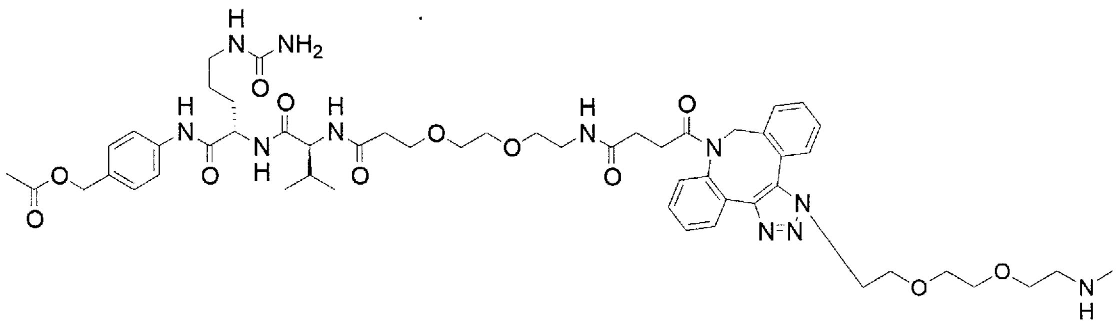

In a particularly preferred embodiment, the production method of the present invention can be carried out as follows.

(1) Cleave glycans of anti-HER2 antibodies, which originally have heterogeneous glycan structures. Cleavage of the glycans can be performed by using endo-β-N-acetylglucosaminidase (EndoS). EndoS cleaves an N-type complex glycan attached to the 297th asparagine (Asn) in the CH domain of the Fc region of the antibody to leave one N-acetylglucosamine (GlcNAc) residue at the reducing end of the N-type complex glycan. Fucose may or may not bind to the N-acetylglucosamine of the reducing end. Then, the antibodies of which glycans have been cleaved can be purified by using chromatography. Examples of the chromatography includes gel filtration chromatography, affinity chromatography, ion exchange chromatography, hydrophobic chromatography, and so forth.
(2) Conjugate a glycosylated drug to the glycan of the antibody-GlcNAc obtained in (1). The conjugation can be performed by using an endo-β-N-acetylglucosaminidase mutant enzyme. To the one N-acetylglucosamine (GlcNAc) residue at the reducing end of the N-type complex glycan bound to the 297th asparagine (Asn) located in the CH domain of the Fc region of the antibody-GlcNAc, N-acetylglucosamine having an oxazolinated reducing end of the glycosylated drug binds. Then, the antibodies of which glycans have been cleaved can be purified by using chromatography. Examples of the chromatography includes gel filtration chromatography, affinity chromatography, ion exchange chromatography, hydrophobic chromatography, and so forth.

[Terminology etc.]

Concerning the present invention, in the expression of "an amino acid sequence derived by substitution, deletion, insertion, and/or addition of one or more amino acids" used for a protein or an amino acid sequence, the number of

amino acids modified by substitution or the like is not particularly limited for any proteins so long as the proteins consisting of the amino acid sequence have the desired function, unless especially specified, but it may be about 1 to 750, 1 to 500, 1 to 250, 1 to 200, 1 to 150, 1 to 100, 1 to 50, 1 to 40, 1 to 30, 1 to 20, 1 to 15, 1 to 9, or 1 to 4. However, in the case of substitutions of amino acids having similar properties, substitutions of a further larger number of amino acids may be possible. The means for preparing polynucleotides for such amino acid sequences or proteins are well known to those skilled in the art.

In the explanations of the present invention, as for amino acids or amino acid residues, A stands for alanine, C for cysteine, D for aspartic acid, E for glutamic acid, F for phenylalanine, G for glycine, H for histidine, I for isoleucine, K for lysine, L for leucine, M for methionine, N for asparagine, P for proline, Q for glutamine, R for arginine, S for serine, T for threonine, U for selenocysteine, V for valine, W for tryptophan, and Y for tyrosine, unless especially noted,.

Concerning the present invention, the term "identity" used for nucleotide sequences (also referred to as "base sequences") or amino acid sequences means percentage of number of matching nucleotides or amino acids between two optimally aligned sequences for any nucleotide sequences and amino acid sequences, unless otherwise noted. In other words, it can be calculated in accordance with the equation: Identity = (Number of matched positions/Total number of positions) x 100, and can be calculated by using commercially available algorithms. Such algorithms are also incorporated in the NBLAST and XBLAST programs described in Altschul et al., J. Mol. Biol., 215(1990) 403-410. In more detail, searches and analyses for nucleotide or amino acid sequence identities can be performed with algorithms or programs well known to those skilled in the art (e.g., BLASTN, BLASTP, BLASTX, ClustalW). When using programs, the parameters can be appropriately set by those skilled in the art, or the default parameters of each program can also be used. The specific methods of these analyses are also well known to those skilled in the art. The genetic information processing software Genetyx (registered trademark, Genetyx Corporation) may be used to calculate the identity. If the target sequence for which the % identity is to be calculated has an additional sequence at the end, such as a tag sequence, that is not present in the sequence to be compared, the additional sequence is not included in the calculation of the % identity.

Concerning the present invention, the term identity used for nucleotide sequences or amino acid sequences means sequence identity of at least 50%, for example, 60% or higher, 70% or higher, preferably 80% or higher, more preferably 85% or higher, even more preferably 90% or higher, even more preferably 95% or higher, even more preferably 97.5% or higher, even more preferably 99% or higher, in any case, unless otherwise stated.

The polynucleotides or genes and proteins or enzymes used in the present invention can be prepared by those skilled in the art using conventional techniques.

## Examples

Hereafter, the present invention will be specifically explained with reference to the following examples. However, the present invention is not limited to these examples. The reagents, solvents and starting materials mentioned without any particular explanations are commercially available or can be obtained from well-known supply sources.

The protein concentrations mentioned in this description were measured by the Bradford method (measurement wavelength: 595 nm).

The following abbreviations are used in the following examples.

CDMBI: 2-Chloro-1,3-dimethyl-1H-benzimidazol-3-ium chloride
HOAt: 1-Hydroxy-7-azabenzotriazole
HOBt: 1-Hydroxybenzotirazole
IPTG: Isopropyl β-D-1-thiogalactopyranoside
MMAE: Monomethyl auristatin E

### [Example 1: Preparation of EndoF3 mutant enzyme]

#### (1) Preparation of pRSFDuet-1-MBP-EndoF3 D165Q

The nucleotide sequence of EndoF3 (NZ_CP067018, locus_tag I6H88_03475; WP_034868774.1 for the amino acid sequence) was modified with the D165Q mutation (EndoF3 D165Q amino acid sequence is shown as SEQ ID NO: 1), a codon-optimized sequence thereof for expression in *E. coli* (SEQ ID NO: 2) was artificially synthesized (ThermoFisher Scientific), and this sequence was amplified by PCR using primers FEndoF3-NtermMBP and REndoF3-NtermMBP (30 cycles of 98°C for 10 seconds, 60°C for 5 seconds, and 68°C for 8 seconds).

Nucleotide sequence of FEndoF3-NtermMBP (SEQ ID NO: 3)

ACTATCGAGGGAAGGGCAACCGCACTGGCAGGTAGC
Nucleotide sequence of REndoF3-NtermMBP (SEQ ID NO: 4)
CCAATTGAGATCTTCAGTTTTTAACGGCATCACGAACTG

Similarly, the maltose binding protein (amino acid sequence is shown as SEQ ID NO: 5) and Factor Xa cleavage sequence ATCGAGGGAAGG (SEQ ID NO: 6; amino acid sequence is IEGR (SEQ ID NO: 7)) were cloned into the pRSF-MBP vector (pRSDuet-1, Fig. 2) at the BglII site, and the vector was amplified by PCR using primers FNtermMBP and RNtermMBP (30 cycles of 98°C for 10 seconds, 60°C for 5 seconds, and 68°C for 8 seconds).

Nucleotide sequence of FNtermMBP (SEQ ID NO: 8)
AGATCTCAATTGGATATCGG
Nucleotide sequence of RNtermMBP (SEQ ID NO: 9)
CCTTCCCTCGATAGTCTGCGCGTCTTTCAGGGC

The two fragments obtained were cloned by using the In-Fusion kit (TAKARA) so that the Factor Xa sequence should be placed at the C-terminus of MBP and the EndoF3 sequence of which N-terminal signal sequence is removed should be placed downstream thereof. *E. coli* JM109 (TAKARA) cells were transformed with the resulting In-Fusion solution and cultured overnight at 37°C. Colony PCR was performed on the obtained colonies, and pRSFDuet-1-MBP-EndoF3 D165Q (SEQ ID NO: 10) was obtained as a plasmid from a positive clone.

(2) Point mutation-introduced construct

PCR was performed to introduce the target point mutation into pRSFDuet-1-MBP-EndoF3 D165Q. DpnI was added in a volume of 0.001 mL to the fragment obtained by PCR, the reaction was allowed at 37°C for 1 hour, and the DNA fragment was purified. The obtained fragment was introduced into *E. coli* JM109 cells by the heat shock method, and then the cells were applied to an LK plate, and incubated overnight at 37°C to obtain colonies. The colonies were inoculated into the LB medium containing kanamycin (3 mL), and incubated overnight, and plasmid extraction was performed with Wizard Plus SV Minipreps DNA Purification Systems to obtain 0.05 mL of a plasmid DNA solution from the culture.

(3) Transformation

The above plasmid (0.001 mL) was added to BL21(DE3) competent cells (Novagen, 0.05 mL), and the cells were left standing at 0°C for 20 minutes, and left standing on an aluminum block at 42°C for 45 seconds for heat shock treatment. The SOC medium (0.45 mL) warmed to 37°C was added, and the mixture was incubated at 37°C for 1 hour. The culture was centrifuged (room temperature, 6000 g, 3 minutes) to precipitate the cells. After removing the supernatant (0.4 mL), the cells were resuspended, inoculated on LB-Agar plates (containing 0.05 mg/mL of kanamycin), and cultured overnight at 37°C as standing culture.

(4) Expression culture

One colony obtained by the above transformation was added to Superbroth (10 mL, containing Bacto Trypton, (32 g), Bacto Yeast Extract (5 g), NaCl (5 g), 1 M aqueous sodium hydroxide (5 mL), and pure water (950 mL), and containing 0.05 mg/mL of kanamycin), and cultured overnight at 37°C. The culture (2 mL) was added to Superbroth (200 mL, containing 0.05 mg/mL kanamycin) in a 500 mL baffled flask. The cells were cultured at 37°C for 6 hours, IPTG was added at a final concentration of 1 mM, and the cells were further cultured overnight at 37°C. The culture was centrifuged (4°C, 10000 rpm, 5 minutes), the supernatant was removed, and the cells were collected.

(5) Preparation of insoluble fraction and refolding

BugBuster (40 mL) and Lysonase (0.04 mL) were added to the cells, and the cells were suspended by pipetting, and the suspension was made homogenous by inversion for 20 minutes. This cell suspension was centrifuged (room temperature, 10000 rpm, 20 minutes), the supernatant was removed, and the precipitates were obtained as the insoluble fraction. To this insoluble fraction, a 0.15% CHAPS/20% acetic acid solution (40 mL) was added, they were mixed by inversion at 37°C for 2.5 hours, and the mixture was centrifuged (room temperature, 10000 rpm, 10 minutes) to obtain a supernatant. This supernatant was added to Slide-A-Lyzer Dialysis Flask (20 kDa MWCO, Thermo Fischer Scientific) and dialyzed against ultrapure water at 4°C for 2 days (the outer dialysis solution was changed 3 times with intervals of

4 hours or longer). The dialysis was further continued overnight at 4°C against 10 mM Tris-HCl buffer (pH 8.0). This solution was subjected to buffer replacement with a 50 mM Tris-HCl buffer, pH7.4 by ultrafiltration (Amicon Ultra 30 kDa) to obtain an EndoF3 mutant enzyme.

[Example 2: Preparation of EndoS mutant enzyme]

(1) Construction of plasmid

The nucleotide sequence of EndoS (CP043530, locus_tag MGAS2221_1552, WP 011285695.1 for the amino acid sequence) was modified with the D233Q mutation (amino acid sequence of EndoS _D233Q is shown as SEQ ID NO: 11), and a codon-optimized sequence thereof for expression in *E. coli* (SEQ ID NO: 12) was artificially synthesized as two parts, those for the N-terminus portion and the C-terminus portion (ThermoFisher Scientific). By using the above sequence as a template, the N-terminus side sequence and the C-terminus side sequence were amplified by PCR (30 cycles of 98°C for 10 seconds, 60°C for 5 seconds, and 68°C for 8 seconds) using primers FEndoS_D233Q_His_pRSF and RendoS_D233Q_front, and FendoS_D233Q_back and REndoS D233QHis_pRSF, respectively. Similarly, pRSFDuet-1 was amplified by PCR using primers FpRSF_InF_His_BamHI and RpRSF_InF_His_NcoI (30 cycles of 98°C for 10 seconds, 60°C for 5 seconds, and 68°C for 8 seconds).

FendoS_D233Q_His_pRSF (SEQ ID NO: 13)
CACCATCATCACCACGATAAACATTTGTTGGTAAAAAG
RendoS_D233Q_front (SEQ ID NO: 14)
GCTAATTTTTTAAATTTATTTAGACCTTCTAAAC
FendS_D233Q_back (SEQ ID NO: 15)
GGTCTAAATAAATTTAAAAAATTAGCTCAATTAGACTTG
RendoS_D233Q_His_pRSF (SEQ ID NO: 16)
CTCGAATTCGGATCCTTATTTTTTTAGCAGCTGCC
FpRSF_InF_His BamHI (SEQ ID NO: 17)
GGATCCGAATTCGAGCTCG
RpRSF_InF His NcoI (SEQ ID NO: 18)
GTGGTGATGATGGTGATGGCTGCTGCCCAT

The resulting three fragments were ligated using the In-Fusion kit (TAKARA). The start codon Met of EndoS sequence was removed and the fragment was placed downstream of the His tag. *E. coli* JM109 cells (TAKARA) were transformed with the resulting In-Fusion solution, and cultured overnight at 37°C. Colony PCR was performed on the obtained colonies, and pRSFDuet-1-EndoS D233Q (SEQ ID NO: 19) was obtained as a plasmid from a positive clone.

(2) Transformation

The above plasmid (0.001 mL) was added to BL21(DE3) competent cells (0.05 mL, Novagen), and the cells were left standing at 0°C for 20 minutes, and then left standing on an aluminum block at 42°C for 45 seconds for heat shock treatment. The SOC medium (0.45 mL) warmed to 37°C was added, and the mixture was incubated at 37°C for 1 hour. The culture was centrifuged (room temperature, 6000 g, 3 minutes) to precipitate the cells. After removing the supernatant (0.4 mL), the cells were resuspended, inoculated on LB-Agar plates (containing 0.05 mg/mL of kanamycin), and cultured overnight at 37°C as standing culture.

(3) Expression culture

One colony obtained by the above transformation was added to Superbroth (10 mL, containing Bacto Trypton, (32 g), Bacto Yeast Extract (5 g), NaCl (5 g), 1 M aqueous sodium hydroxide (5 mL), and pure water (950 mL), and containing 0.05 mg/mL of kanamycin), and cultured overnight at 37°C. The culture (2 mL) was added to Superbroth (200 mL, containing 0.05 mg/mL kanamycin) in a 500 mL baffled flask. The cells were cultured at 37°C for 6 hours, IPTG was added at a final concentration of 1 mM, and the cells were further cultured overnight at 37°C. The culture was centrifuged (4°C, 10000 rpm, 5 minutes), the supernatant was removed, and the cells were collected.

(4) Obtaining EndoS D233Q

BugBuster (5 mL) and Lysonase (0.5 mL) were added to the cells, the cells were suspended by pipetting, and

the suspension was made homogenous by inversion for 20 minutes. This cell suspension was centrifuged (4°C, 10000 rpm, 20 minutes), and the supernatant was collected. Streptomycin sulfate was added to the supernatant at a final concentration of 1%, and the mixture was left standing overnight at 4°C. This suspension was centrifuged (4°C, 10000 rpm, 20 minutes), and the supernatant was collected. To this supernatant, ammonium sulfate was added to a final concentration of 40%, and the mixture was left standing at 0°C for 1 hour. This suspension was centrifuged (4°C, 10000 rpm, 20 minutes), and the supernatant was collected. To this supernatant, ammonium sulfate was added to a final concentration of 60%, and the mixture was left standing at 0°C for 1 hour. This suspension was centrifuged (4°C, 10000 rpm, 20 minutes), and the supernatant was collected. To this supernatant, 1 M Tris-HCl buffer, pH 7.5 was added to a final concentration of 20%, and purification was performed by column chromatography (His Trap HP) to obtain EndoS D233Q (SEQ ID NO: 11).

[Example 3: Preparation of Antibody-GlcNAc]

A schematic diagram of the preparation of antibody-GlcNAc is shown in Fig. 3. In an Eppendorf tube, 0.050 g of anti-HER2 antibody (prepared with reference to U.S. Patent No. 5,821,337, subclass is $IgG_1$) was subjected to buffer replacement with a sodium acetate buffer (GlycoBuffer 1, NEW ENGLAND BioLabs), and the total volume was adjusted to 0.3 mL with the same buffer. EndoS (0.025 mL, NEW ENGLAND BioLabs) was added to the solution, and the mixture was incubated at 37°C for 16 hours. EndoS in the reaction solution was removed with Chitin Beads (NEW ENGLAND BioLabs), and the recovered fraction was purified on an affinity column (rProtein A Sepharose Fast Flow, GE Healthcare). The purified fraction was subjected to buffer replacement with a 50 mM Tris-HCl buffer, pH 7.4 by ultrafiltration (Amicon Ultra 30 kDa) to obtain antibody-GlcNAc (0.050 g).

[Example 4: Measurement of activities of EndoF3 mutant enzyme and EndoS mutant enzyme]

By using the EndoF3 mutant enzymes prepared in Example 1 or the EndoS mutant enzyme prepared in Example 2, the antibody-GlcNAc prepared in Example 3, and the payload-attached glycan (the daunorubicin-attached glycan of Example 6 or MMAE-attached glycan of Example 11 described later) were conjugated (see Examples 5 and 6 described later). A schematic diagram of this conjugation is shown as Fig. 4. The obtained conjugate was measured by HPLC. The HPLC analysis conditions are shown below.

Column used: AdvanceBio HIC

Column size: 30 x 4.6 mm
Packing material particle size: 3.5 μm
Mobile phase A: 50 mM Aqueous sodium phosphate, pH 7.4
Mobile phase B: 2 M Aqueous ammonium sulfate, pH 7.0
Mobile phase C: 2-Propanol

LC Conditions:

The gradient was as follows.

[Table 1]

| Time (min) | Mobile phase A (%) | Mobile phase B (%) | Mobile phase C (%) |
|---|---|---|---|
| 0 | 50 | 45 | 5 |
| 12 | 70 | 9 | 21 |
| 13 | 75 | 0 | 25 |
| 14 | 75 | 0 | 25 |
| 15 | 50 | 45 | 5 |
| 23 | 50 | 45 | 5 |

Analysis time: 23 Minutes

Flow rate, 0.50 mL/minute; Column temperature. 25°C; Detection wavelength, 214 nm, 280 nm; Injection volume, 10 μL.

The HPLC chromatogram for the resultant of the conjugation with daunorubicin-attached glycan by the EndoF3 mutant enzyme is shown in Fig. 5, and that for the resultant of the conjugation with MMAE-attached glycan by the EndoF3 mutant enzyme is shown in Fig. 6. In the obtained HPLC chromatograms, the unreacted antibody-GlcNAc, 1-adduct consisting of one molecule of antibody-GlcNAc conjugated with one molecule of payload-attached glycan, and 2-adduct consisting of one molecule of antibody-GlcNAc conjugated with two molecules of payload-attached glycan were identified as the separated peaks. From the peak area ratios of the antibody-GlcNAc, 1-adduct and 2-adduct, the remaining ratio of the antibody-GlcNAc and the conjugation conversion ratio were calculated in accordance with the following equations. For the calculation of the conversion ratio, the peak area values observed with the detection wavelength of 214 nm were used.

Antibody-GlcNAc remaining ratio (%) = [Peak area value of antibody-GlcNAc]/[(Peak area value of antibody-GlcNAc) + (Peak area value of 1-adduct) + (Peak area value of 2-adduct)] × 100

1-Adduct conversion ratio (%) = [Peak area value of 1-adduct]/[(Peak area value of antibody-GlcNAc) + (Peak value area of 1-adduct) + (Peak area value of 2-adduct)] × 100

2-Adduct conversion ratio (%) = [Peak area value of 2-adduct]/[(Peak area value of antibody-GlcNAc) + (Peak area value of 1-adduct) + (Peak area value of 2-adduct)] × 100

Intact mass analysis of each conjugate was also performed. The obtained MS chromatograms were deconvoluted. The result of conjugation with daunorubicin-attached glycan attained with the EndoF3 mutant enzyme is shown in Fig. 7, and the result of conjugation with MMAE-attached glycan attained with the EndoF3 mutant enzyme in Fig. 8.

The activity of EndoS mutant enzyme was also measured in the same manner as that for the EndoF3 mutant enzyme.

The activities of the mutant enzymes for daunorubicin-attached glycan are shown in the following table.

[Table 2]

| Activities of EndoF3 mutant enzyme and EndoS mutant enzyme (for daunorubicin-attached glycan | | | |
|---|---|---|---|
| EndoF3 mutant enzyme | Remaining ratio (%) | Conversion ratio (%) | |
| | Antibody-GlcNAc | 1-Adduct | 2-Adduct |
| D165Q | 48 | 28 | 24 |
| D165Q/V129G | 64 | 25 | 11 |
| D165Q/Q250I | 80 | 17 | 3 |
| D165Q/N270R | 63 | 23 | 14 |
| D165Q/N270L | 80 | 20 | 0 |
| D165Q/N270T | 78 | 22 | 0 |
| D165Q/K81T | 67 | 20 | 13 |
| D165Q/M97W | 60 | 25 | 15 |
| D165Q/M97Y | 67 | 21 | 12 |

(continued)

| Activities of EndoF3 mutant enzyme and EndoS mutant enzyme (for daunorubicin-attached glycan | | | |
|---|---|---|---|
| EndoF3 mutant enzyme | Remaining ratio (%) | Conversion ratio (%) | |
| | Antibody-GlcNAc | 1-Adduct | 2-Adduct |
| D165Q/M97F | 63 | 23 | 14 |
| D165Q/M97V | 57 | 28 | 15 |
| D165Q/M97G | 45 | 25 | 30 |
| D165Q/M97S | 59 | 21 | 20 |
| D165Q/M97Q | 58 | 19 | 23 |
| D165Q/Y282W | 50 | 27 | 23 |
| EndoS mutant enzyme | Remaining ratio (%) | Conversion ratio (%) | |
| | Antibody-GlcNAc | 1-Adduct | 2-Adduct |
| D233D | 64 | 21 | 15 |

Among the EndoF3 mutant enzymes, D165Q/M97G showed the lowest remaining ratio. This mutant enzyme is expected to show similarly high activity even for different glycan structures.

The activities of the mutant enzymes for the MMAE-attached glycan are shown in the following table.

[Table 3]

| Activities of EndoF3 mutant enzyme and EndoS mutant enzyme (for MMAE-attached glycan | | | |
|---|---|---|---|
| EndoF3 mutant enzyme | Remaining ratio (%) | Conversion ratio (%) | |
| | Antibody-GlcNAc | 1-Adduct | 2-Adduct |
| D165Q | 7 | 38 | 55 |
| EndoS mutant enzyme | Remaining ratio (%) | Conversion ratio (%) | |
| | Antibody-GlcNAc | 1-Adduct | 2-Adduct |
| D233Q | 0 | 14 | 86 |

The EndoS mutant enzyme showed high conversion ratio.

[Table 4]

| Enzyme activities for conjugation with simple glycan*1 and linker-attached glycan*2 | | | | |
|---|---|---|---|---|
| Enzyme | Deglycosylated antibody + simple glycan conversion ratio | | Deglycosylated antibody + linker-attached glycan conversion ratio | |
| | 1-Adduct | 2-Adduct | 1-Adduct | 2-Adduct |
| Endo S D233Q | 9 | 91 | 6 | 94 |
| Endo S2 D184M | 35 | 5 | 0 | 0 |
| Endo F3 D165Q | 50 | 30 | 50 | 0 |
| * 1: Glycan having oxazolinized reducing end to which nothing is attached (compound 12 or compound 19 to which no payload was added).<br>*2: Glycan attached with a linker and having oxazolinized reducing end. | | | | |

Although the conversion can also be attained with the EndoS2 mutant enzyme at a certain degree, the EndoF3 mutant enzyme and EndoS mutant enzyme showed higher conversion ratios under the experimental conditions. In addition, it had been expected that the conversion ratio would be reduced in the case of conjugation of glycans to which a payload was added compared with the case of conjugation of glycans having a linker, but contrary to the expectation, higher conversion ratios were obtained in the case of conjugation of glycans with daunorubicin or MMAE.

[Preparation Example 1: Compound 3]

[Formula 6]

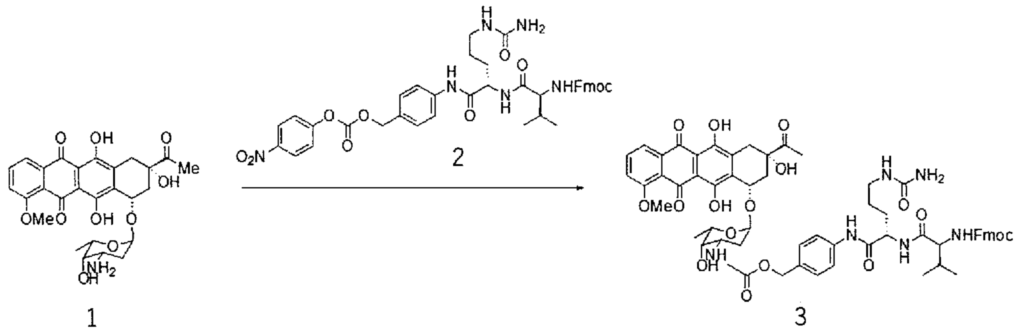

To a solution of the compound 2 (Fmoc-Val-Cit-PAB-PNP, 0.99 g) synthesized by the method of WO2004/010957 in DMF (50 mL), the compound 1 (daunomycin, 0.72 g) and diisopropylethylamine (0.45 mL) were added, and the mixture was stirred at room temperature for 19 hours. The reaction solution was concentrated under reduced pressure, and the resulting residue was washed with chloroform and filtered. The filtration residue was dissolved in DMF, and the solution was filtered and concentrated to obtain the compound 3 (1.43 g).
MS (ESI) m/z: 1155 $(M+H)^+$

[Preparation Example 2: Compound 5]

[Formula 7]

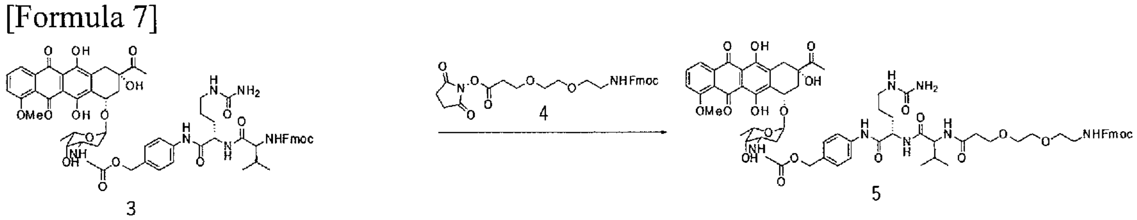

To a solution of the compound 3 (1.43 g) in DMF (20 mL), diethylamine (0.5 mL) was added, and the resulting mixture was stirred at room temperature for 1.5 hours. After concentrating the reaction solution under reduced pressure, the resulting residue was washed with ethyl acetate and filtered to obtain a filtration residue (0.90 g). Of the filtration residue obtained, 0.40 g was dissolved in DMF (10 mL). To this solution, the compound 4 (Fmoc-$PEG_2$-NHS ester, 0.38 g) and diisopropylethylamine (0.15 mL) were added, and the resulting mixture was stirred at room temperature for 17 hours. After concentrating the reaction solution under reduced pressure, the resulting residue was purified by gel filtration chromatography (LH-20, chloroform/methanol = 1/1) to obtain the compound 5 (0.45 g).
MS (ESI) m/z: 1315 $(M+H)^+$

[Preparation Example 3: Compound 7]

[Formula 8]

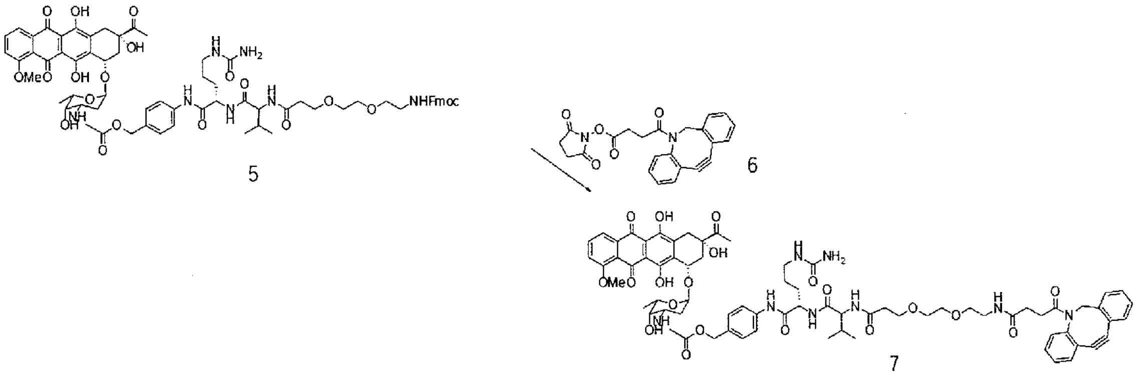

5 6 7

To a solution of the compound 5 (0.45 g) in DMF (15 mL), diethylamine (1.0 mL) was added, and the resulting mixture was stirred at room temperature for 30 minutes. The reaction solution was concentrated under reduced pressure, and the resulting residue was washed with ethyl acetate, and filtered to obtain a filtration residue (0.30 g). Of the filtration residue obtained, 0.15 g was dissolved in DMF (15 mL).
To this solution, the compound 6 (DBCO-NHS ester, 0.06 g) and diisopropylethylamine (0.05 mL) were added, and the resulting mixture was stirred at room temperature for 4 hours. After concentrating the reaction solution under reduced pressure, the resulting residue was purified by gel filtration chromatography (LH-20, chloroform/methanol = 1/1) to obtain the compound 7 (0.05 g).
MS (ESI) m/z: 1380 $(M+H)^+$

[Preparation Example 4: Compound 10]

[Formula 9]

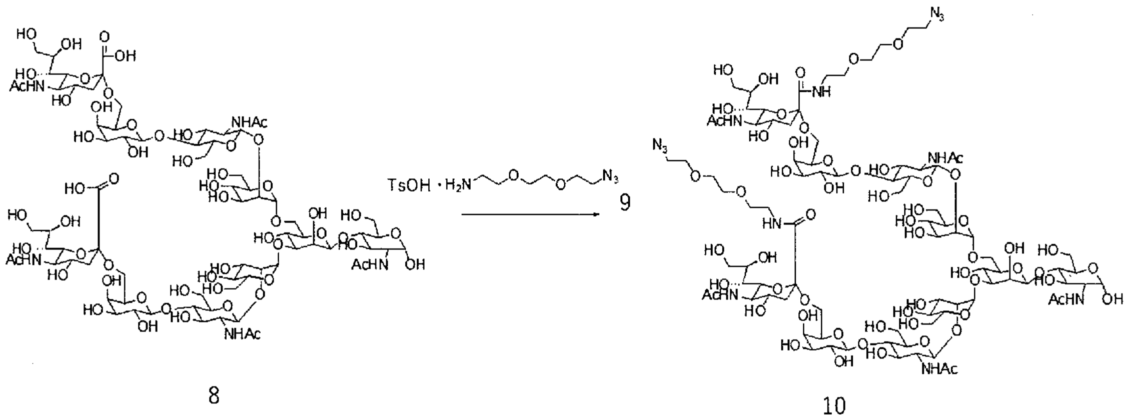

8 9 10

To a solution of the compound 8 (α2,6-sialylglycan, 0.10 g) in DMF (12 mL), the compound 9 (azido-PEG2-amine·Tos-OH, 0.88 g, Tokyo Chemical Industry) and DMT-MM (0.72 g) were added, and the resulting mixture was stirred overnight at 37°C. The reaction solution was purified by gel filtration chromatography (LH-20; methanol). The obtained purified fraction was further purified by silica gel column chromatography (water/acetonitrile) using Wakosil C-18 to obtain the compound 10 (0.11 g).
MS (ESI) m/z: 2333 $(M+H)^+$

[Preparation Example 5: Compound 11]

[Formula 10]

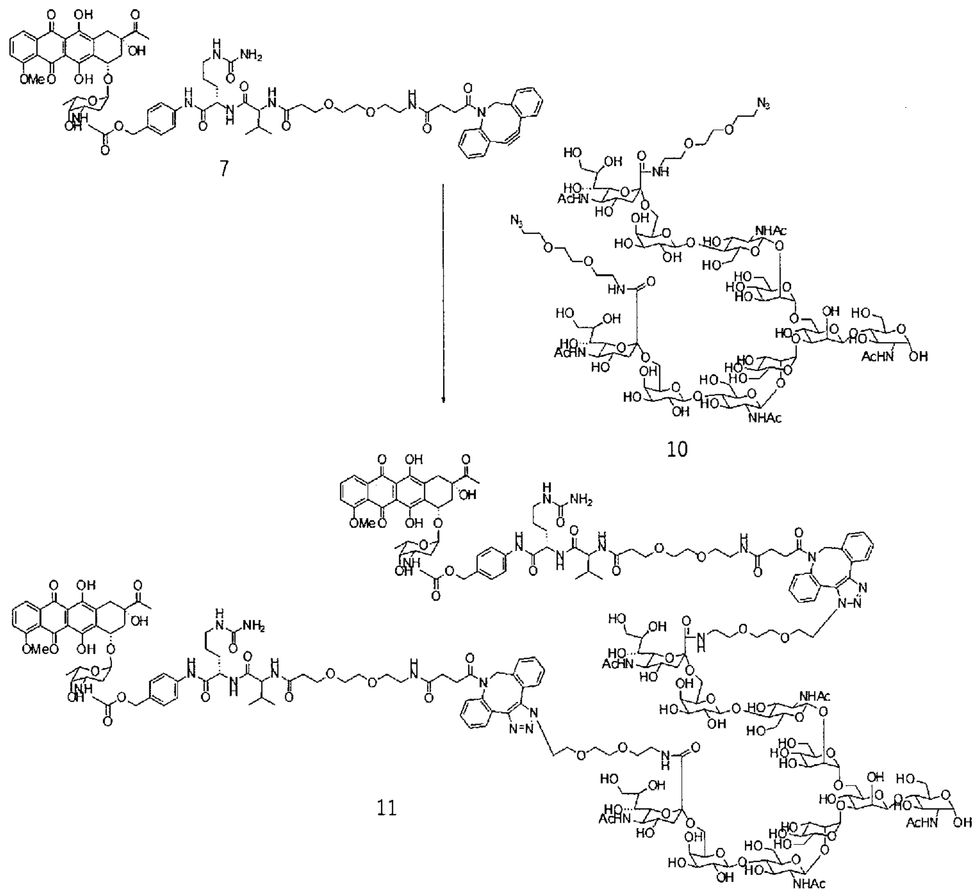

7

10

11

To a solution of the compound 7 (0.05 g) in DMF (10 mL), the compound 10 (0.02 g) was added, and the resulting mixture was stirred overnight at room temperature. The reaction solution was concentrated under reduced pressure, and the resulting residue was purified by preparative HPLC (water/acetonitrile) to obtain the compound 11 (0.03 g).

MS (ESI) m/z: 1698 $(M+3H)^{3+}$, 1274 $(M+4H)^{4+}$

[Preparation Example 6: Compound 12]

[Formula 11]

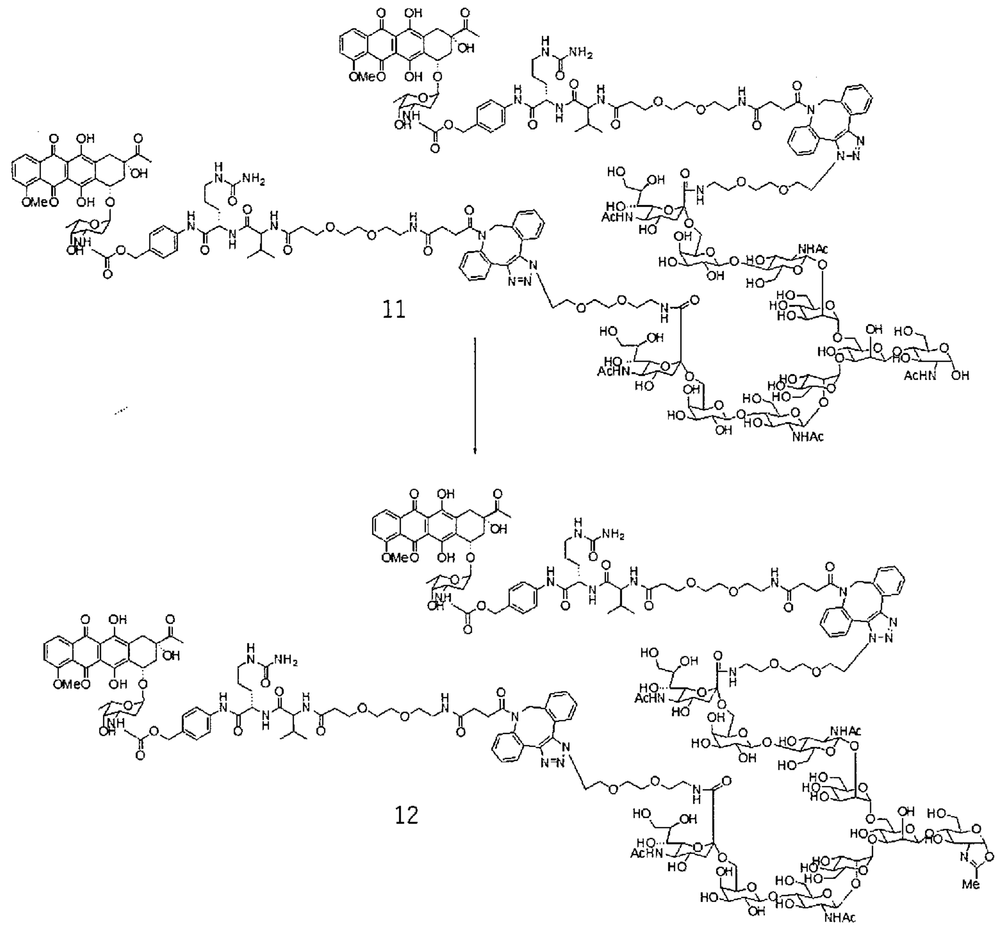

11

12

To a solution of the compound 11 (0.02 g) in a mixture of DMF/water (1.5 mL/1.5 mL), CDMBI (0.02 g) and triethylamine (0.02 mL) were added, and the resulting mixture was stirred at 0°C for 30 minutes. The reaction solution was purified by gel filtration column chromatography (LH-20, DMF) to obtain the compound 12 (0.01 g).

[Example 5: Conjugation of antibody-GlcNAc with daunorubicin-attached glycan]

[Formula 12]

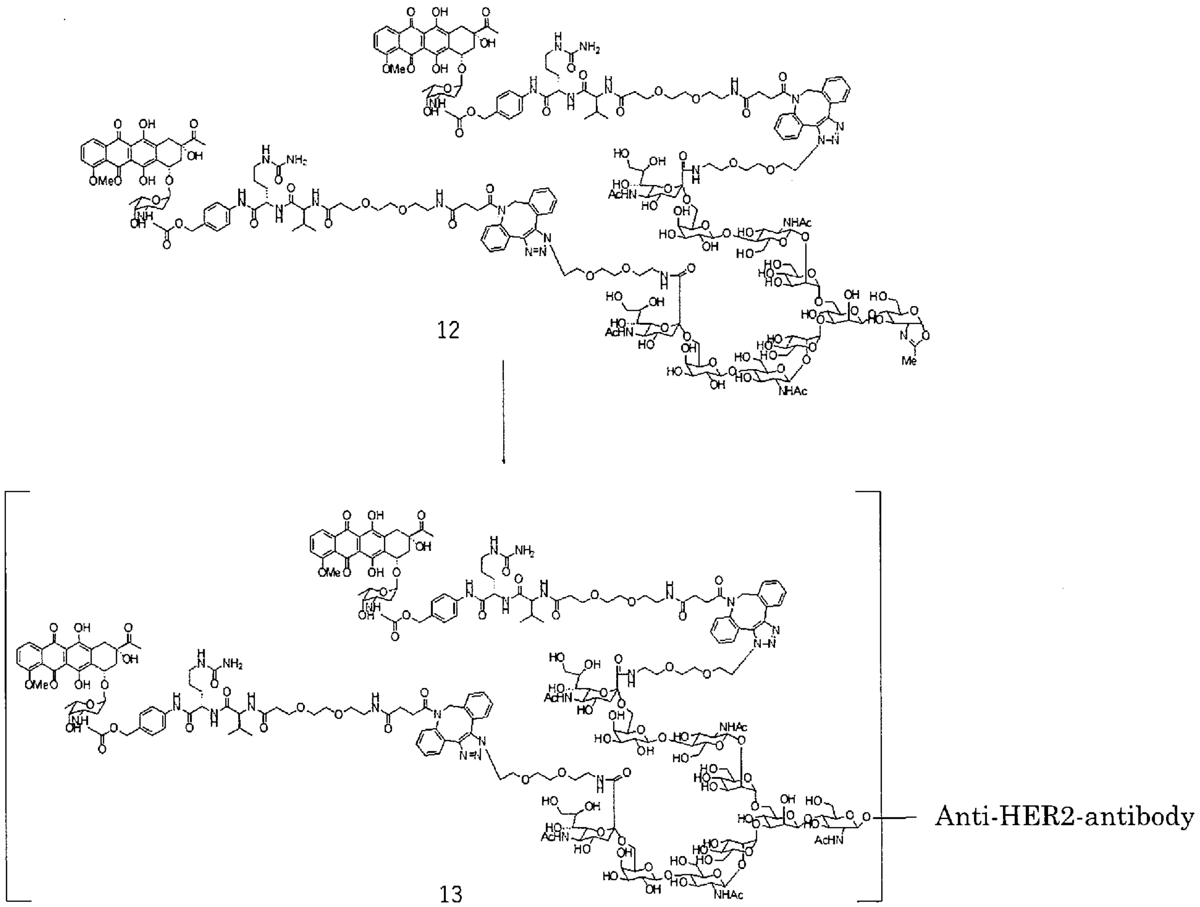


12

13

To an Eppendorf tube, the mutant enzyme (one of the EndoF3 mutant enzymes prepared in Example 1, 0.003 mg) or the EndoS mutant enzyme prepared in Example 2 (0.003 mg)), the antibody-GlcNAc prepared in Example 3 (0.03 mg), and a solution of the compound 12 in DMSO (40 mg/ mL, 0.001 mL) were added, and a 50 mM Tris-HCl buffer, pH 7.4 was further added to the mixture to adjust the antibody-GlcNAc concentration to 5.0 mg/mL. The reaction solution was incubated at 37°C for 3 hours. The reaction solution was subjected to buffer replacement with a 50 mM phosphate buffer, pH7.0 by ultrafiltration (Amicon Ultra 100 kDa), and the obtained compound 13 was analyzed (Figs. 5 and 7).

[Preparation Example 7: Compound 15]

[Formula 13]

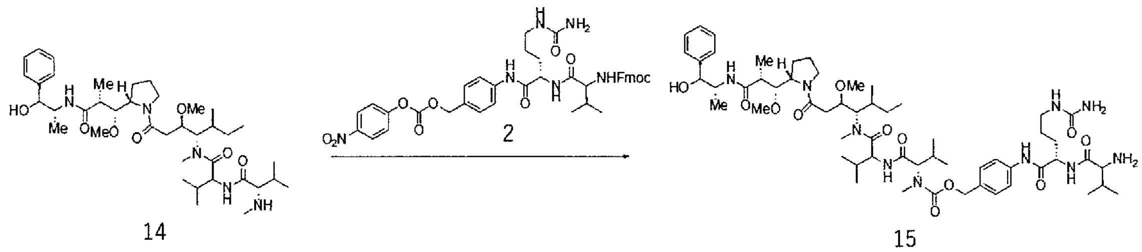

14

2

15

A solution A was prepared by adding HOBt (0.11 g) and diisopropylethylamine (0.08 mL) to DMF (10 mL). The compound 14 (0.22 g, Asta Tech, Inc.) and the compound 2 (0.22 g) were dissolved in this solution A (6.3 mL). This reaction solution was stirred at room temperature for 3 hours. The reaction solution was concentrated under reduced

pressure, and the resulting residue was purified by reprecipitation (chloroform/ethyl acetate) to obtain the compound 15 (0.31 g). MS (ESI) m/z: 1346 (M+H)$^+$

[Preparation Example 8: Compound 16]

[Formula 14]

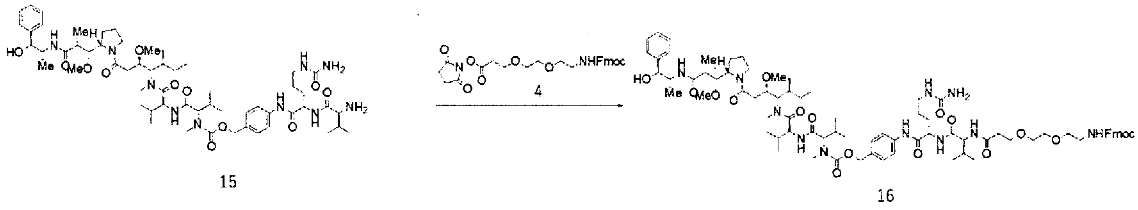

15 4 16

To a solution of the compound 15 (0.31 g) in DMF (4.0 mL), diethylamine (1.0 mL) was added, and the resulting mixture was stirred overnight at room temperature. The reaction solution was concentrated under reduced pressure, and the resulting residue was purified by reprecipitation (chloroform/ethyl acetate) to obtain a yellow solid (0.17 g). Of the solid obtained, 0.11 g was dissolved in the solution A described above (2.5 mL), the compound 4 (0.11 g) was added, and the resulting mixture was stirred at 30°C for 3 hours. The reaction solution was concentrated under reduced pressure, and the resulting residue was purified by reprecipitation (DMF/ethyl acetate) to obtain the compound 16 (0.11 g). MS (ESI) m/z: 1505 (M+H)$^+$.

[Preparation Example 9: Compound 17]

[Formula 15]

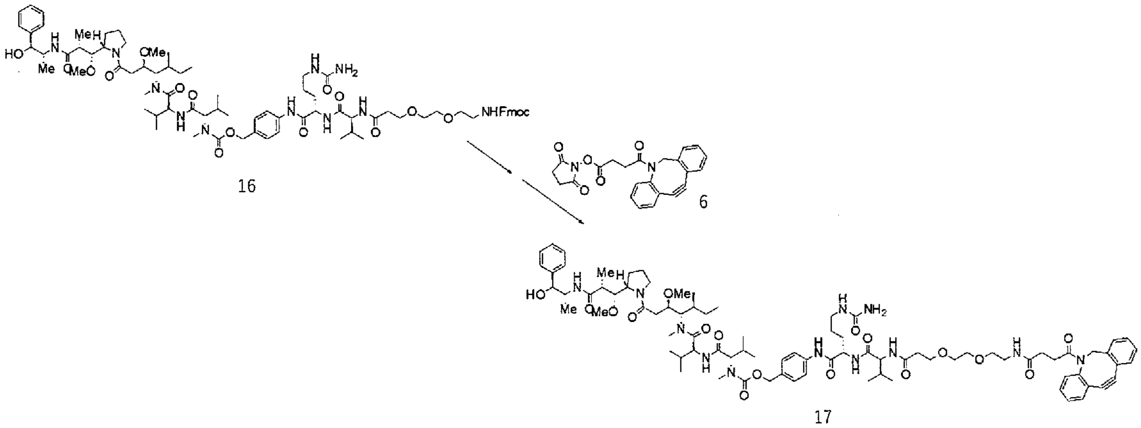

16 6 17

A solution B was prepared with DMF (10 mL), HOAt (0.11 g), and diisopropylethylamine (0.08 mL). The compound 9 (0.09 g) and the compound 6 (0.03 g) were dissolved in this solution B (2.5 mL). This reaction solution was stirred at 30°C for 3 hours. The reaction solution was concentrated under reduced pressure, and the resulting residue was purified by reprecipitation (chloroform/methanol/ether) to obtain the compound 17 (0.11 g). MS (ESI) m/z: 1571 (M+H)$^+$

[Preparation Example 10: Compound 18]

[Formula 16]

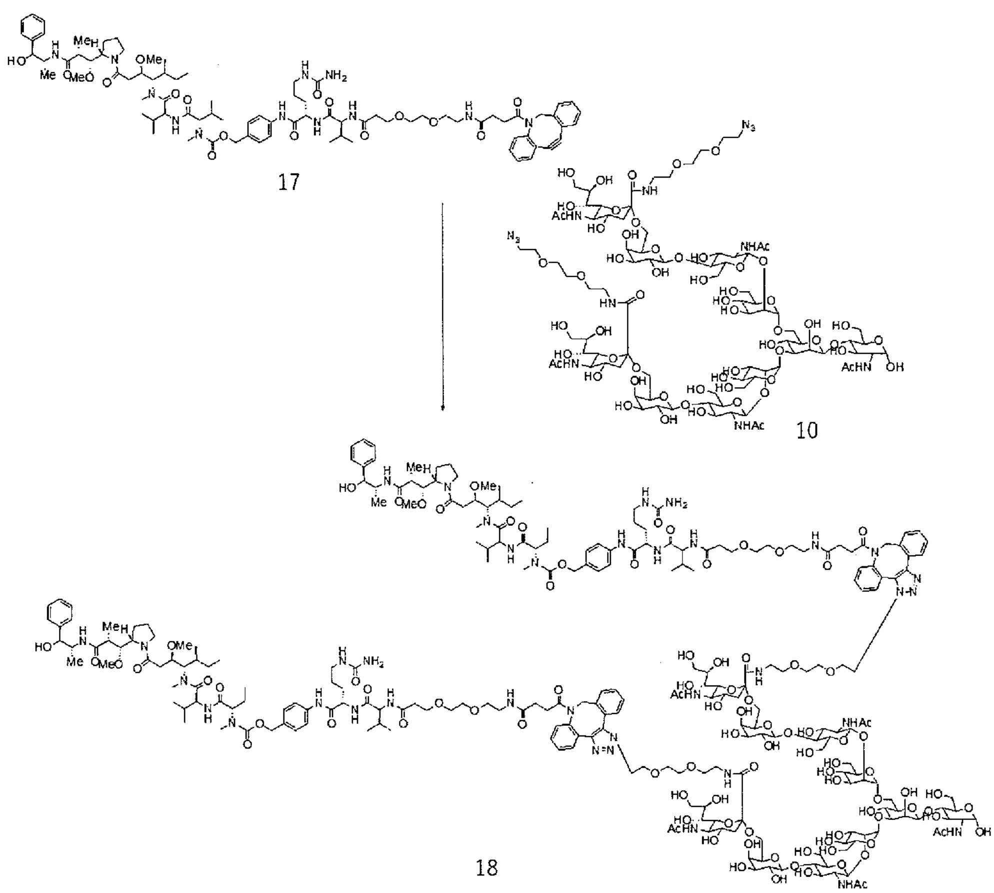

17

10

18

To a solution of the compound 17 (0.07 g) in DMF (1.5 mL), the compound 10 (0.02 g) was added, and the resulting mixture was stirred at 30°C for 3 hours. The reaction solution was concentrated under reduced pressure, and purified by preparative HPLC (methanol/water) to obtain the compound 18 (0.01 g).
MS (ESI) m/z: 1822 $(M+H+H+Na)^{3+}$, 1373 $(M+H+H+Na+Na)^{4+}$

[Preparation Example 11: Compound 19]

[Formula 17]

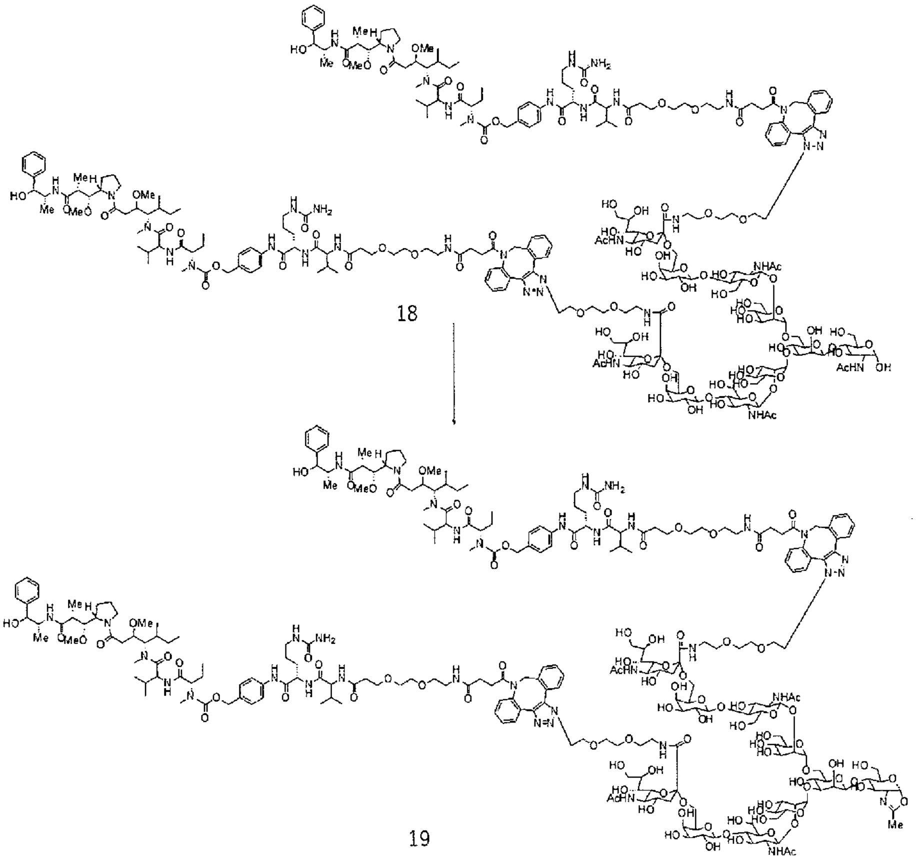

18

19

To a solution of the compound 18 (0.004 g) in a mixture of DMF/water (2.0 mL/2.0 mL), CDMBI (0.01 g) and triethylamine (0.1 mL) were added, and the resulting mixture was stirred at 0°C for 2 hours. This reaction solution was purified by gel filtration chromatography (LH-20, DMF) to obtain the compound 19 (0.005 g).

[Example 6: Conjugation of antibody-GlcNAc with MMAE-attached glycan]

[Formula 18]

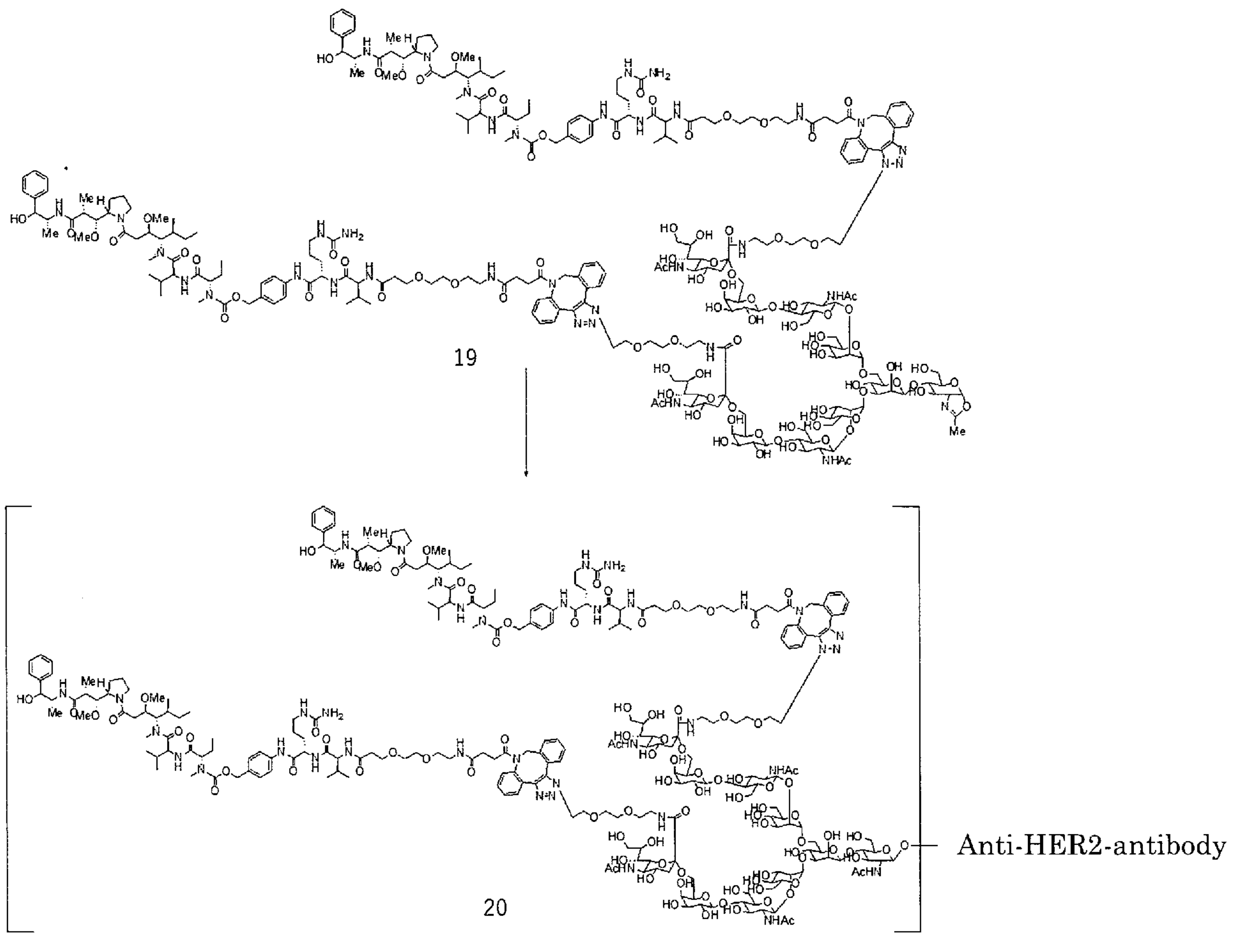


19

20

To an Eppendorf tube, the antibody-GlcNAc obtained in Example 2 (0.03 mg), one of the EndoF3 mutant enzymes prepared in Example 1 (0.003 mg) or EndoS mutant enzyme prepared in Example 2 (0.003 mg), and a solution of the compound 19 in DMSO (40 mg/mL, 0.002 mL) were added, and a 50 mM Tris-HCl buffer, pH 7.4 was further added to the mixture to adjust the antibody-GlcNAc concentration to 5.0 mg/mL. The reaction solution was incubated at 37°C for 1.5 hours. The reaction solution was subjected to buffer replacement with a 50 mM phosphate buffer, pH 7.0 by ultrafiltration (Amicon Ultra 100 kDa), and the compound 20 was analyzed (Figs. 6 and 8).

[Preparation Example 12: Compound 23]

[Formula 19]

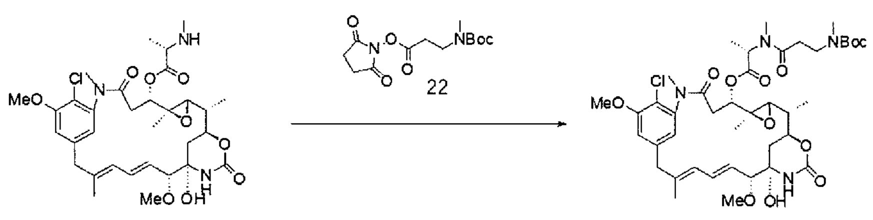

22

21

23

To a solution of the compound 21 (0.1 g) prepared from maytansinol as the starting material according to the descriptions of WO2014/145090 in a mixture of acetonitrile/water (6.0 mL/2.0 mL), the compound 22 (0.3 g) prepared according to the descriptions of WO2014/145090 and 1 M aqueous sodium hydrogencarbonate (0.5 mL) were added, and the resulting mixture was stirred at room temperature for 23 hours. This reaction solution was extracted with ethyl

acetate, and the organic layer was dried over magnesium sulfate. The organic layer was concentrated under reduced pressure, and the resulting residue was purified by silica gel column chromatography (ethyl acetate/methanol) to obtain the compound 23 (0.08 g).
MS (ESI) m/z: 835 $(M+H)^+$.

[Preparation Example 13: Compound 24]

[Formula 20]

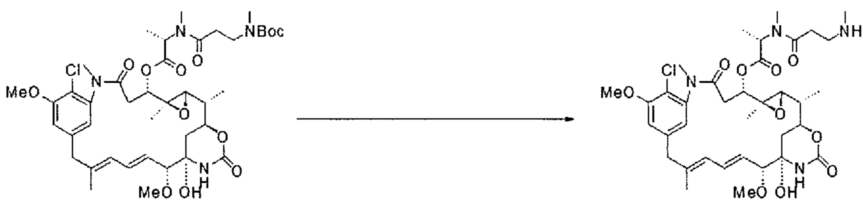

23

24

To a solution of the compound 23 (0.04 g) in a mixture of acetonitrile/water (3.0 mL/1.0 mL), TFA (1.0 mL) was added, and the resulting mixture was stirred at room temperature for 30 minutes. To this reaction solution, saturated aqueous sodium hydrogencarbonate was added, the mixture was extracted with a mixture of chloroform/methanol (7/1), and the organic layer was dried over magnesium sulfate. The organic layer was concentrated under reduced pressure to obtain the compound 24 (0.04 g).
MS (ESI) m/z: 735 $(M+H)^+$

[Preparation Example 14: Compound 26]

[Formula 21]

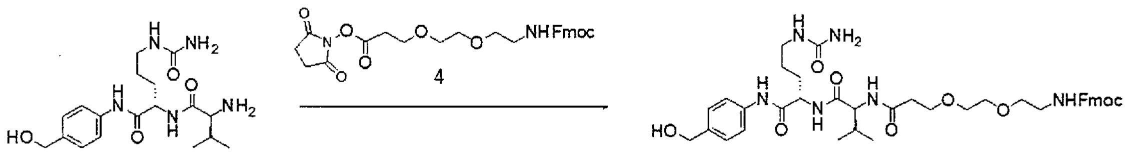

25

26

To a solution of the compound 25 (0.07 g, MedChemExpress) in DMF (5.0 mL), the compound 4 (0.07 g) and diisopropylethylamine (0.04 mL) were added, and the resulting mixture was stirred at room temperature for 3 hours. This reaction solution was concentrated under reduced pressure, and the resulting residue was purified by reprecipitation (DMF/ether) to obtain the compound 26 (0.07 g).
MS (ESI) m/z: 761 $(M+H)^+$

[Preparation Example 15: Compound 27]

[Formula 22]

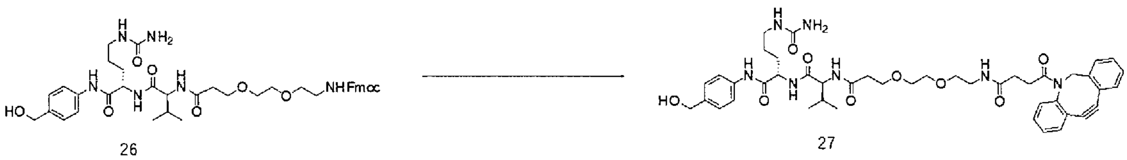

26

27

To a solution of the compound 26 (0.07 g) in DMF (5.0 mL), diethylamine (0.5 mL) was added, and the resulting mixture was stirred at room temperature for 1 hour. The reaction solution was concentrated under reduced pressure, the resulting residue was dissolved in DMF (5.0 mL), DBCO-NHS (0.04 g) and diisopropylethylamine (0.04 mL) were added, and the resulting mixture was stirred overnight at room temperature. The reaction solution was concentrated under reduced pressure, and the resulting residue was purified by reprecipitation (DMF/ether) to obtain the compound 27 (0.07 g).
MS (ESI) m/z: 826 $(M+H)^+$

[Preparation Example 16: Compound 28]

[Formula 23]

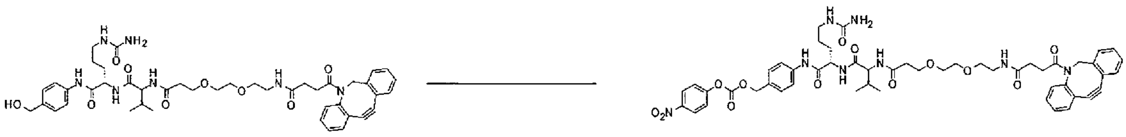

27 28

To a solution of the compound 27 (0.07 g) in DMF (5.0 mL), bis(4-nitrophenyl) carbonate (0.03 g) and diisopropylethylamine (0.04 mL) were added, and the resulting mixture was stirred at room temperature for 3 hours. The reaction solution was concentrated under reduced pressure, and the resulting residue was purified by reprecipitation (DMF/ether) to obtain the compound 28 (0.07 g).
MS (ESI) m/z: 991 $(M+H)^+$

[Preparation Example 17: Compound 29]

[Formula 24]

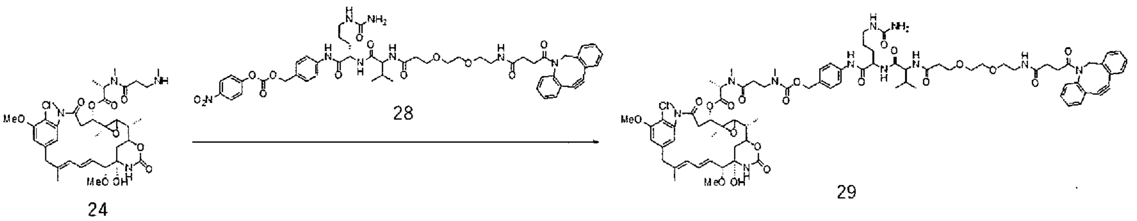

28

24 29

To a solution of the compound 24 (0.008 g) in DMF (0.9 mL), the compound 28 (0.01 g) and triethylamine (0.008 mL) were added, and the resulting mixture was stirred at room temperature for 3 hours. The reaction solution was concentrated under reduced pressure, and the resulting residue was purified by preparative HPLC (acetonitrile/water) to obtain the compound 29 (0.011 g).
MS (ESI) m/z: 1587 $(M+H)^+$

[Preparation Example 18: Compound 30]

[Formula 25]

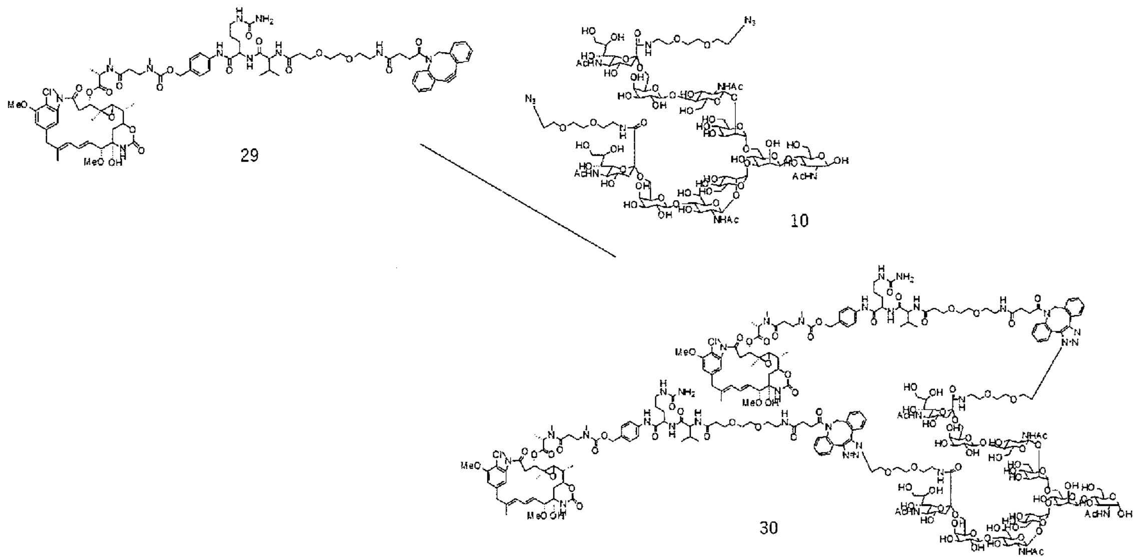

29 10 30

To a solution of the compound 29 (0.008 g) in DMF (1.6 mL), the compound 10 (0.002 g) was added, and the resulting mixture was stirred overnight at 50°C. The reaction solution was purified by gel filtration chromatography (LH-20, DMF) to obtain the compound 30 (0.004 g).
MS (ESI) m/z: 1837 $(M+3H)^{3+}$, 1378 $(M+4H)^{4+}$

[Preparation Example 19: Compound 31]

[Formula 26]

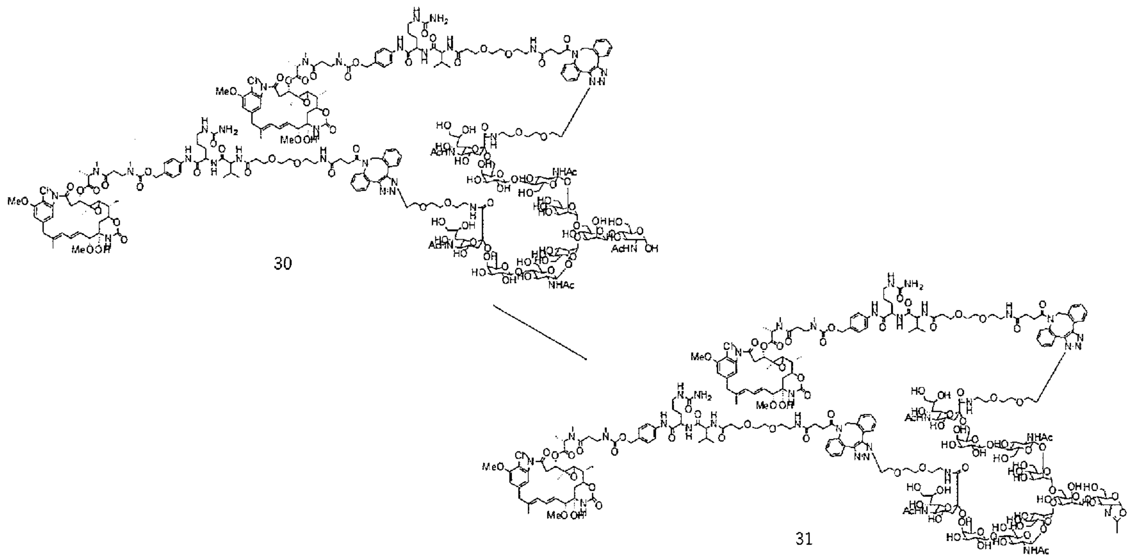

30 31

To a solution of the compound 30 (0.002 g) in a mixture of DMF/water (0.5 mL/0.5 mL), CDMBI (0.04 g) and triethylamine (0.002 mL) were added, and the resulting mixture was stirred at 0°C for 1 hour. This reaction solution was purified by gel filtration chromatography (LH-20, DMF) to obtain the compound 31 (0.003 g).

[Example 7: Conjugation of antibody-GlcNAc with maytansinoid-attached glycan]

[Formula 27]

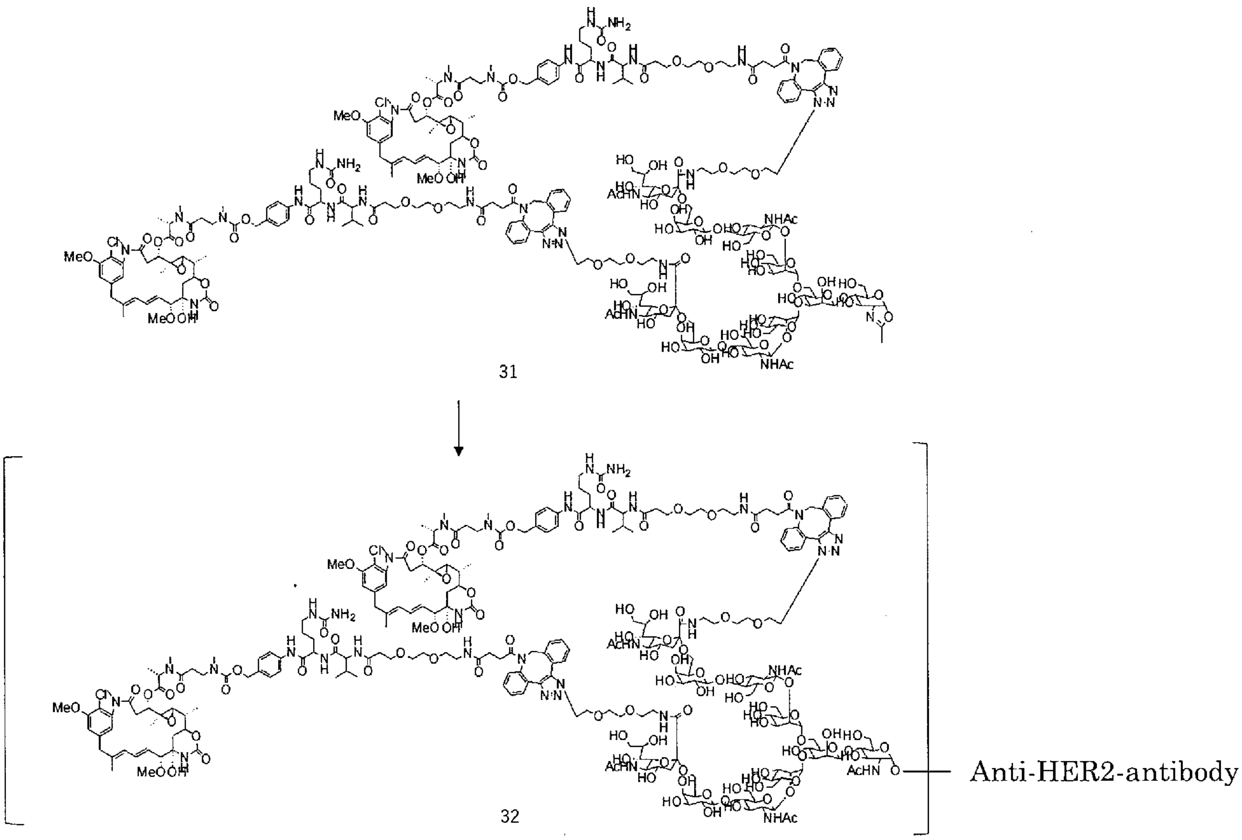


31

32

To an Eppendorf tube, the antibody-GlcNAc obtained in Example 2 (0.03 mg), the EndoF3 mutant enzymes prepared in Example 1 (0.003 mg), or EndoS mutant enzyme prepared in Example 2 (0.003 mg), or WP1328 enzyme prepared in Example 12 (0.003 mg), and a solution of the compound 31 in DMSO (40 mg/mL, 0.002 mL) were added, and a 50 mM Tris-HCl buffer, pH 7.4 was further added to the mixture to adjust the antibody-GlcNAc concentration to 5.0 mg/mL. The reaction solution was incubated at 30°C or 37°C for 1.5 to 3.0 hours. The reaction solution was subjected to buffer replacement with a 50 mM phosphate buffer, pH 7.0 by ultrafiltration (Amicon Ultra 100 kDa), and the compound 32 was analyzed (Figs. 13 and 14).

[Preparation Example 20: Compound 34]

[Formula 28]

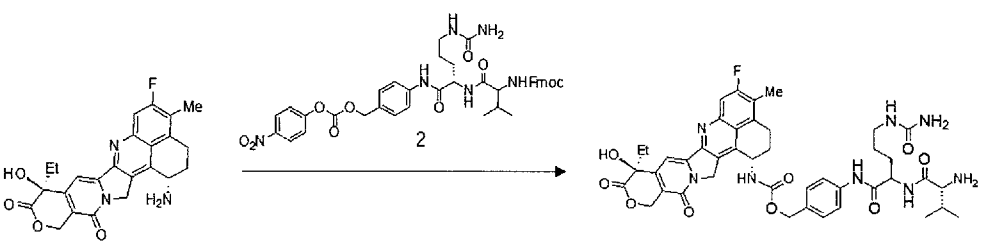

33

2

34

To a solution of the compound 33 (0.07 g, MedChemExpress) in DMF (5.0 mL), the compound 2 (0.04 g), diisopropylethylamine (0.04 mL), and HOBt (0.02 g) were added, and the resulting mixture was stirred at 30°C for 3 hours. The reaction solution was concentrated under reduced pressure, and the resulting residue was purified by reprecipitation (DMF/ether). To the resulting purple solid, DMF (5.0 mL) and diethylamine (0.1 mL) were added, and the resulting mixture was stirred at room temperature for 1 hour. The reaction solution was concentrated under reduced pressure, and the resulting residue was purified by reprecipitation (DMF/ether) and further purified by gel filtration chromatography (LH-20, DMF) to obtain the compound 34 (0.09 g).
MS (ESI) m/z: 841 $(M+H)^+$

[Preparation Example 21: Compound 35]

[Formula 29]

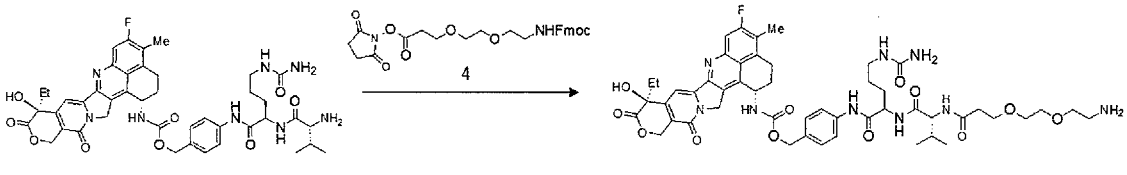


34 35

To a solution of the compound 34 (0.09 g) in DMF (5.0 mL), the compound 4 (0.03 g), diisopropylethylamine (0.02 mL) and HOBt (0.01 g) were added, and the resulting mixture was stirred at 30°C for 3 hours. The reaction solution was concentrated under reduced pressure, and the resulting residue was purified by reprecipitation (DMF/ether). To the resulting purple solid, DMF (5.0 mL) and diethylamine (0.1 mL) were added, and the resulting mixture was stirred at room temperature for 1 hour. The reaction solution was concentrated under reduced pressure, and the resulting residue was purified by reprecipitation (DMF/ether) and further purified by gel filtration chromatography (LH-20, DMF) to obtain the compound 35 (0.07 g).
MS (ESI) m/z: 1001 $(M+H)^+$

[Preparation Example 22: Compound 36]

[Formula 30]

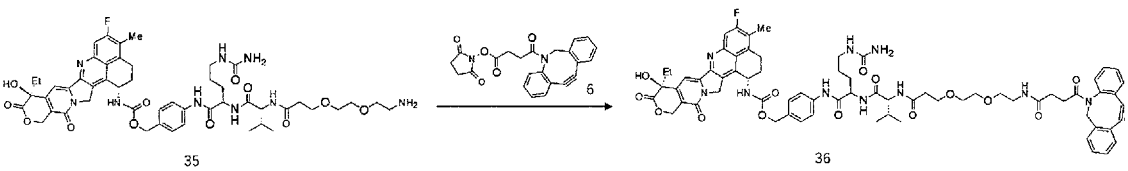


35 36

To a solution of the compound 35 (0.07 g) in DMF (5.0 mL), the compound 6 (DBCO-NHS ester, 0.03 g), diisopropylethylamine (0.01 mL), and HOBt (0.01 g) were added, and the resulting mixture was stirred at 30°C for 1 hour. The reaction solution was concentrated under reduced pressure, and the resulting residue was purified by reprecipitation (DMF/ether) to obtain the compound 36 (0.05 g).
MS (ESI) m/z: 1288 $(M+H)^+$

[Preparation Example 23: Compound 37]

[Formula 31]

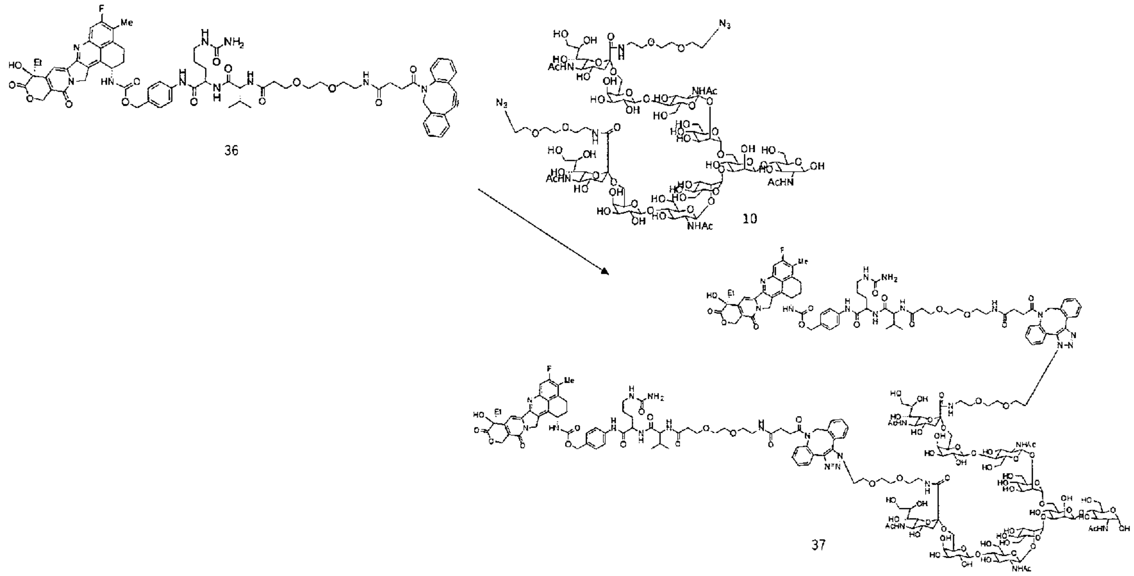

36

10

37

To a solution of the compound 36 (0.009 g) in DMF (3.0 mL), the compound 10 (0.005 g) was added, and the resulting mixture was stirred at 40°C for 2 hours. The reaction solution was concentrated under reduced pressure, and the resulting residue was purified by preparative HPLC (acetonitrile/water) to obtain the compound 37 (0.003 g).
MS (ESI) m/z: 1637 $(M+3H)^{3+}$, 1228 $(M+4H)^{4+}$

[Preparation Example 24: Compound 38]

[Formula 32]

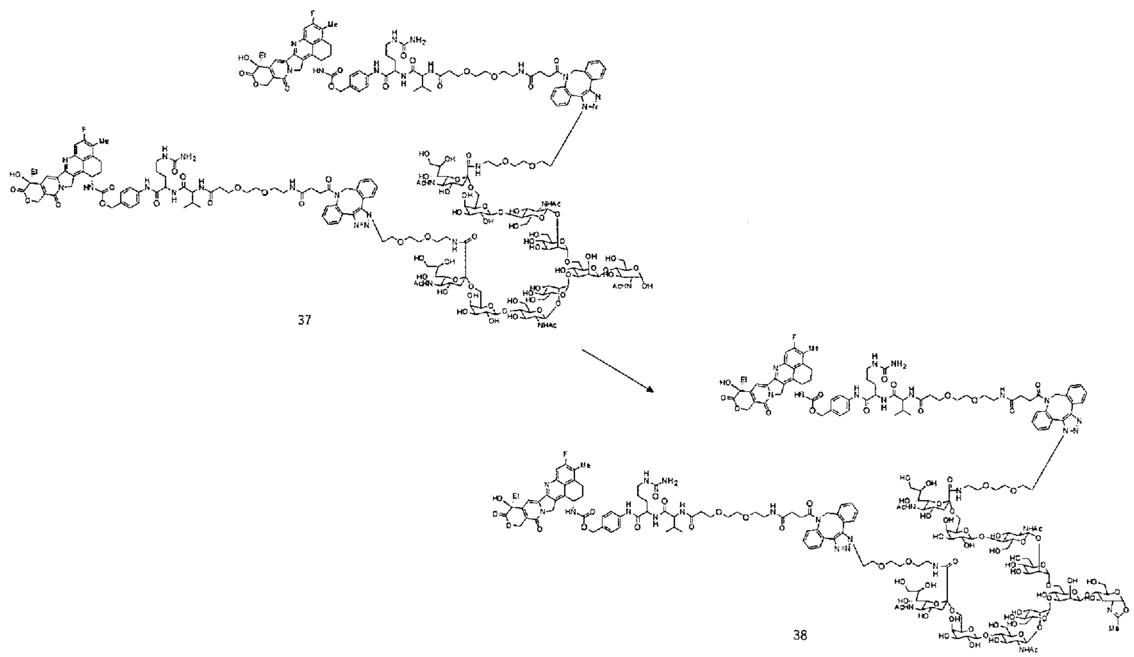

37

38

To a solution of the compound 37 (0.003 g) in a mixture of DMF/water (1.0 mL/1.0 mL), CDMBI (0.007 g) and triethylamine (0.001 mL) were added, and the resulting mixture was stirred at 0°C for 30 minutes. This reaction solution was purified by gel filtration chromatography (LH-20, DMF) to obtain the compound 38 (0.003 g).

[Example 8: Conjugation of antibody-GlcNAc with exatecan-attached glycan]

[Formula 33]

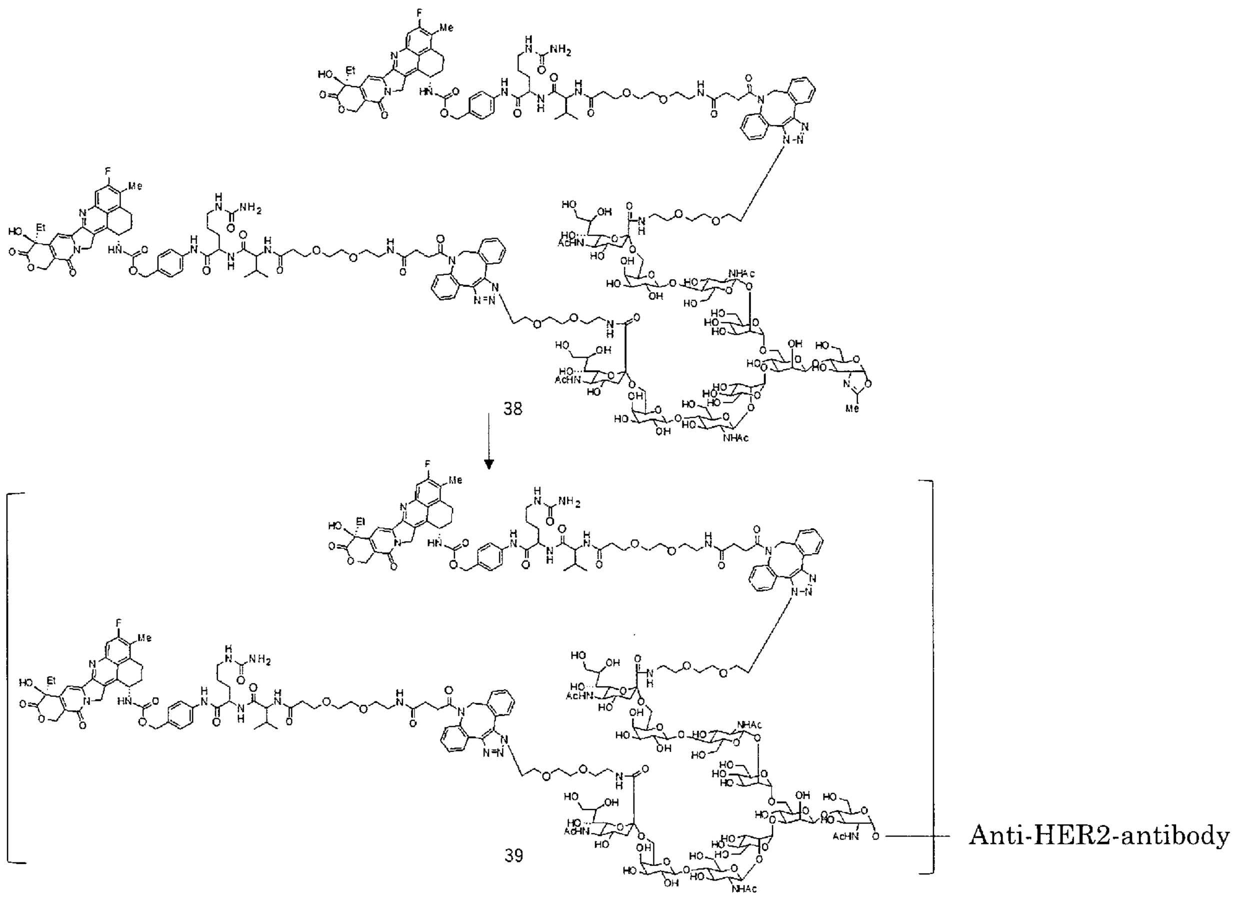

38

39

To an Eppendorf tube, the antibody-GlcNAc obtained in Example 2 (0.03 mg), one of the EndoF3 mutant enzymes prepared in Example 1 (0.003 mg), EndoS mutant enzyme prepared in Example 2 (0.003 mg), or WP1328 enzyme prepared in Example 12 (0.003 mg), and a solution of the compound 38 in DMSO (40 mg/mL, 0.002 mL) were added, and a 50 mM Tris-HCl buffer, pH 7.4 was further added to the mixture to adjust the antibody-GlcNAc concentration to 5.0 mg/mL. The reaction solution was incubated at 30°C or 37°C for 1.5 to 3.0 hours. The reaction solution was subjected to buffer replacement with a 50 mM phosphate buffer, pH 7.0 by ultrafiltration (Amicon Ultra 100 kDa), and the compound 39 was analyzed (Figs. 15 and 16).

[Preparation Example 25: Compound 41]

[Formula 34]

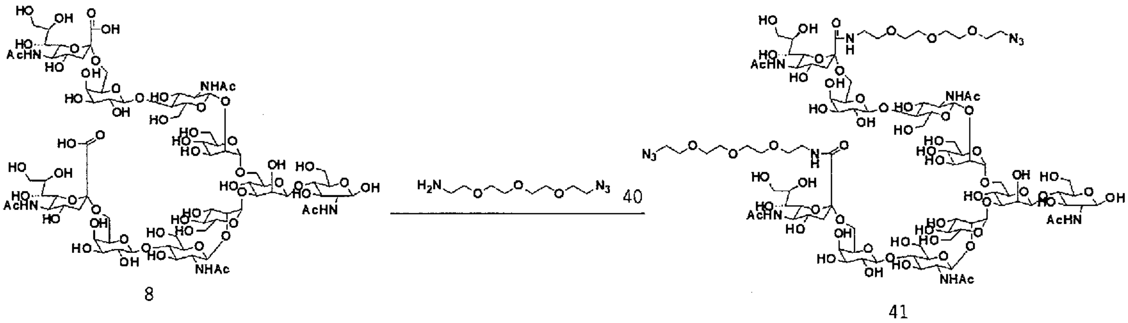

8 40 41

To a solution of the compound 8 (0.05 g) in DMF (6.0 mL), the compound 40 (azido-PEG3-amine, 0.25 g, Combi-Blocks) and DMT-MM (0.34 g) were added, and the resulting mixture was stirred overnight at 37°C. The reaction solution was purified by gel filtration chromatography (LH-20, methanol). The obtained purified fraction was further purified by silica gel column chromatography (water/acetonitrile) using Wakosil C-18 to obtain the compound 41 (0.02 g). MS (ESI) m/z: 2422 $(M+H)^+$

[Preparation Example 26: Compound 42]

[Formula 35]

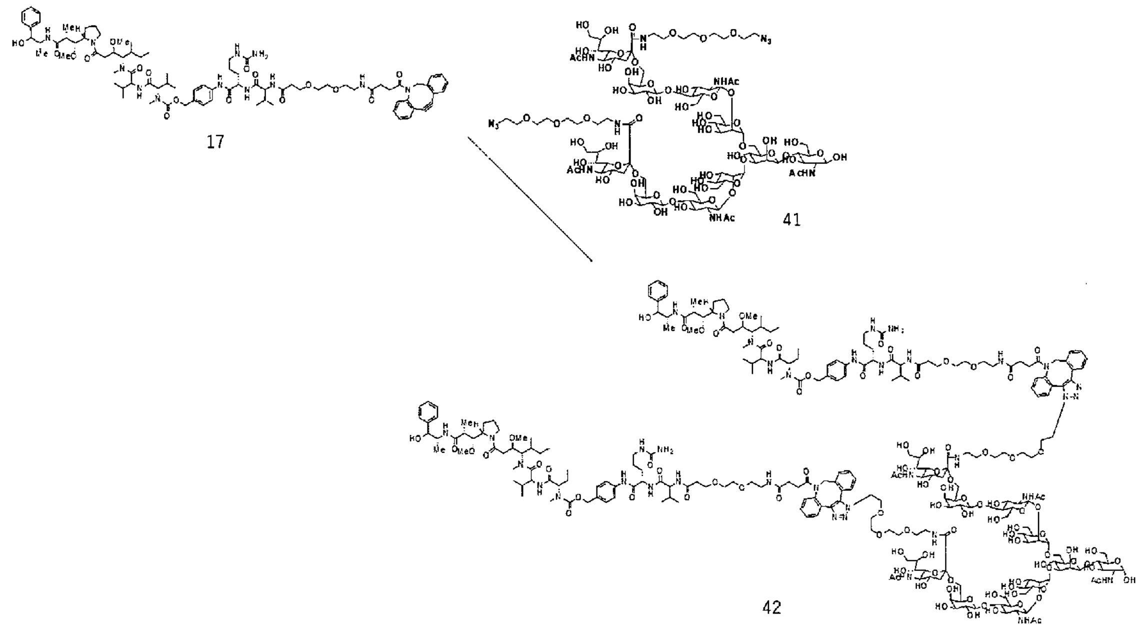

17 41 42

To a solution of the compound 17 (0.02 g) in DMF (3.0 mL), the compound 41 (0.006 g) was added, and the resulting mixture was stirred at room temperature for 22 hours. The reaction solution was concentrated under reduced pressure, and the resulting residue was purified by preparative HPLC (acetonitrile/water) to obtain the compound 42 (0.004 g).

MS (ESI) m/z: 1855 $(M+3H)^{3+}$, 1391 $(M+4H)^{4+}$

[Preparation Example 27: Compound 43]

[Formula 36]

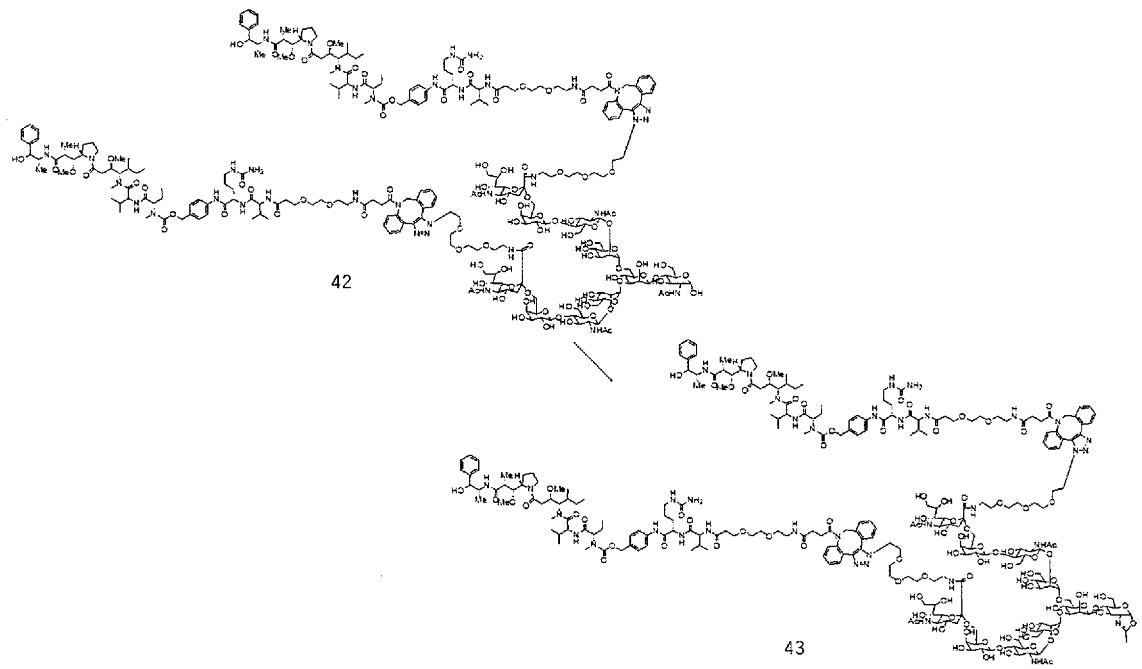

42

43

To a solution of the compound 42 (0.004 g) in a mixture of DMF/water (1.0 mL/1.0 mL), CDMBI (0.008 g) and triethylamine (0.001 mL) were added, and the resulting mixture was stirred at 0°C for 30 minutes. This reaction solution was purified by gel filtration chromatography (LH-20, DMF) to obtain the compound 43 (0.004 g).

[Example 9: Conjugation of antibody-GlcNAc with MMAE-PEG3-attached glycan]

[Formula 37]

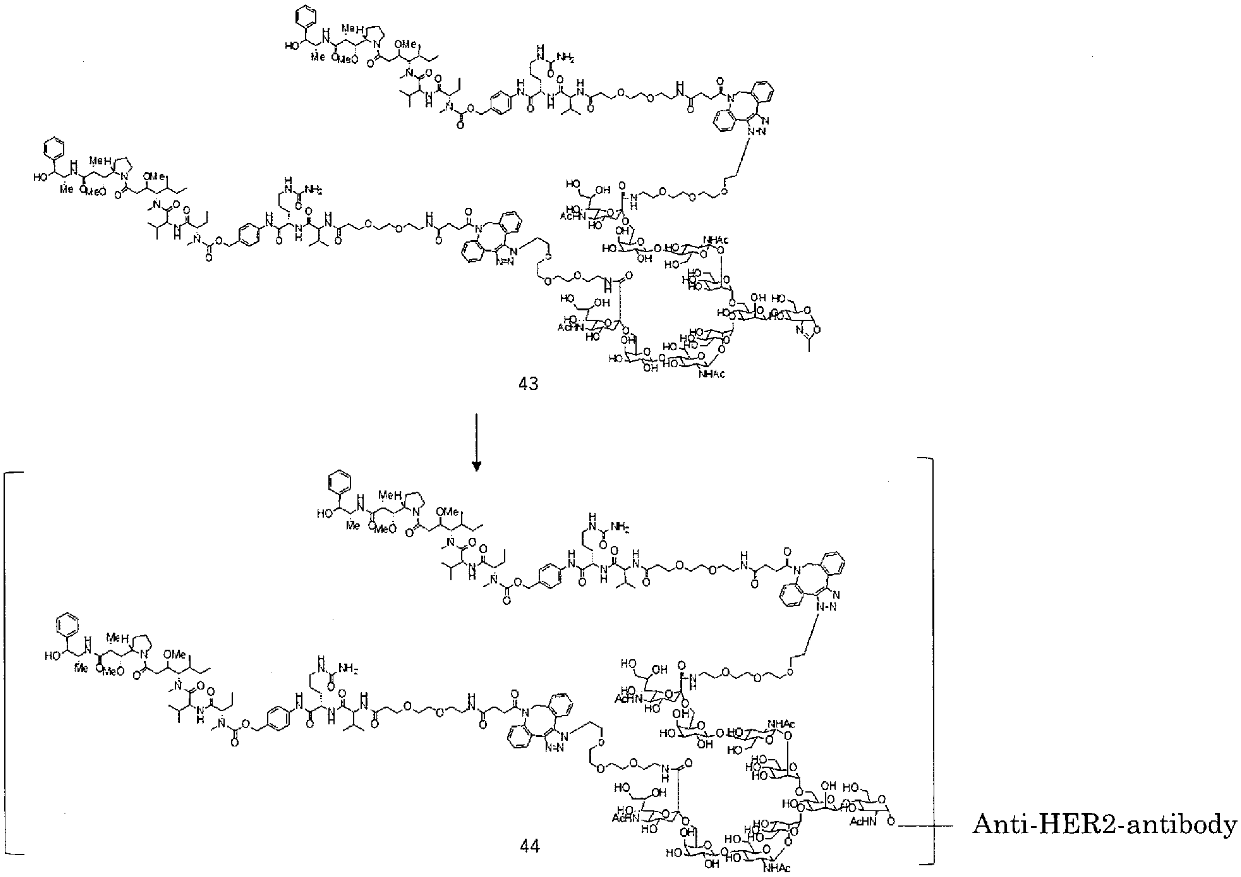

43

44

To an Eppendorf tube, the antibody-GlcNAc obtained in Example 2 (0.03 mg), one of the EndoF3 mutant enzymes prepared in Example 1 (0.003 mg), EndoS mutant enzyme prepared in Example 2 (0.003 mg), or WP1328 enzyme prepared in Example 12 (0.003 mg), and a solution of the compound 43 in DMSO (40 mg/mL, 0.002 mL) were added, and a 50 mM Tris-HCl buffer, pH 7.4 was further added to the mixture to adjust the antibody-GlcNAc concentration to 5.0 mg/mL. The reaction solution was incubated at 30°C or 37°C for 1.5 to 3.0 hours. The reaction solution was subjected to buffer replacement with a 50 mM phosphate buffer, pH 7.0 by ultrafiltration (Amicon Ultra 100 kDa), and the compound 44 was analyzed (Figs. 17, 18, and 19).

[Preparation Example 28: Compound 46]

[Formula 38]

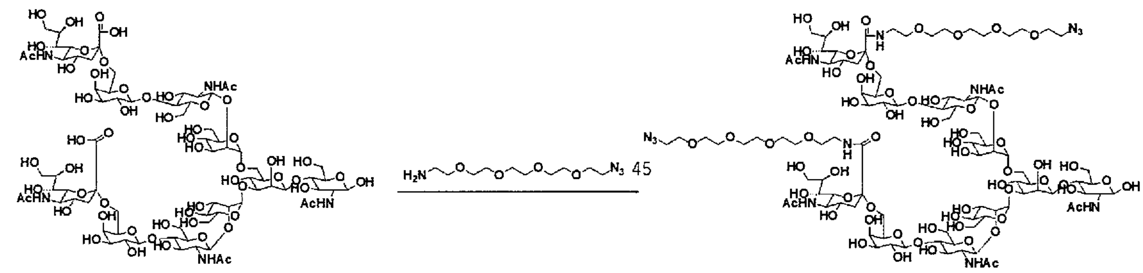


8

46

To a solution of the compound 8 (0.05 g) in DMF (6 mL), the compound 45 (azido-PEG4-amine, 0.30 g, Combi-Blocks) and DMT-MM (0.34 g) were added, and the resulting mixture was stirred overnight at 37°C. The reaction solution

was purified by gel filtration chromatography (LH-20, methanol). The obtained purified fraction was further purified by silica gel column chromatography (water/acetonitrile) using Wakosil C-18 to obtain the compound 46 (0.02 g).
MS (ESI) m/z: 1256 $(M+2H)^{2+}$, 837 $(M+3H)^{3+}$

[Preparation Example 29: Compound 47]

[Formula 39]

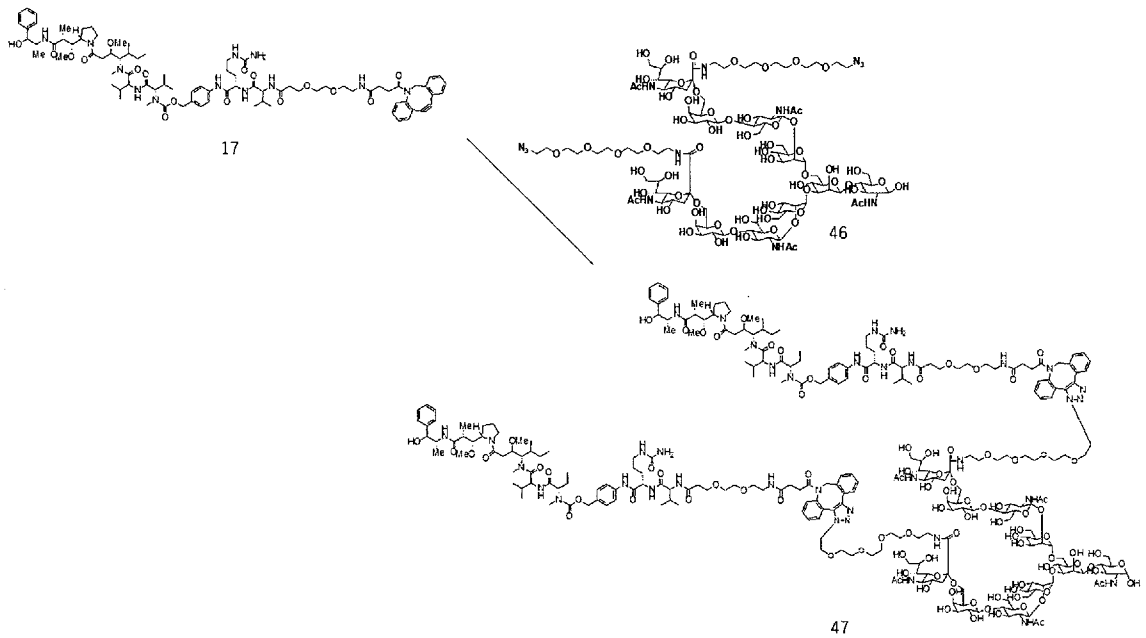

17

46

47

To a solution of the compound 17 (0.02 g) in DMF (3.0 mL), the compound 41 (0.006 g) was added, and the resulting mixture was stirred at room temperature for 17 hours. The reaction solution was concentrated under reduced pressure, and the resulting residue was purified by preparative HPLC (acetonitrile/water) to obtain the compound 47 (0.007 g).
MS (ESI) m/z: 1884 $(M+3H)^{3+}$, 1413 $(M+4H)^{4+}$

[Preparation Example 30: Compound 48]

[Formula 40]

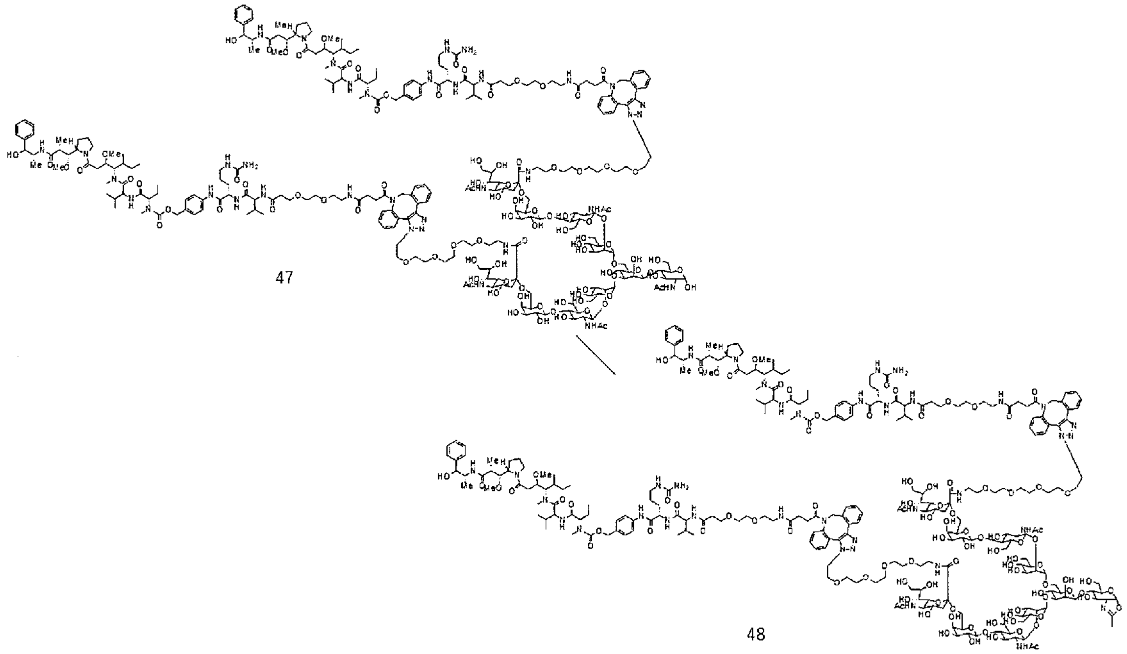

47

48

To a solution of the compound 47 (0.004 g) in a mixture of DMF/water (0.5 mL/0.5 mL), CDMBI (0.008 g) and triethylamine (0.001 mL) were added, and the resulting mixture was stirred at 0°C for 30 minutes. This reaction solution was purified by gel filtration chromatography (LH-20, DMF) to obtain the compound 48 (0.004 g).

[Example 10: Conjugation of antibody-GlcNAc with MMAE-PEG4-attached glycan]

[Formula 41]

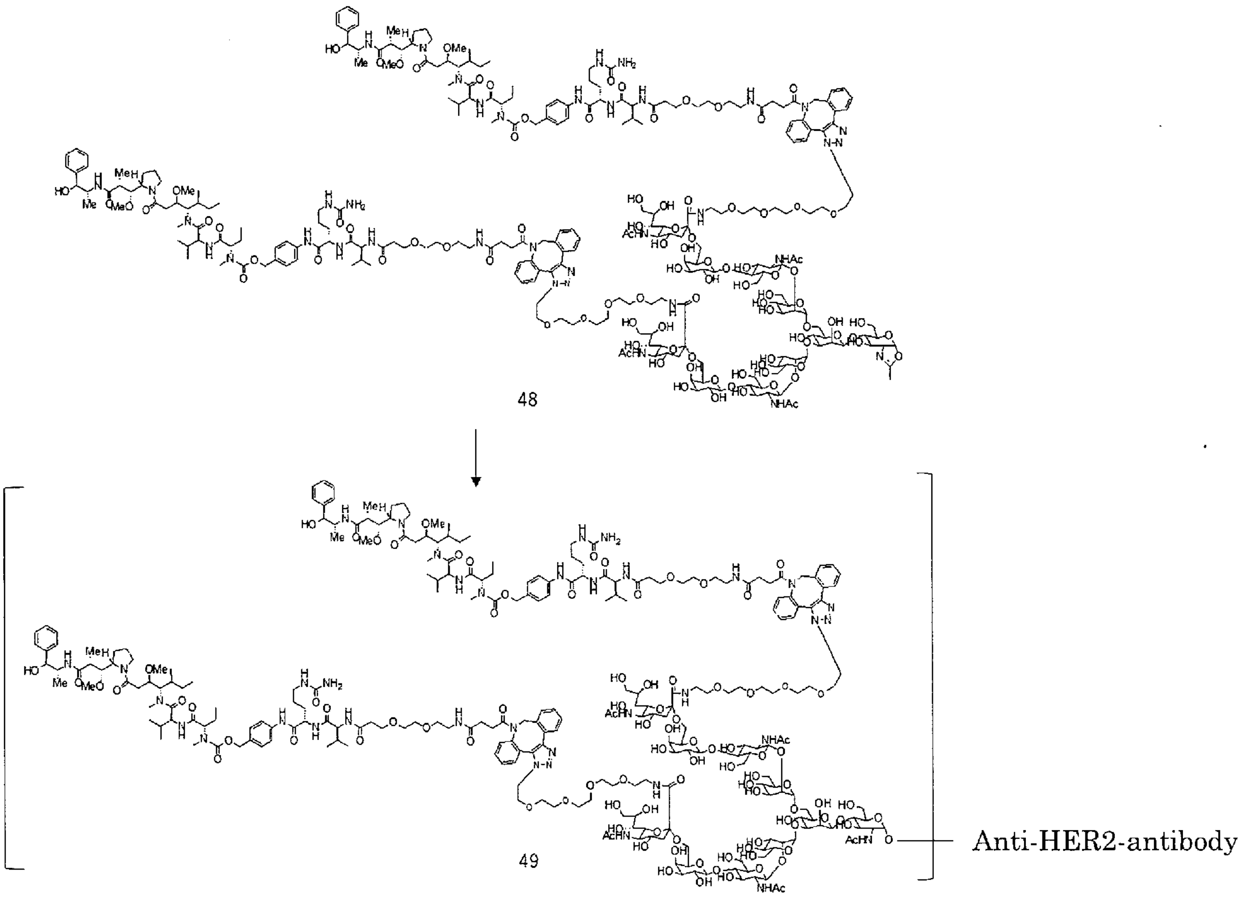


48

49

To an Eppendorf tube, the antibody-GlcNAc obtained in Example 2 (0.03 mg), one of the EndoF3 mutant enzymes prepared in Example 1 (0.003 mg), EndoS mutant enzyme prepared in Example 2 (0.003 mg), or WP1328 enzyme prepared in Example 12 (0.003 mg), and a solution of the compound 48 in DMSO (40 mg/mL, 0.002 mL) were added, and a 50 mM Tris-HCl buffer, pH 7.4 was further added to the mixture to adjust the antibody-GlcNAc concentration to 5.0 mg/mL. The reaction solution was incubated at 30°C or 37°C for 1.5 to 3.0 hours. The reaction solution was subjected to buffer replacement with a 50 mM phosphate buffer, pH 7.0 by ultrafiltration (Amicon Ultra 100 kDa), and the compound 49 was analyzed (Figs. E and G).

[Preparation Example 31: Compound 52]

[Formula 42]

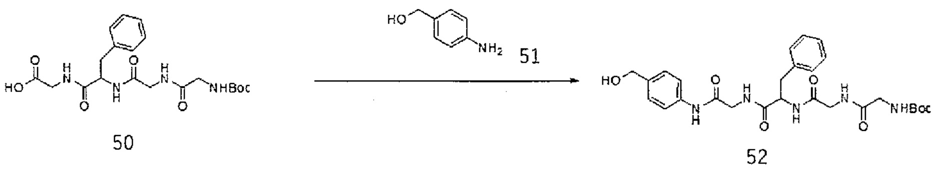

51

50

52

To a solution of the compound 50 (Boc-GGFG-OH, 0.2 g, Broadpharm) in DMF (2.0 mL), the compound 51 (4-aminobenzyl alcohol, 0.1 g), diisopropylethylamine (0.3 mL), and HATU (0.2 g, Watanabe Chemical) were added, and the resulting mixture was stirred at room temperature for 4 hours. To the reaction solution, 0.1 M hydrochloric acid was added, and the resulting mixture was extracted with a mixed solvent of chloroform/methanol (9/1). The organic layer was concentrated under reduced pressure to obtain the compound 52 (0.3 g).
MS (ESI) m/z: 542 $(M+H)^+$

[Preparation Example 32: Compound 53]

[Formula 43]

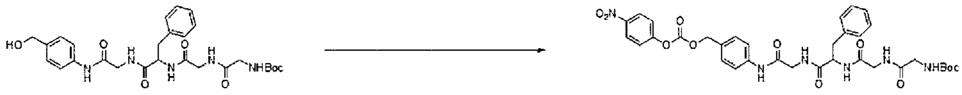

52

53

To a solution of the compound 52 (0.1 g) in DMF (5.0 mL), bis(4-nitrophenyl) carbonate (0.2 g) and diisopropylethylamine (0.5 mL) were added, and the resulting mixture was stirred at room temperature for 3 hours. The reaction solution was concentrated under reduced pressure to obtain the compound 53 (0.1 g).
MS (ESI) m/z: 707 $(M+H)^+$

[Preparation Example 33: Compound 54]

[Formula 44]

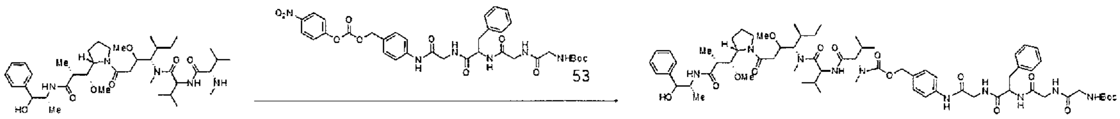

53

14

54

To a solution of the compound 14 (0.1 g) in DMF (5.0 mL), the compound 53 (0.1 g), diisopropylethylamine (0.1 mL), and HOBt (0.2 g) were added, and the resulting mixture was stirred at room temperature for 48 hours. The reaction solution was concentrated under reduced pressure, and the resulting residue was purified by silica gel column chromatography (ODS-A, acetonitrile/water/formic acid) to obtain the compound 54 (0.02 g).
MS (ESI) m/z: 1286 $(M+H)^+$

[Preparation Example 34: Compound 55]

[Formula 45]

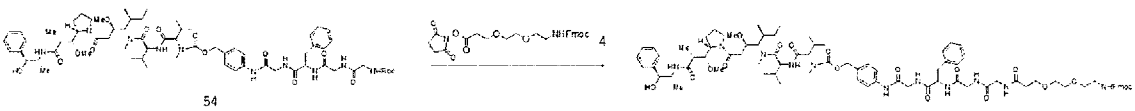

4

54

55

To a solution of the compound 54 (0.02 g) in dichloromethane (5.0 mL), TFA (0.3 mL) was added, and the resulting mixture was stirred at room temperature for 3 hours. Diisopropylethylamine (0.6 mL) and the compound 4 (0.02 g) were further added, and the resulting mixture was stirred at room temperature for 18 hours. The reaction solution was concentrated under reduced pressure, and the resulting residue was purified by preparative HPLC (acetonitrile/water/formic acid) to obtain the compound 55 (0.006 mg).
MS (ESI) m/z: 1567 $(M+H)^+$

[Preparation Example 35: Compound 56]

[Formula 46]

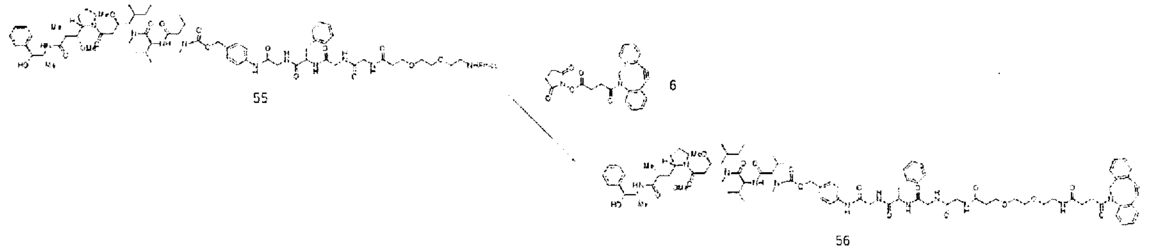

55

6

56

To a solution of the compound 55 (0.006 g) in DMF (1.0 mL), diethylamine (0.1 mL) was added, and the resulting mixture was stirred at room temperature for 1 hour. The reaction solution was concentrated under reduced pressure, to the resulting residue, DMF (1.0 mL) and the compound 6 (DBCO-NHS, 0.003 mg) were added, and the resulting mixture was stirred at room temperature for 17 hours. The reaction solution was purified by gel filtration chromatography (LH-20, DMF) to obtain the compound 56 (0.009 mg).
MS (ESI) m/z: 1632 $(M+H)^+$

[Preparation Example 36: Compound 57]

[Formula 47]

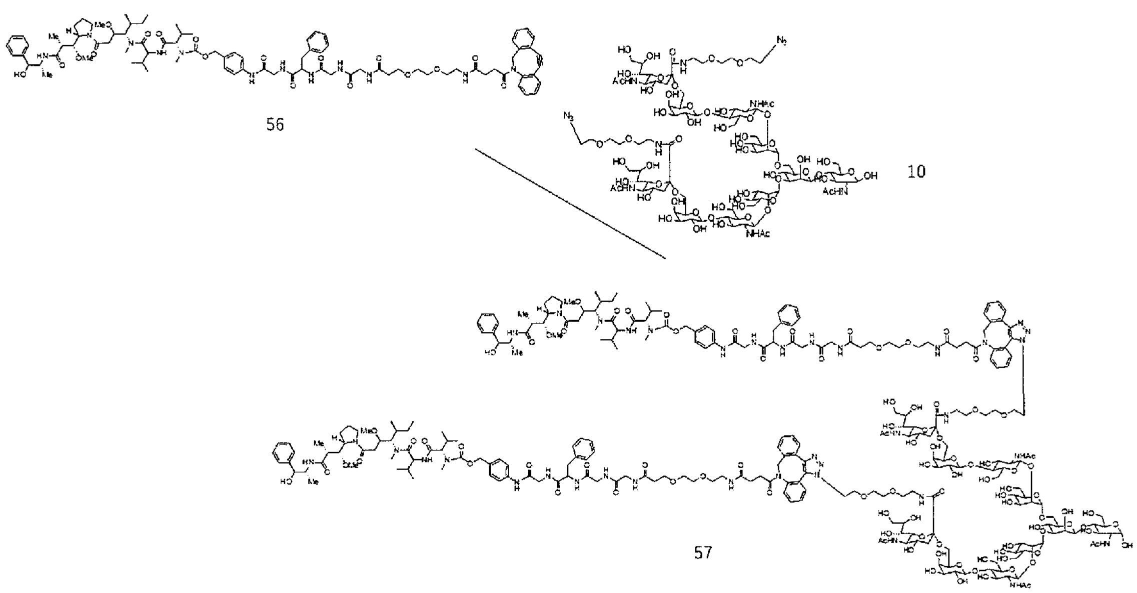

56

10

57

To a solution of the compound 56 (0.001 g) in DMF (0.5 mL), the compound 10 (0.007 g) was added, and the resulting mixture was stirred at room temperature for 24 hours. The reaction solution was concentrated under reduced pressure, and the resulting residue was purified by preparative HPLC (acetonitrile/water/formic acid) to obtain the compound 57 (0.003 g).
MS (ESI) m/z: 1867 $(M+3H)^{3+}$, 1400 $(M+4H)^{4+}$

[Preparation Example 37: Compound 58]

[Formula 48]

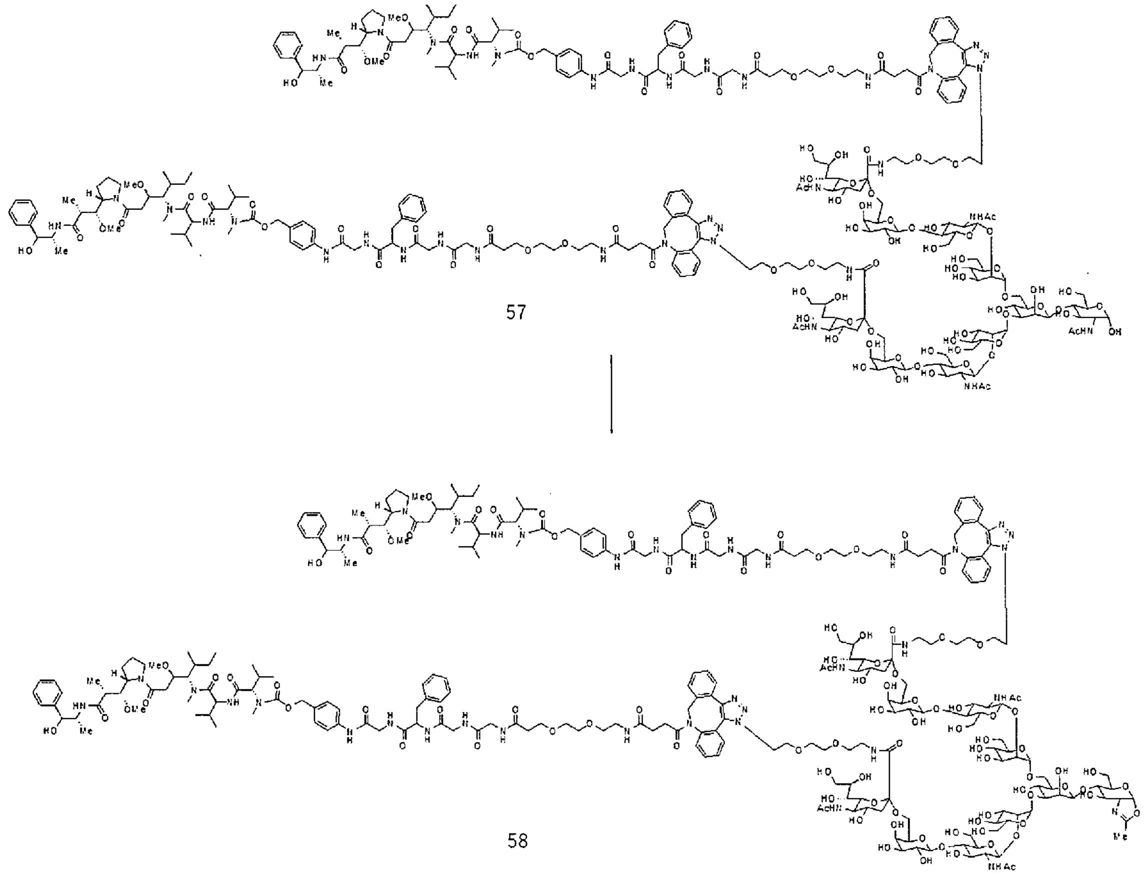

57

58

To a solution of the compound 57 (0.003 g) in a mixture of DMF/water (0.5 mL/0.5 mL), CDMBI (0.006 g) and triethylamine (0.002 mL) were added, and the resulting mixture was stirred at 0°C for 30 minutes. This reaction solution was purified by gel filtration chromatography (LH-20, DMF) to obtain the compound 58 (0.001 g).

[Example 11: Conjugation of antibody-GlcNAc with MMAE-GGFG-attached glycan]

[Formula 49]

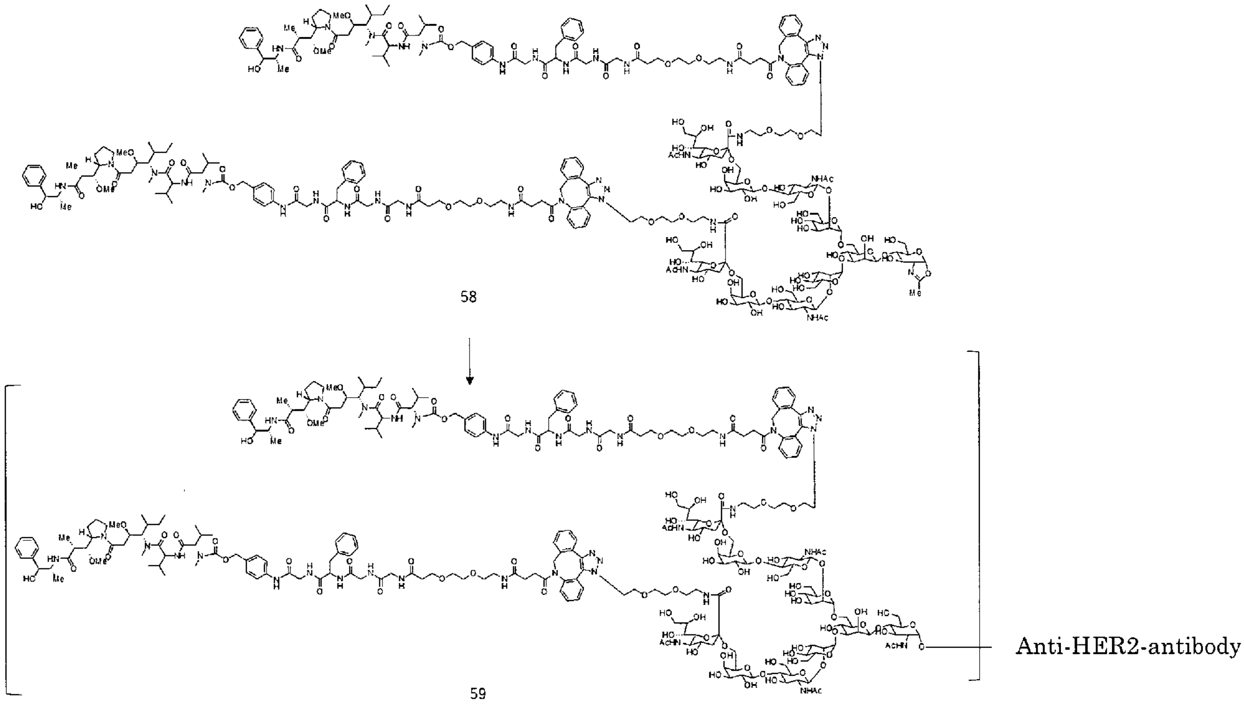

58

59

To an Eppendorf tube, the antibody-GlcNAc obtained in Example 2 (0.03 mg), one of the EndoF3 mutant enzymes prepared in Example 1 (0.003 mg), EndoS mutant enzyme prepared in Example 2 (0.003 mg), or WP1328 enzyme prepared in Example 12 (0.003 mg), and a solution of the compound 58 in DMSO (40 mg/mL, 0.002 mL) were added, and a 50 mM Tris-HCl buffer, pH 7.4 was further added to the mixture to adjust the antibody-GlcNAc concentration to 5.0 mg/mL. The reaction solution was incubated at 30°C or 37°C for 1.5 to 3.0 hours. The reaction solution was subjected to buffer replacement with a 50 mM phosphate buffer, pH 7.0 by ultrafiltration (Amicon Ultra 100 kDa), and the compound 59 was analyzed (Figs. 20 and 21).

[Table 5]

| Enzymatic activity of EndoF3 D165Q for conjugation with MMAE-attached glycan | | | |
|---|---|---|---|
| Substrate | Remaining ratio (%) | Conversion ratio (%) | |
| | Antibody-GlcNAc | 1-Adduct | 2-Adduct |
| MMAE-attached glycan | 7 | 38 | 55 |

[Table 6]

| Enzymatic activity of EndoS D233Q for conjugation with various substrates | | | |
|---|---|---|---|
| Substrate | Remaining ratio (%) | Conversion ratio (%) | |
| | Antibody-GlcNAc | 1-Adduct | 2-Adduct |
| MMAE-attached glycan | 6 | 15 | 79 |
| AP-3-attached glycan | 4 | 9 | 87 |
| Exatecan-attached glycan | 10 | 7 | 83 |
| MMAE-PEG3-attached glycan | 3 | 14 | 83 |
| MMAE- PEG4-attached glycan | 1 | 10 | 89 |
| MMAE-GGFG-attached glycan | 6 | 13 | 81 |

[Table 7]

| Enzymatic activity of WP 1328 for conjugation with various substrates | | | |
|---|---|---|---|
| Substrate | Remaining ratio (%) | Conversion ratio (%) | |
| | Antibody-GlcNAc | 1-Adduct | 2-Adduct |
| MMAE-attached glycan | 9 | 36 | 54 |
| AP-3-attached glycan | 11 | 51 | 39 |
| Exatecan-attached glycan | 7 | 5 | 88 |
| MMAE-PEG3-attached glycan | 3 | 40 | 57 |
| MMAE- PEG4-attached glycan | 5 | 40 | 55 |
| MMAE-GGFG-attached glycan | 13 | 42 | 45 |

[Example 12: Preparation of WP1328 enzyme]

(1) Preparation of pRSFDuet-1-MBP-WP1328

A codon-optimized sequence (SEQ ID NO: 20) of the nucleotide sequence of WP1328 (amino acid sequence is WP 069215570.1) for *E. coli* expression was artificially synthesized (Genewiz), and this sequence was amplified by PCR using primers WP_1328_f and WP_1328-r (30 cycles of 98°C for 10 seconds, 60°C for 5 seconds, and 68°C for 30 seconds).

Nucleotide sequence of WP_1328_f (SEQ ID NO: 21)
ACTATCGAGGGAAGGAGTCGCGCGCTGGCG
Nucleotide sequence of WP_1328_r (SEQ ID NO: 22)
ATCCAATTGAGATCTTCATTTCACCGCGTTCTTAACGG

Similarly, a vector obtained by cloning the maltose binding protein (amino acid sequence is that of SEQ ID NO: 5) and Factor Xa cleavage sequence ATCGAGGGAAGG (SEQ ID NO: 6; amino acid sequence is IEGR (SEQ ID NO: 7)) into the pRSF-1-MBP vector (pRSDuet-1, Fig. 2) at the BglII site was amplified by PCR using primers FNtermMBP and RNtermMBP (30 cycles of 98°C for 10 seconds, 60°C for 5 seconds, and 68°C for 30 seconds).

Nucleotide sequence of FNtermMBP (SEQ ID NO: 8)
AGATCTCAATTGGATATCGG
Nucleotide sequence of RNtermMBP (SEQ ID NO: 9)
CCTTCCCTCGATAGTCTGCGCGTCTTTCAGGGC

The two fragments obtained were cloned by using the In-Fusion kit (TAKARA) so that the Factor Xa sequence was placed at the C-terminus of MBP and the WP1328 sequence of which N-terminal signal sequence was removed was placed downstream thereof. *E. coli* JM109 (TAKARA) cells were transformed with the resulting In-Fusion solution and cultured overnight at 37°C. Colony PCR was performed on the obtained colonies, and a plasmid pRSFDuet-1-MBP-WP1328 (SEQ ID NO: 23) was obtained from a positive clone.

Free Text of Sequence Listing

SEQ ID NO: 1, EndoF3_D165Q
SEQ ID NO: 2, EndoF3_D165Q
SEQ ID NO: 3, FEndoF3-NtermMBP
SEQ ID NO: 4, REndoF3-NtermMBP
SEQ ID NO: 5, Matlose binding protein

SEQ ID NO: 6, Factor Xa
SEQ ID NO: 7, Factor Xa
SEQ ID NO: 8, FNtermMBP
SEQ ID NO: 9, RNtermMBP
SEQ ID NO: 10, pRSFDuet-1-MBP-EndoF3 D165Q
SEQ ID NO: 11, EndoS_D233Q
SEQ ID NO: 12, EndoS_D233Q
SEQ ID NO: 13, FendoS_D233Q_His_pRSF
SEQ ID NO: 14, RendoS_D233Q_front
SEQ ID NO: 15, FendS_D233Q_back
SEQ ID NO: 16, RendoS_D233QHis_pRSF
SEQ ID NO: 17, FpRSF_InF_His_BamHI
SEQ ID NO: 18, RpRSF_InF_His_NcoI
SEQ ID NO: 19, pRSFDuet-1-EndoS_D233Q
SEQ ID NO: 20, EndoF3_D165Q
SEQ ID NO: 21, WP1328f
SEQ ID NO: 22, WP1328r
SEQ ID NO: 23, pRSFDuet-1-MBP-WP1328
SEQ ID NO: 24, WP1328

## Claims

1. A method for producing an antibody-drug conjugate, the method comprising:
conjugating a glycan-homogenized antibody and a glycosylated drug, the glycosylated drug being a drug bound to a glycan having an oxazolinated reducing end via a linker.

2. The method according to claim 1, wherein the glycan-homogenized antibody and the glycosylated drug are conjugated by using an endo-β-N-acetylglucosaminidase mutant enzyme.

3. The method according to claim 2, wherein the endo-β-N-acetylglucosaminidase mutant enzyme is any one of the following proteins:

(1) a protein consisting of the amino acid sequence of SEQ ID NO: 1, or the amino acid sequence of SEQ ID NO: 1 in which the 165th amino acid is replaced with an amino acid other than aspartic acid (D);
(2) a protein consisting of an amino acid sequence derived from the amino acid sequence of the protein described in (1) by substitution, deletion, insertion, and/or addition of one or more amino acids, provided that the 165th amino acid is an amino acid other than D, and having a conjugation activity;
(3) a protein consisting of an amino acid sequence having an identity of at least 90% to the amino acid sequence of the protein described in (1), provided that the 165th amino acid is an amino acid other than D, and having a conjugation activity;
(4) a protein consisting of the amino acid sequence of SEQ ID NO: 11, or the amino acid sequence of SEQ ID NO: 11 in which the 233rd amino acid is replaced with an amino acid other than D;
(5) a protein consisting of an amino acid sequence derived from the amino acid sequence of the protein described in (4) by substitution, deletion, insertion, and/or addition of one or more amino acids, provided that the 233rd amino acid is an amino acid other than D, and having a conjugation activity;
(6) a protein consisting of an amino acid sequence having an identity of at least 90% to the amino acid sequence of the protein described in (4), provided that the 233rd amino acid is an amino acid other than D, and having a conjugation activity; and
(7) a protein consisting of the amino acid sequence of SEQ ID NO: 24, or the amino acid sequence of SEQ ID NO: 24 in which the 165th amino acid is replaced with an amino acid other than D.

4. The method according to any one of claims 1 to 3, wherein the linker contains a structure that can be cleaved in vivo.

5. The method according to any one of claims 1 to 4, wherein the linker has a structure containing a peptide.

6. The method according to any one of claims 1 to 5, wherein the linker has a structure represented by the general formula -E-D-C-B-A-, A binds to the glycan, and E binds to the drug.

[in the formula,

A, B, C, and E are each independently selected from the group consisting of an alkyl group, ester group, carbamoyl group, alkoxyalkyl group, imine group, hydrazone group, azo group, sulfone group, aromatic group, and single bond; and
D is a peptide consisting of 2 to 6 amino acid residues].

**7.** The method according to claim 6, wherein:

A is $-(CH_2CH_2O)_{m1}-(CH_2)_{m2}-$, $-(CH_2)_{m2}-$, or a single bond;
B is selected from the group consisting of

[Formula 1]

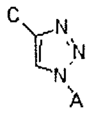
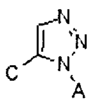
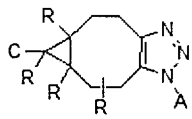
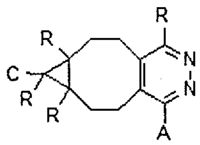
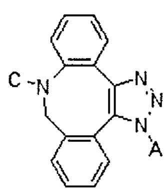
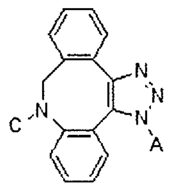

[in the formula, R is independently hydrogen atom, alkyl having 1 to 4 carbon atoms, alkoxy having 1 to 4 carbon atoms, -CN, -OH, $-CF_3$, or NRR];
C is $-C(O)-(CH_2)_{n1}-C(O)-$, $-C(O)-(CH_2)_{n1}-C(O)-NH-(CH_2CH_2O)_{n2}-(CH_2)_{n3}-C(O)-$, $-C(O)-(CH_2)_{n1}-C(O)-NH-(CH_2)_{n3}-C(O)-$, or a single bond,
E is -aminobenzylalcohol-C(O)-, $NH-(CH_2)_o-$, or a single bond,
m1 is an integer of from 1 to 12
m2 is an integer of from 1 to 6,
n1 is an integer of from 1 to 6,
n2 is an integer of from 1 to 12,
n3 is an integer of from 1 to 6, and
o is an integer of from 1 to 6].

**8.** The method according to claim 7, wherein B is either of

[Formula 2]

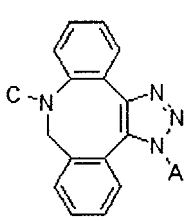
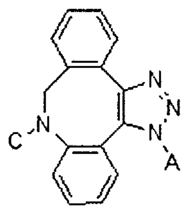

**9.** The method according to any one of claims 1 to 8, wherein the drug is any selected from the group consisting of anthracyclines, auristatins, maytansines, camptothecins, pyrrolobenzodiazepine dimers, calicheamicins, and duocarmycins.

**10.** An enzyme agent for use in a reaction of a glycan-homogenized antibody with a glycosylated drug, the agent comprising any one of the following proteins:

(1) a protein consisting of the amino acid sequence of SEQ ID NO: 1, or the amino acid sequence of SEQ ID NO: 1 in which the 165th amino acid is replaced with an amino acid other than aspartic acid (D);
(2) a protein consisting of an amino acid sequence derived from the amino acid sequence of the protein described in (1) by substitution, deletion, insertion, and/or addition of one or more amino acids, provided that the 165th amino acid is an amino acid other than D, and having a conjugation activity;
(3) a protein consisting of an amino acid sequence having an identity of at least 90% to the amino acid sequence

of the protein described in (1), provided that the 165th amino acid is an amino acid other than D, and having a conjugation activity;
(4) a protein consisting of the amino acid sequence of SEQ ID NO: 11, or the amino acid sequence of SEQ ID NO: 11 in which the 233rd amino acid is replaced with an amino acid other than D;
(5) a protein consisting of an amino acid sequence derived from the amino acid sequence of the protein described in (4) by substitution, deletion, insertion, and/or addition of one or more amino acids, provided that the 233rd amino acid is an amino acid other than D, and having a conjugation activity;
(6) a protein consisting of an amino acid sequence having an identity of at least 90% to the amino acid sequence of the protein described in (4), provided that the 233rd amino acid is an amino acid other than D, and having a conjugation activity; and
(7) a protein consisting of the amino acid sequence of SEQ ID NO: 24, or the amino acid sequence of SEQ ID NO: 24 in which the 165th amino acid is replaced with an amino acid other than D.

**11.** Use of a polynucleotide encoding any one of the following proteins in the production of an enzyme for use in a reaction of a glycan-homogenized antibody with a glycosylated drug:

(1) a protein consisting of the amino acid sequence of SEQ ID NO: 1, or the amino acid sequence of SEQ ID NO: 1 in which the 165th amino acid is replaced with an amino acid other than aspartic acid (D);
(2) a protein consisting of an amino acid sequence derived from the amino acid sequence of the protein described in (1) by substitution, deletion, insertion, and/or addition of one or more amino acids, provided that the 165th amino acid is an amino acid other than D, and having a conjugation activity;
(3) a protein consisting of an amino acid sequence having an identity of at least 90% to the amino acid sequence of the protein described in (1), provided that the 165th amino acid is an amino acid other than D, and having a conjugation activity;
(4) a protein consisting of the amino acid sequence of SEQ ID NO: 11, or the amino acid sequence of SEQ ID NO: 11 in which the 233rd amino acid is replaced with an amino acid other than D;
(5) a protein consisting of an amino acid sequence derived from the amino acid sequence of the protein described in (4) by substitution, deletion, insertion, and/or addition of one or more amino acids, provided that the 233rd amino acid is an amino acid other than D, and having a conjugation activity;
(6) a protein consisting of an amino acid sequence having an identity of at least 90% to the amino acid sequence of the protein described in (4), provided that the 233rd amino acid is an amino acid other than D, and having a conjugation activity; and
(7) a protein consisting of the amino acid sequence of SEQ ID NO: 24, or the amino acid sequence of SEQ ID NO: 24 in which the 165th amino acid is replaced with an amino acid other than D.

[Fig.1]

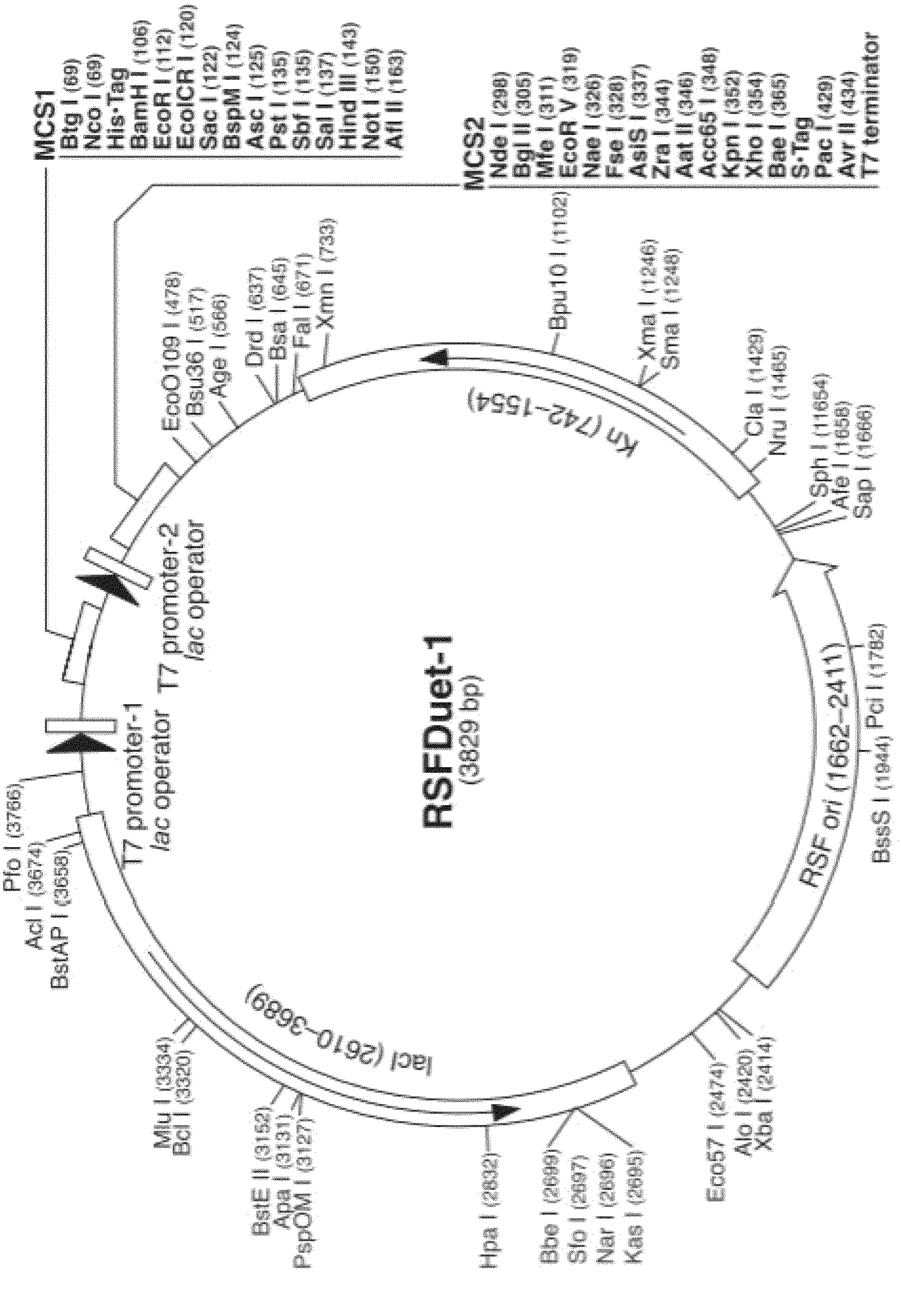

RSFDuet-1
(3829 bp)
MCS1
Btg I (69)
Nco I (69)
His·Tag
BamH I (106)
EcoR I (112)
EcoICR I (120)
Sac I (122)
BspM I (124)
Asc I (125)
Pst I (135)
Sbf I (135)
Sal I (137)
Hind III (143)
Not I (150)
Afl II (163)
MCS2
Nde I (298)
Bgl II (305)
Mfe I (311)
EcoR V (319)
Nae I (326)
Fse I (328)
AsiS I (337)
Zra I (344)
Aat II (346)
Acc65 I (348)
Kpn I (352)
Xho I (354)
Bae I (365)
S·Tag
Pac I (429)
Avr II (434)
T7 terminator
EcoO109 I (478)
Bsu36 I (517)
Age I (566)
Drd I (637)
Bsa I (645)
Fal I (671)
Xmn I (733)
Bpu10 I (1102)
Xma I (1246)
Sma I (1248)
Kn (742-1554)
Cla I (1429)
Nru I (1465)
Sph I (1654)
Afe I (1658)
Sap I (1666)
Pci I (1782)
BssS I (1944)
RSF ori (1662-2411)
Eco57 I (2474)
Alo I (2420)
Xba I (2414)
Hpa I (2832)
Bbe I (2699)
Sfo I (2697)
Nar I (2696)
Kas I (2695)
lacI (2610-3689)
BstE II (3152)
Apa I (3131)
PspOM I (3127)
Mlu I (3334)
Bcl I (3320)
Pfo I (3766)
Acl I (3674)
BstAP I (3658)
T7 promoter-1
lac operator
T7 promoter-2
lac operator

[Fig.2-1]

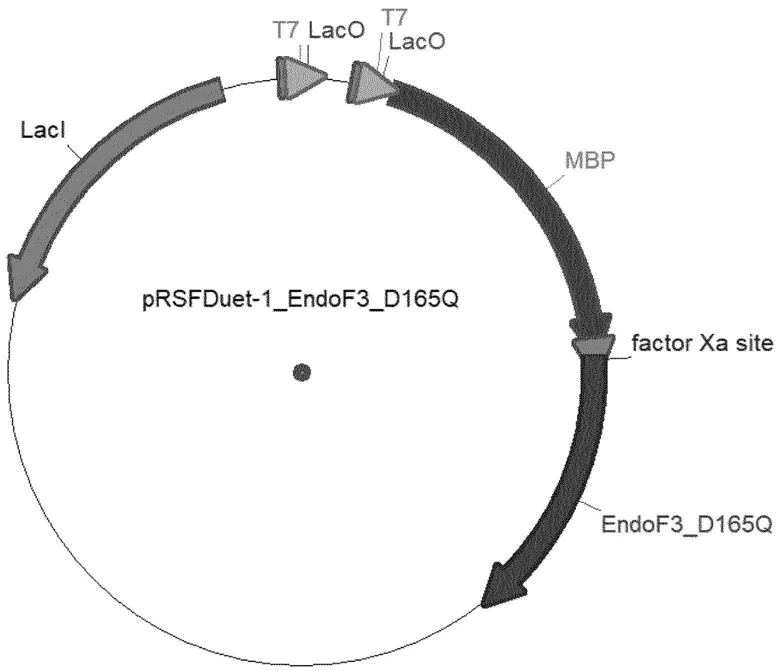
T7 LacO
T7
LacO
LacI
MBP
pRSFDuet-1_EndoF3_D165Q
factor Xa site
EndoF3_D165Q

[Fig.2-2]

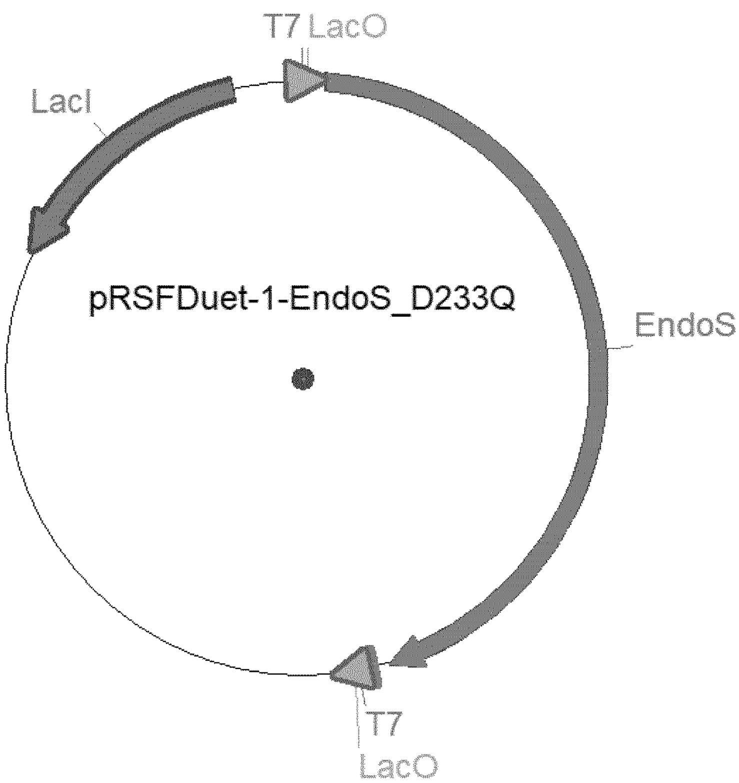
T7 LacO
LacI
pRSFDuet-1-EndoS_D233Q
EndoS
T7
LacO

[Fig.3]

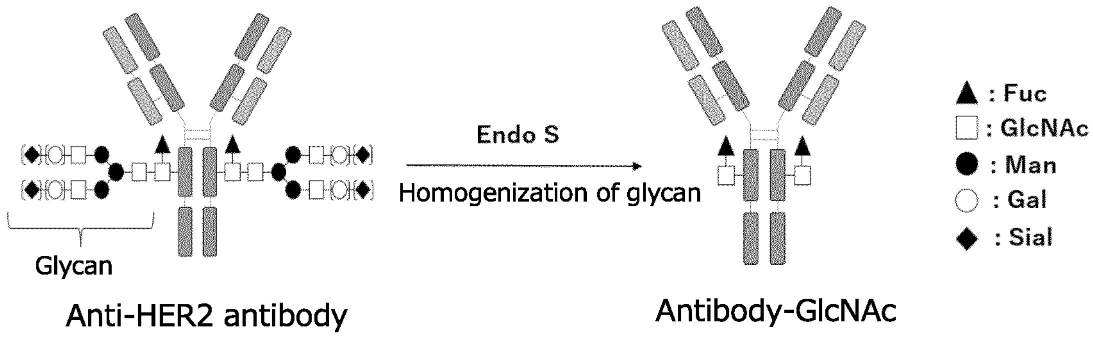
Endo S
Homogenization of glycan
Glycan
Anti-HER2 antibody
Antibody-GlcNAc
: Fuc
: GlcNAc
: Man
: Gal
: Sial

[Fig.4]

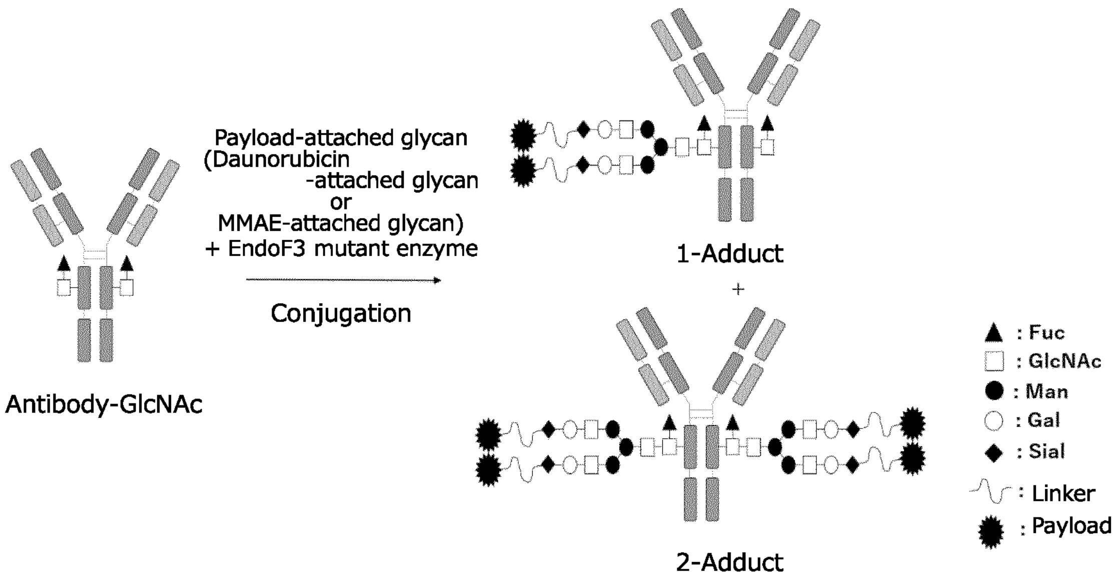
Payload-attached glycan
(Daunorubicin
-attached glycan
or
MMAE-attached glycan)
+ EndoF3 mutant enzyme
Conjugation
Antibody-GlcNAc
1-Adduct
+
2-Adduct
: Fuc
: GlcNAc
: Man
: Gal
: Sial
: Linker
: Payload

[Fig.5]

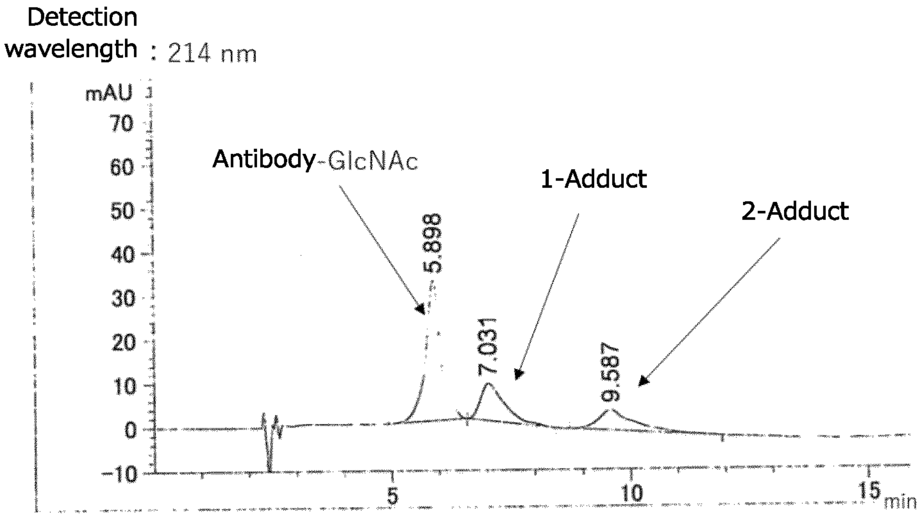
Detection wavelength : 214 nm
mAU
Antibody-GlcNAc
1-Adduct
2-Adduct
5.898
7.031
9.587
min

[Fig.6]

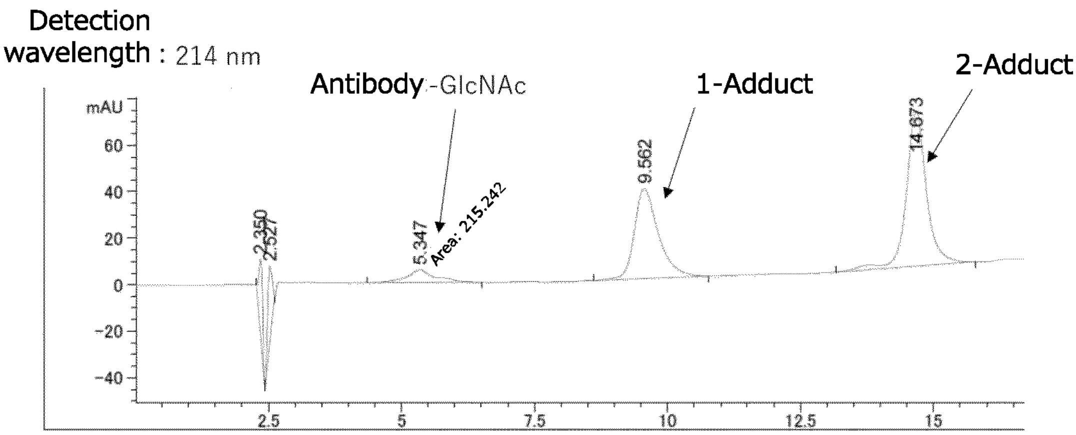
Detection wavelength : 214 nm
Antibody-GlcNAc
1-Adduct
2-Adduct
mAU
2.350
2.527
5.347
Area: 215.242
9.562
14.673

[Fig.7]

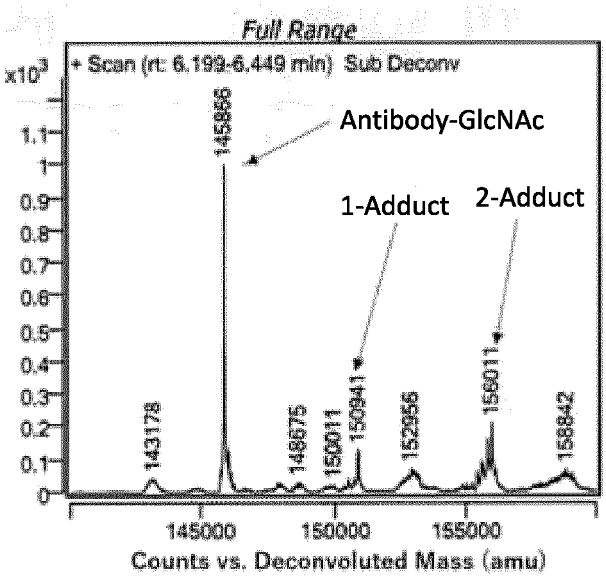


- (1-Adduct) - (Antibody-GlcNAc) =
  5,071 : Calculated (m/z)
  5,075 : Deconvolution data (m/z)
- (2-Adduct) - (Antibody-GlcNAc) =
  10.142 : Calculated (m/z)
  10,143 : Deconvolution data (m/z)

[Fig.8]

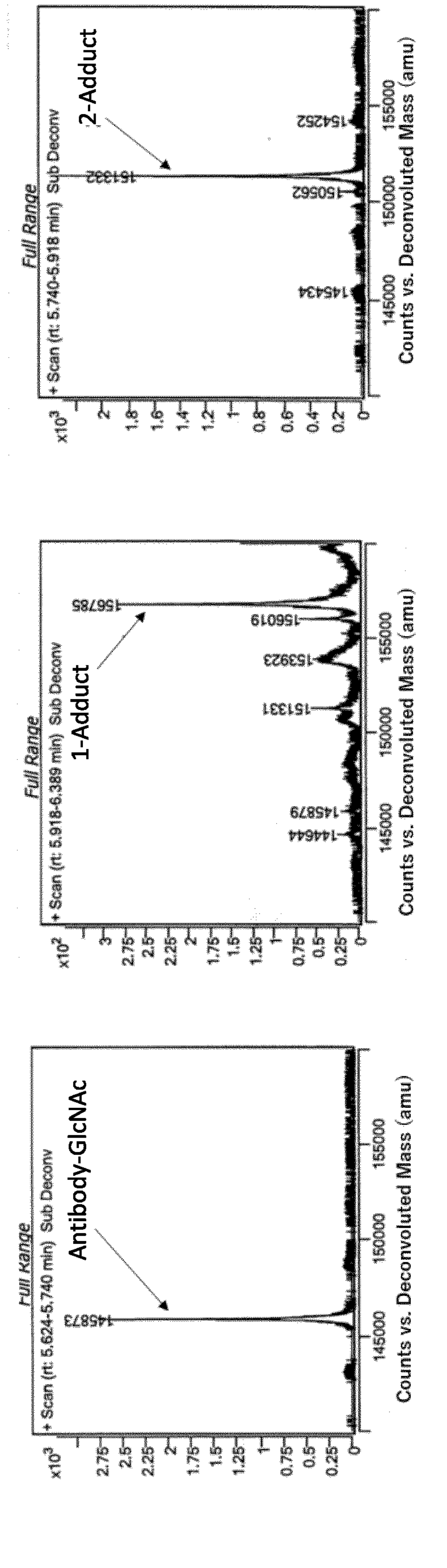


- (1-Adduct) - (Antibody-GlcNAc) =
  5,452 : Calculated (m/z)
  5,459 : Deconvolution data (m/z)
- (2-Adduct) - (Antibody-GlcNAc) =
  10.904 : Calculated (m/z)
  10,912 : Deconvolution data (m/z)

[Fig.9]

**Amino acid sequence of EndoF3_D165Q (SEQ ID NO:1)**

MKKIFFAQCSILLLMLGSCSKMTEDMTPESVNKEASVKSATALAGSNGVC
IAYYITDGRNPTFKLKDIPDKVDMVILFGLKYWSLQDTTKLPGGTGMMGS
FKSYKDLDTQIRSLQSRGIKVLQNIDDDVSWQSSKPGGFASAAAYGDAIK
SIVIDKWKLDGISLQIEHSGAKPNPIPTFPGYAATGYNGWYSGSMAATPA
FLNVISELTKYFGTTAPNNKQLQIASGIDVYAWNKIMENFRNNFNYIQLQ
SYGANVSRTQLMMNYATGTNKIPASKMVFGAYAEGGTNQANDVEVAKWTP
TQGAKGGMMIYTYNSNVSYANAVRDAVKN

**Optimized nucleotide sequence of EndoF3_D165Q (SEQ ID NO:2)**

GCAACCGCACTGGCAGGTAGCAATGGTGTTTGTATTGCCTATTATATCACCGATGGTCGTAACCCGA
CCTTCAAACTGAAAGATATTCCGGATAAAGTGGACATGGTGATTCTGTTTGGTCTGAAATATTGGAG
CCTGCAGGATACCACCAAACTGCCTGGTGGCACCGGTATGATGGGTAGCTTTAAAAGTTATAAAGAT
CTGGACACCCAGATTCGTAGCCTGCAGAGCCGTGGTATTAAAGTGCTGCAGAATATTGATGATGATG
TTAGCTGGCAGAGCAGCAAACCTGGTGGTTTTGCAAGCGCAGCAGCATATGGTGATGCAATTAAAAG
CATCGTGATCGACAAATGGAAACTGGATGGTATTAGTCTGCAGATTGAACATAGCGGTGCAAAACCG
AATCCGATTCCGACCTTTCCGGGTTATGCAGCAACCGGTTATAATGGTTGGTATAGCGGTAGCATGG
CAGCAACACCGGCATTTCTGAATGTTATTAGCGAACTGACCAAATATTTCGGCACCACCGCACCGAA
TAACAAACAGCTGCAGATCGCAAGCGGTATTGATGTTTATGCATGGAACAAGATCATGGAAAACTTC
CGCAACAACTTCAACTATATTCAGCTGCAGTCTTATGGTGCCAATGTTAGTCGTACCCAGCTGATGA
TGAATTATGCAACCGGCACCAACAAAATTCCGGCAAGCAAAATGGTTTTTGGTGCATATGCCGAAGG
TGGCACCAATCAGGCAAATGATGTTGAAGTGGCAAAATGGACCCCGACACAGGGTGCAAAAGGTGGT
ATGATGATTTATACCTATAACAGCAATGTGAGCTATGCCAATGCAGTTCGTGATGCCGTTAAAAACT
GA

**Amino acid sequence of matlose binding protein (SEQ ID NO:5)**

MKTEEGKLVIWINGDKGYNGLAEVGKKFEKDTGIKVTVEHPDKLEEKFPQVAATGDGPDIIFWAHDR
FGGYAQSGLLAEITPDKAFQDKLYPFTWDAVRYNGKLIAYPIAVEALSLIYNKDLLPNPPKTWEEIP
ALDKELKAKGKSALMFNLQEPYFTWPLIAADGGYAFKYENGKYDIKDVGVDNAGAKAGLTFLVDLIK
NKHMNADTDYSIAEAAFNKGETAMTINGPWAWSNIDTSKVNYGVTVLPTFKGQPSKPFVGVLSAGIN
AASPNKELAKEFLENYLLTDEGLEAVNKDKPLGAVALKSYEEELAKDPRIAATMENAQKGEIMPNIP
QMSAFWYAVRTAVINAASGRQTVDEALKDAQT

[Fig.10]

Full length nucleotide sequence of pRSFDuet-1-MBP-EndoF3 D165Q (SEQ ID NO:10)

GGGGAATTGTGAGCGGATAACAATTCCCCTGTAGAAATAATTTTGTTTAACTTTAATAAGGAGATATACCATGGGCAGCAGCCATCAC
CATCATCACCACAGCCAGGATCCGAATTCGAGCTCGGCGCGCCTGCAGGTCGACAAGCTTGCGGCCGCATAATGCTTAAGTCGAACAG
AAAGTAATCGTATTGTACACGGCCGCATAATCGAAATTAATACGACTCACTATAGGGGAATTGTGAGCGGATAACAATTCCCCATCTT
AGTATATTAGTTAAGTATAAGAAGGAGATATACATATGATGAAAACTGAAGAAGGTAAACTGGTAATCTGGATTAACGGCGATAAAGG
CTATAACGGTTTGGCTGAAGTCGGTAAGAAATTCGAGAAAGATACCGGAATTAAAGTCACCGTTGAGCATCCGGATAAACTGGAAGAG
AAATTCCCACAGGTTGCGGCAACTGGCGATGGCCCTGACATTATCTTCTGGGCACACGACCGCTTTGGTGGCTACGCTCAATCTGGCC
TGTTGGCTGAAATCACCCCGGACAAAGCGTTCCAGGACAAGCTGTATCCGTTTACCTGGGATGCCGTACGTTACAACGGCAAGCTGAT
TGCTTACCCGATCGCTGTTGAAGCGTTATCGCTGATTTATAACAAAGATCTGCTGCCGAACCCGCCAAAAACCTGGGAAGAGATCCCG
GCGCTGGATAAAGAACTGAAAGCGAAAGGTAAGAGCGCGCTGATGTTCAACCTGCAAGAACCGTACTTCACCTGGCCGCTGATTGCTG
CTGACGGGGGTTATGCGTTCAAGTATGAAAACGGCAAGTACGACATTAAAGACGTGGGCGTGGATAACGCTGGCGCGAAAGCGGGTCT
GACCTTCCTGGTTGACCTGATTAAAAACAAACACATGAATGCAGACACCGATTACTCCATCGCAGAAGCTGCCTTTAATAAAGGCGAA
ACAGCGATGACCATCAACGGCCCGTGGGCATGGTCCAACATCGACACCAGCAAAGTGAATTATGGTGTAACGGTACTGCCGACCTTCA
AGGGTCAACCATCCAAACCGTTCGTTGGCGTGCTGAGCGCAGGTATTAACGCCGCCAGTCCGAACAAAGAGCTGGCAAAAGAGTTCCT
CGAAAACTATCTGCTGACTGATGAAGGTCTGGAAGCGGTTAATAAAGACAAACCGCTGGGTGCCGTAGCGCTGAAGTCTTACGAGGAA
GAGTTGGCGAAAGATCCACGTATTGCCGCCACTATGGAAAACGCCCAGAAAGGTGAAATCATGCCGAACATCCCGCAGATGTCCGCTT
TCTGGTATGCCGTGCGTACTGCGGTGATCAACGCCGCCAGCGGTCGTCAGACTGTCGATGAAGCCCTGAAAGACGCGCAGACTATCGA
GGGAAGGGCAACCGCACTGGCAGGTAGCAATGGTGTTTGTATTGCCTATTATATCACCGATGGTCGTAACCCGACCTTCAAACTGAAA
GATATTCCGGATAAAGTGGACATGGTGATTCTGTTTGGTCTGAAATATTGGAGCCTGCAGGATACCACCAAACTGCCTGGTGGCACCG
GTATGATGGGTAGCTTTAAAAGTTATAAAGATCTGGACACCCAGATTCGTAGCCTGCAGAGCCGTGGTATTAAAGTGCTGCAGAATAT
TGATGATGATGTTAGCTGGCAGAGCAGCAAACCTGGTGGTTTTGCAAGCGCAGCAGCATATGGTGATGCAATTAAAAGCATCGTGATC
GACAAATGGAAACTGGATGGTATTAGTCTGCAGATTGAACATAGCGGTGCAAAACCGAATCCGATTCCGACCTTTCCGGGTTATGCAG
CAACCGGTTATAATGGTTGGTATAGCGGTAGCATGGCAGCAACACCGGCATTTCTGAATGTTATTAGCGAACTGACCAAATATTTCGG
CACCACCGCACCGAATAACAAACAGCTGCAGATCGCAAGCGGTATTGATGTTTATGCATGGAACAAGATCATGGAAAACTTCCGCAAC
AACTTCAACTATATTCAGCTGCAGTCTTATGGTGCCAATGTTAGTCGTACCCAGCTGATGATGAATTATGCAACCGGCACCAACAAAA
TTCCGGCAAGCAAAATGGTTTTTGGTGCATATGCCGAAGGTGGCACCAATCAGGCAAATGATGTTGAAGTGGCAAAATGGACCCCGAC
ACAGGGTGCAAAAGGTGGTATGATGATTTATACCTATAACAGCAATGTGAGCTATGCCAATGCAGTTCGTGATGCCGTTAAAAACTGA
AGATCTCAATTGGATATCGGCCGGCCACGCGATCGCTGACGTCGGTACCCTCGAGTCTGGTAAAGAAACCGCTGCTGCGAAATTTGAA
CGCCAGCACATGGACTCGTCTACTAGCGCAGCTTAATTAACCTAGGCTGCTGCCACCGCTGAGCAATAACTAGCATAACCCCTTGGGG
CCTCTAAACGGGTCTTGAGGGGTTTTTTGCTGAAACCTCAGGCATTTGAGAAGCACACGGTCACACTGCTTCCGGTAGTCAATAAACC
GGTAAACCAGCAATAGACATAAGCGGCTATTTAACGACCCTGCCCTGAACCGACGACAAGCTGACGACCGGGTCTCCGCAAGTGGCAC
TTTTCGGGGAAATGTGCGCGGAACCCCTATTTGTTTATTTTTCTAAATACATTCAAATATGTATCCGCTCATGAATTAATTCTTAGAA
AAACTCATCGAGCATCAAATGAAACTGCAATTTATTCATATCAGGATTATCAATACCATATTTTTGAAAAAGCCGTTTCTGTAATGAA
GGAGAAAACTCACCGAGGCAGTTCCATAGGATGGCAAGATCCTGGTATCGGTCTGCGATTCCGACTCGTCCAACATCAATACAACCTA
TTAATTTCCCCTCGTCAAAAATAAGGTTATCAAGTGAGAAATCACCATGAGTGACGACTGAATCCGGTGAGAATGGCAAAAGTTTATG
CATTTCTTTCCAGACTTGTTCAACAGGCCAGCCATTACGCTCGTCATCAAAATCACTCGCATCAACCAAACCGTTATTCATTCGTGAT
TGCGCCTGAGCGAGACGAAATACGCGGTCGCTGTTAAAAGGACAATTACAAACAGGAATCGAATGCAACCGGCGCAGGAACACTGCCA
GCGCATCAACAATATTTTCACCTGAATCAGGATATTCTTCTAATACCTGGAATGCTGTTTTCCCGGGGATCGCAGTGGTGAGTAACCA
TGCATCATCAGGAGTACGGATAAAATGCTTGATGGTCGGAAGAGGCATAAATTCCGTCAGCCAGTTTAGTCTGACCATCTCATCTGTA
ACATCATTGGCAACGCTACCTTTGCCATGTTTCAGAAACAACTCTGGCGCATCGGGCTTCCCATACAATCGATAGATTGTCGCACCTG
ATTGCCCGACATTATCGCGAGCCCATTTATACCCATATAAATCAGCATCCATGTTGGAATTTAATCGCGGCCTAGAGCAAGACGTTTC
CCGTTGAATATGGCTCATACTCTTCCTTTTTCAATATTATTGAAGCATTTATCAGGGTTATTGTCTCATGAGCGGATACATATTTGAA
TGTATTTAGAAAAATAAACAAATAGGCATGCAGCGCTCTTCCGCTTCCTCGCTCACTGACTCGCTACGCTCGGTCGTTCGACTGCGGC
GAGCGGTGTCAGCTCACTCAAAAGCGGTAATACGGTTATCCACAGAATCAGGGGATAAAGCCGGAAAGAACATGTGAGCAAAAAGCAA
AGCACCGGAAGAAGCCAACGCCGCAGGCGTTTTTCCATAGGCTCCGCCCCCCTGACGAGCATCACAAAAATCGACGCTCAAGCCAGAG
GTGGCGAAACCCGACAGGACTATAAAGATACCAGGCGTTTCCCCCTGGAAGCTCCCTCGTGCGCTCTCCTGTTCCGACCCTGCCGCTT
ACCGGATACCTGTCCGCCTTTCTCCCTTCGGGAAGCGTGGCGCTTTCTCATAGCTCACGCTGTTGGTATCTCAGTTCGGTGTAGGTCG
TTCGCTCCAAGCTGGGCTGTGTGCACGAACCCCCCGTTCAGCCCGACCGCTGCGCCTTATCCGGTAACTATCGTCTTGAGTCCAACCC
GGTAAGACACGACTTATCGCCACTGGCAGCAGCCATTGGTAACTGATTTAGAGGACTTTGTCTTGAAGTTATGCACCTGTTAAGGCTA
AACTGAAAGAACAGATTTGGTGAGTGCGGTCCTCCAACCCACTTACCTTGGTTCAAAGAGTTGGTAGCTCAGCGAACCTTGAGAAAA
CCACCGTTGGTAGCGGTGGTTTTTCTTTATTTATGAGATGATGAATCAATCGGTCTATCAAGTCAACGAACAGCTATTCCGTTACTCT
AGATTTCAGTGCAATTTATCTCTTCAAATGTAGCACCTGAAGTCAGCCCCATACGATATAAGTTGTAATTCTCATGTTAGTCATGCCC
CGCGCCCACCGGAAGGAGCTGACTGGGTTGAAGGCTCTCAAGGGCATCGGTCGAGATCCCGGTGCCTAATGAGTGAGCTAACTTACAT
TAATTGCGTTGCGCTCACTGCCCGCTTTCCAGTCGGGAAACCTGTCGTGCCAGCTGCATTAATGAATCGGCCAACGCGCGGGGAGAGG
CGGTTTGCGTATTGGGCGCCAGGGTGGTTTTTCTTTTCACCAGTGAGACGGGCAACAGCTGATTGCCCTTCACCGCCTGGCCCTGAGA
GAGTTGCAGCAAGCGGTCCACGCTGGTTTGCCCCAGCAGGCGAAAATCCTGTTTGATGGTGGTTAACGGCGGGATATAACATGAGCTG
TCTTCGGTATCGTCGTATCCCACTACCGAGATGTCCGCACCAACGCGCAGCCCGGACTCGGTAATGGCGCGCATTGCGCCCAGCGCCA
TCTGATCGTTGGCAACCAGCATCGCAGTGGGAACGATGCCCTCATTCAGCATTTGCATGGTTTGTTGAAAACCGGACATGGCACTCCA
GTCGCCTTCCCGTTCCGCTATCGGCTGAATTTGATTGCGAGTGAGATATTTATGCCAGCCAGCCAGACGCAGACGCGCCGAGACAGAA
CTTAATGGGCCCGCTAACAGCGCGATTTGCTGGTGACCCAATGCGACCAGATGCTCCACGCCCAGTCGCGTACCGTCTTCATGGGAGA
AAATAATACTGTTGATGGGTGTCTGGTCAGAGACATCAAGAAATAACGCCGGAACATTAGTGCAGGCAGCTTCCACAGCAATGGCATC
CTGGTCATCCAGCGGATAGTTAATGATCAGCCCACTGACGCGTTGCGCGAGAAGATTGTGCACCGCCGCTTTACAGGCTTCGACGCCG
CTTCGTTCTACCATCGACACCACCACGCTGGCACCCAGTTGATCGGCGCGAGATTTAATCGCCGCGACAATTTGCGACGGCGCGTGCA
GGGCCAGACTGGAGGTGGCAACGCCAATCAGCAACGACTGTTTGCCCGCCAGTTGTTGTGCCACGCGGTTGGGAATGTAATTCAGCTC
CGCCATCGCCGCTTCCACTTTTTCCCGCGTTTTCGCAGAAACGTGGCTGGCCTGGTTCACCACGCGGGAAACGGTCTGATAAGAGACA
CCGGCATACTCTGCGACATCGTATAACGTTACTGGTTTCACATTCACCACCCTGAATTGACTCTCTTCCGGGCGCTATCATGCCATAC
CGCGAAAGGTTTTGCGCCATTCGATGGTGTCCGGGATCTCGACGCTCTCCCTTATGCGACTCCTGCATTAGGAAATTAATACGACTCA
CTATA

[Fig.11]

## Amino acid sequence of EndoS_D233Q(SEQ ID NO:11)

MDKHLLVKRTLGCVCAATLMGAALATHHDSLNTVKAEEKTVQVQKGLPSIDSLHYLSENSKKEFKEELSKAGQESQKV
KEILAKAQQADKQAQELAKMKIPEKIPMKPLHGPLYGGYFRTWHDKTSDPTEKDKVNSMGELPKEVDLAFIFHDWTKD
YSLFWKELATKHVPKLNKQGTRVIRTIPWRFLAGGDNSGIAEDTSKYPNTPEGNKALAKAIVDEYVYKYNLDGLDVQV
EHDSIPKVDKKEDTAGVERSIQVFEEIGKLIGPKGVDKSRLFIMDSTYMADKNPLIERGAPYINLLLVQVYGSQGEKG
GWEPVSNRPEKTMEERWQGYSKYIRPEQYMIGFSFYEENAQEGNLWYDINSRKDEDKANGINTDITGTRAERYARWQP
KTGGVKGGIFSYAIDRDGVAHQPKKYAKQKEFKDATDNIFHSDYSVSKALKTVMLKDKSYDLIDEKDFPDKALREAVM
AQVGTRKGDLERFNGTLRLDNPAIQSLEGLNKFKKLAQLDLIGLSRITKLDRSVLPANMKPGKDTLETVLETYKKDNK
EEPATIPPVSLKVSGLTGLKELDLSGFDRETLAGLDAATLTSLEKVDISGNKLDLAPGTENRQIFDTMLSTISNHVGS
NEQTVKFDKQKPTGHYPDTYGKTSLRLPVANEKVDLQSQLLFGTVTNQGTLINSEADYKAYQNHKIAGRSFVDSNYHY
NNFKVSYENYTVKVTDSTLGTTTDKTLATDKEETYKVDFFSPADKTKAVHTAKVIVGDEKTMMVNLAEGATVIGGSAD
PVNARKVFDGQLGSETDNISLGWDSKQSIIFKLKEDGLIKHWRFFNDSARNPETTNKPIQEASLQIFNIKDYNLDNLL
ENPNKFDDEKYWITVDTYSAQGERATAFSNTLNNITSKYWRVVFDTKGDRYSSPVVPELQILGYPLPNADTIMKTVTT
AKELSQQKDKFSQKMLDELKIKEMALETSLNSKIFDVTAINANAGVLKDCIEKRQLLKK

## Optimized nucleotide sequence of EndoS_D233Q (SEQ ID NO:12)

ATGGATAAACATTTGTTGGTAAAAAGAACACTAGGGTGTGTTTGTGCTGCAACGTTGATGGGAGCTGCCTTAGCGACC
CACCATGATTCACTCAATACTGTAAAAGCGGAGGAGAAGACTGTTCAGGTTCAGAAAGGATTACCTTCTATCGATAGC
TTGCATTATCTGTCAGAGAATAGCAAAAAAGAATTTAAAGAAGAACTCTCAAAAGCGGGGCAAGAATCTCAAAAGGTC
AAAGAGATATTAGCAAAAGCTCAGCAGGCAGATAAACAAGCTCAAGAACTTGCCAAAATGAAAATTCCTGAGAAAATA
CCGATGAAACCGTTACATGGTCCTCTCTACGGTGGTTACTTTAGAACTTGGCATGACAAAACATCAGATCCAACAGAA
AAAGACAAAGTTAACTCGATGGGAGAGCTTCCTAAAGAAGTAGATCTAGCCTTTATTTTCCACGATTGGACAAAAGAT
TATAGCCTTTTTTGGAAAGAATTGGCCACCAAACATGTGCCAAAGTTAAACAAGCAAGGGACACGTGTCATTCGTACC
ATTCCATGGCGTTTCCTAGCTGGGGGTGATAACAGTGGTATTGCAGAAGATACCAGTAAATACCCAAATACACCAGAG
GGAAATAAAGCTTTAGCCAAAGCTATTGTTGATGAATATGTTTATAAATACAACCTTGATGGCTTAGATGTGCAAGTT
GAACATGATAGTATTCCAAAAGTTGACAAAAAAGAAGATACAGCAGGCGTAGAACGCTCTATTCAAGTGTTTGAAGAA
ATTGGGAAATTAATTGGACCAAAAGGTGTTGATAAATCGCGGTTATTTATTATGGATAGCACCTACATGGCTGATAAA
AACCCATTGATTGAGCGAGGAGCTCCTTATATTAATTTATTACTGGTACAGGTCTATGGTTCACAAGGAGAGAAAGGT
GGTTGGGAGCCTGTTTCTAATCGACCTGAAAAAACAATGGAAGAACGATGGCAAGGTTATAGCAAGTATATTCGTCCT
GAACAATACATGATTGGTTTTTCTTTCTATGAGGAAAATGCTCAAGAAGGGAATCTTTGGTATGATATTAATTCTCGC
AAGGACGAGGACAAAGCAAATGGAATTAACACTGACATAACTGGAACGCGTGCCGAACGGTATGCAAGGTGGCAACCT
AAGACAGGTGGGGTTAAGGGAGGTATCTTCTCCTACGCTATTGACCGAGATGGTGTAGCTCATCAACCTAAAAAATAT
GCTAAACAGAAAGAGTTTAAGGACGCAACTGATAACATCTTCCACTCAGATTATAGTGTCTCCAAGGCATTAAAGACA
GTTATGCTAAAAGATAAGTCGTATGATCTGATTGATGAGAAAGATTTCCCAGATAAGGCTTTGCGAGAAGCTGTGATG
GCGCAGGTTGGAACCAGAAAAGGTGATTTGGAACGTTTCAATGGCACATTACGATTGGATAATCCAGCGATTCAAAGT
TTAGAAGGTCTAAATAAATTTAAAAAATTAGCTCAATTAGACTTGATTGGCTTATCTCGCATTACAAAGCTCGACCGT
TCTGTTTTACCCGCTAATATGAAGCCAGGCAAAGATACCTTGGAAACAGTTCTTGAAACCTATAAAAAGGATAACAAA
GAAGAACCTGCTACTATCCCACCAGTATCTTTGAAGGTTTCTGGTTTAACTGGTCTGAAAGAATTAGATTTGTCAGGT
TTTGACCGTGAAACCTTGGCTGGTCTTGATGCCGCTACTCTAACGTCTTTAGAAAAAGTTGATATTTCTGGCAACAAA
CTTGATTTGGCTCCAGGAACAGAAAATCGACAAATTTTTGATACTATGCTATCAACTATCAGCAATCATGTTGGAAGC
AATGAACAAACAGTGAAATTTGACAAGCAAAAACCAACTGGGCATTACCCAGATACCTATGGGAAAACTAGTCTGCGC
TTACCAGTGGCAAATGAAAAAGTTGATTTGCAAAGCCAGCTTTTGTTTGGGACTGTGACAAATCAAGGAACCCTAATC
AATAGCGAAGCAGACTATAAGGCTTACCAAAATCATAAAATTGCTGGACGTAGCTTTGTTGATTCAAACTATCATTAC
AATAACTTTAAAGTTTCTTATGAGAACTATACCGTTAAAGTAACTGATTCCACATTGGGAACCACTACTGACAAAACG
CTAGCAACTGATAAAGAAGAGACCTATAAGGTTGACTTCTTTAGCCCAGCAGATAAGACAAAAGCTGTTCATACTGCT
AAAGTGATTGTTGGTGACGAAAAAACCATGATGGTTAATTTGGCAGAAGGCGCAACAGTTATTGGAGGAAGTGCTGAT
CCTGTAAATGCAAGAAAGGTATTTGATGGGCAACTGGGCAGTGAGACTGATAATATCTCTTTAGGATGGGATTCTAAG
CAAAGTATTATATTTAAATTGAAAGAAGATGGATTAATAAAGCATTGGCGTTTCTTCAATGATTCAGCCCGAAATCCT
GAGACAACCAATAAACCTATTCAGGAAGCAAGTCTACAAATTTTTAATATCAAAGATTATAATCTAGATAATTTGTTG
GAAAATCCCAATAAATTTGATGATGAAAAATATTGGATTACTGTAGATACTTACAGTGCACAAGGAGAGAGAGCTACT
GCATTCAGTAATACATTAAATAATATTACTAGTAAATATTGGCGAGTTGTCTTTGATACTAAAGGAGATAGATATAGT
TCGCCAGTAGTCCCTGAACTCCAAATTTTAGGTTATCCGTTACCTAACGCCGACACTATCATGAAAACAGTAACTACT
GCTAAAGAGTTATCTCAACAAAAAGATAAGTTTTCTCAAAAGATGCTTGATGAGTTAAAAATAAAAGAGATGGCTTTA
GAAACTTCTTTGAACAGTAAGATTTTTGATGTAACTGCTATTAATGCTAATGCTGGAGTTTTGAAAGATTGTATTGAG
AAAAGGCAGCTGCTAAAAAAATAA

[Fig.12]

## Full length nucleotide sequence of pRSFDuet-1-EndoS_D233Q(SEQ ID NO:19)

GGGGAATTGTGAGCGGATAACAATTCCCCTGTAGAAATAATTTTGTTTAACTTTAATAAGGAGATATACCATGGGCAGCAGCCATCACCATCATCACCACG
ATAAACATTTGTTGGTAAAAAGAACACTAGGGTGTGTTTGTGCTGCAACGTTGATGGGAGCTGCCTTAGCGACCCACCATGATTCACTCAATACTGTAAAA
GCGGAGGAGAAGACTGTTCAGGTTCAGAAAGGATTACCTTCTATCGATAGCTTGCATTATCTGTCAGAGAATAGCAAAAAAGAATTTAAAGAAGAACTCTC
AAAAGCGGGGCAAGAATCTCAAAAGGTCAAAGAGATATTAGCAAAAGCTCAGCAGGCAGATAAACAAGCTCAAGAACTTGCCAAAATGAAAATTCCTGAGA
AAATACCGATGAAACCGTTACATGGTCCTCTCTACGGTGGTTACTTTAGAACTTGGCATGACAAAACATCAGATCCAACAGAAAAAGACAAAGTTAACTCG
ATGGGAGAGCTTCCTAAAGAAGTAGATCTAGCCTTTATTTTCCACGATTGGACAAAAGATTATAGCCTTTTTTGGAAAGAATTGGCCACCAAACATGTGCC
AAAGTTAAACAAGCAAGGGACACGTGTCATTCGTACCATTCCATGGCGTTTCCTAGCTGGGGGTGATAACAGTGGTATTGCAGAAGATACCAGTAAATACC
CAAATACACCAGAGGGAAATAAAGCTTTAGCCAAAGCTATTGTTGATGAATATGTTTATAAATACAACCTTGATGGCTTAGATGTGCAAGTTGAACATGAT
AGTATTCCAAAAGTTGACAAAAAAGAAGATACAGCAGGCGTAGAACGCTCTATTCAAGTGTTTGAAGAAATTGGGAAATTAATTGGACCAAAAGGTGTTGA
TAAATCGCGGTTATTTATTATGGATAGCACCTACATGGCTGATAAAAACCCATTGATTGAGCGAGGAGCTCCTTATATTAATTTATTACTGGTACAGGTCT
ATGGTTCACAAGGAGAGAAAGGTGGTTGGGAGCCTGTTTCTAATCGACCTGAAAAAACAATGGAAGAACGATGGCAAGGTTATAGCAAGTATATTCGTCCT
GAACAATACATGATTGGTTTTTCTTTCTATGAGGAAAATGCTCAAGAAGGGAATCTTTGGTATGATATTAATTCTCGCAAGGACGAGGACAAAGCAAATGG
AATTAACACTGACATAACTGGAACGCGTGCCGAACGGTATGCAAGGTGGCAACCTAAGACAGGTGGGGTTAAGGGAGGTATCTTCTCCTACGCTATTGACC
GAGATGGTGTAGCTCATCAACCTAAAAAATATGCTAAACAGAAAGAGTTTAAGGACGCAACTGATAACATCTTCCACTCAGATTATAGTGTCTCCAAGGCA
TTAAAGACAGTTATGCTAAAAGATAAGTCGTATGATCTGATTGATGAGAAAGATTTCCCAGATAAGGCTTTGCGAGAAGCTGTGATGGCGCAGGTTGGAAC
CAGAAAAGGTGATTTGGAACGTTTCAATGGCACATTACGATTGGATAATCCAGCGATTCAAAGTTTAGAAGGTCTAAATAAATTTAAAAAATTAGCTCAAT
TAGACTTGATTGGCTTATCTCGCATTACAAAGCTCGACCGTTCTGTTTTACCCGCTAATATGAAGCCAGGCAAAGATACCTTGGAAACAGTTCTTGAAACC
TATAAAAAGGATAACAAAGAAGAACCTGCTACTATCCCACCAGTATCTTTGAAGGTTTCTGGTTTAACTGGTCTGAAAGAATTAGATTTGTCAGGTTTTGA
CCGTGAAACCTTGGCTGGTCTTGATGCCGCTACTCTAACGTCTTTAGAAAAAGTTGATATTTCTGGCAACAAACTTGATTTGGCTCCAGGAACAGAAAATC
GACAAATTTTTGATACTATGCTATCAACTATCAGCAATCATGTTGGAAGCAATGAACAAACAGTGAAATTTGACAAGCAAAAACCAACTGGGCATTACCCA
GATACCTATGGGAAAACTAGTCTGCGCTTACCAGTGGCAAATGAAAAAGTTGATTTGCAAAGCCAGCTTTTGTTTGGGACTGTGACAAATCAAGGAACCCT
AATCAATAGCGAAGCAGACTATAAGGCTTACCAAAATCATAAAATTGCTGGACGTAGCTTTGTTGATTCAAACTATCATTACAATAACTTTAAAGTTTCTT
ATGAGAACTATACCGTTAAAGTAACTGATTCCACATTGGGAACCACTACTGACAAAACGCTAGCAACTGATAAAGAAGAGACCTATAAGGTTGACTTCTTT
AGCCCAGCAGATAAGACAAAAGCTGTTCATACTGCTAAAGTGATTGTTGGTGACGAAAAAACCATGATGGTTAATTTGGCAGAAGGCGCAACAGTTATTGG
AGGAAGTGCTGATCCTGTAAATGCAAGAAAGGTATTTGATGGGCAACTGGGCAGTGAGACTGATAATATCTCTTTAGGATGGGATTCTAAGCAAAGTATTA
TATTTAAATTGAAAGAAGATGGATTAATAAAGCATTGGCGTTTCTTCAATGATTCAGCCCGAAATCCTGAGACAACCAATAAACCTATTCAGGAAGCAAGT
CTACAAATTTTTAATATCAAAGATTATAATCTAGATAATTTGTTGGAAAATCCCAATAAATTTGATGATGAAAAATATTGGATTACTGTAGATACTTACAG
TGCACAAGGAGAGAGAGCTACTGCATTCAGTAATACATTAAATAATATTACTAGTAAATATTGGCGAGTTGTCTTTGATACTAAAGGAGATAGATATAGTT
CGCCAGTAGTCCCTGAACTCCAAATTTTAGGTTATCCGTTACCTAACGCCGACACTATCATGAAAACAGTAACTACTGCTAAAGAGTTATCTCAACAAAAA
GATAAGTTTTCTCAAAAGATGCTTGATGAGTTAAAAATAAAAGAGATGGCTTTAGAAACTTCTTTGAACAGTAAGATTTTTGATGTAACTGCTATTAATGC
TAATGCTGGAGTTTTGAAAGATTGTATTGAGAAAAGGCAGCTGCTAAAAAAATAAGGATCCGAATTCGAGCTCGGCGCGCCTGCAGGTCGACAAGCTTGCG
GCCGCATAATGCTTAAGTCGAACAGAAAGTAATCGTATTGTACACGGCCGCATAATCGAAATTAATACGACTCACTATAGGGGAATTGTGAGCGGATAACA
ATTCCCCATCTTAGTATATTAGTTAAGTATAAGAAGGAGATATACATATGGCAGATCTCAATTGGATATCGGCCGGCCACGCGATCGCTGACGTCGGTACC
CTCGAGTCTGGTAAAGAAACCGCTGCTGCGAAATTTGAACGCCAGCACATGGACTCGTCTACTAGCGCAGCTTAATTAACCTAGGCTGCTGCCACCGCTGA
GCAATAACTAGCATAACCCCTTGGGGCCTCTAAACGGGTCTTGAGGGGTTTTTTGCTGAAACCTCAGGCATTTGAGAAGCACACGGTCACACTGCTTCCGG
TAGTCAATAAACCGGTAAACCAGCAATAGACATAAGCGGCTATTTAACGACCCTGCCCTGAACCGACGACAAGCTGACGACCGGGTCTCCGCAAGTGGCAC
TTTTCGGGGAAATGTGCGCGGAACCCCTATTTGTTTATTTTTCTAAATACATTCAAATATGTATCCGCTCATGAATTAATTCTTAGAAAAACTCATCGAGC
ATCAAATGAAACTGCAATTTATTCATATCAGGATTATCAATACCATATTTTTGAAAAAGCCGTTTCTGTAATGAAGGAGAAAACTCACCGAGGCAGTTCCA
TAGGATGGCAAGATCCTGGTATCGGTCTGCGATTCCGACTCGTCCAACATCAATACAACCTATTAATTTCCCCTCGTCAAAAATAAGGTTATCAAGTGAGA
AATCACCATGAGTGACGACTGAATCCGGTGAGAATGGCAAAAGTTTATGCATTTCTTTCCAGACTTGTTCAACAGGCCAGCCATTACGCTCGTCATCAAAA
TCACTCGCATCAACCAAACCGTTATTCATTCGTGATTGCGCCTGAGCGAGACGAAATACGCGGTCGCTGTTAAAAGGACAATTACAAACAGGAATCGAATG
CAACCGGCGCAGGAACACTGCCAGCGCATCAACAATATTTTCACCTGAATCAGGATATTCTTCTAATACCTGGAATGCTGTTTTCCCGGGGATCGCAGTGG
TGAGTAACCATGCATCATCAGGAGTACGGATAAAATGCTTGATGGTCGGAAGAGGCATAAATTCCGTCAGCCAGTTTAGTCTGACCATCTCATCTGTAACA
TCATTGGCAACGCTACCTTTGCCATGTTTCAGAAACAACTCTGGCGCATCGGGCTTCCCATACAATCGATAGATTGTCGCACCTGATTGCCCGACATTATC
GCGAGCCCATTTATACCCATATAAATCAGCATCCATGTTGGAATTTAATCGCGGCCTAGAGCAAGACGTTTCCCGTTGAATATGGCTCATACTCTTCCTTT
TTCAATATTATTGAAGCATTTATCAGGGTTATTGTCTCATGAGCGGATACATATTTGAATGTATTTAGAAAAATAAACAAATAGGCATGCAGCGCTCTTCC
GCTTCCTCGCTCACTGACTCGCTACGCTCGGTCGTTCGACTGCGGCGAGCGGTGTCAGCTCACTCAAAAGCGGTAATACGGTTATCCACAGAATCAGGGGA
TAAAGCCGGAAAGAACATGTGAGCAAAAAGCAAAGCACCGGAAGAAGCCAACGCCGCAGGCGTTTTTCCATAGGCTCCGCCCCCCTGACGAGCATCACAAA
AATCGACGCTCAAGCCAGAGGTGGCGAAACCCGACAGGACTATAAAGATACCAGGCGTTTCCCCCTGGAAGCTCCCTCGTGCGCTCTCCTGTTCCGACCCT
GCCGCTTACCGGATACCTGTCCGCCTTTCTCCCTTCGGGAAGCGTGGCGCTTTCTCATAGCTCACGCTGTTGGTATCTCAGTTCGGTGTAGGTCGTTCGCT
CCAAGCTGGGCTGTGTGCACGAACCCCCCGTTCAGCCCGACCGCTGCGCCTTATCCGGTAACTATCGTCTTGAGTCCAACCCGGTAAGACACGACTTATCG
CCACTGGCAGCAGCCATTGGTAACTGATTTAGAGGACTTTGTCTTGAAGTTATGCACCTGTTAAGGCTAAACTGAAAGAACAGATTTTGGTGAGTGCGGTC
CTCCAACCCACTTACCTTGGTTCAAAGAGTTGGTAGCTCAGCGAACCTTGAGAAAACCACCGTTGGTAGCGGTGGTTTTTCTTTATTTATGAGATGATGAA
TCAATCGGTCTATCAAGTCAACGAACAGCTATTCCGTTACTCTAGATTTCAGTGCAATTTATCTCTTCAAATGTAGCACCTGAAGTCAGCCCCATACGATA
TAAGTTGTAATTCTCATGTTAGTCATGCCCCGCGCCCACCGGAAGGAGCTGACTGGGTTGAAGGCTCTCAAGGGCATCGGTCGAGATCCCGGTGCCTAATG
AGTGAGCTAACTTACATTAATTGCGTTGCGCTCACTGCCCGCTTTCCAGTCGGGAAACCTGTCGTGCCAGCTGCATTAATGAATCGGCCAACGCGCGGGGA
GAGGCGGTTTGCGTATTGGGCGCCAGGGTGGTTTTTCTTTTCACCAGTGAGACGGGCAACAGCTGATTGCCCTTCACCGCCTGGCCCTGAGAGAGTTGCAG
CAAGCGGTCCACGCTGGTTTGCCCCAGCAGGCGAAAATCCTGTTTGATGGTGGTTAACGGCGGGATATAACATGAGCTGTCTTCGGTATCGTCGTATCCCA
CTACCGAGATGTCCGCACCAACGCGCAGCCCGGACTCGGTAATGGCGCGCATTGCGCCCAGCGCCATCTGATCGTTGGCAACCAGCATCGCAGTGGGAACG
ATGCCCTCATTCAGCATTTGCATGGTTTGTTGAAAACCGGACATGGCACTCCAGTCGCCTTCCCGTTCCGCTATCGGCTGAATTTGATTGCGAGTGAGATA
TTTATGCCAGCCAGCCAGACGCAGACGCGCCGAGACAGAACTTAATGGGCCCGCTAACAGCGCGATTTGCTGGTGACCCAATGCGACCAGATGCTCCACGC
CCAGTCGCGTACCGTCTTCATGGGAGAAAATAATACTGTTGATGGGTGTCTGGTCAGAGACATCAAGAAATAACGCCGGAACATTAGTGCAGGCAGCTTCC
ACAGCAATGGCATCCTGGTCATCCAGCGGATAGTTAATGATCAGCCCACTGACGCGTTGCGCGAGAAGATTGTGCACCGCCGCTTTACAGGCTTCGACGCC
GCTTCGTTCTACCATCGACACCACCACGCTGGCACCCAGTTGATCGGCGCGAGATTTAATCGCCGCGACAATTTGCGACGGCGCGTGCAGGGCCAGACTGG
AGGTGGCAACGCCAATCAGCAACGACTGTTTGCCCGCCAGTTGTTGTGCCACGCGGTTGGGAATGTAATTCAGCTCCGCCATCGCCGCTTCCACTTTTTCC
CGCGTTTTCGCAGAAACGTGGCTGGCCTGGTTCACCACGCGGGAAACGGTCTGATAAGAGACACCGGCATACTCTGCGACATCGTATAACGTTACTGGTTT
CACATTCACCACCCTGAATTGACTCTCTTCCGGGCGCTATCATGCCATACCGCGAAAGGTTTTGCGCCATTCGATGGTGTCCGGGATCTCGACGCTCTCCC
TTATGCGACTCCTGCATTAGGAAATTAATACGACTCACTATA

[Fig.13]

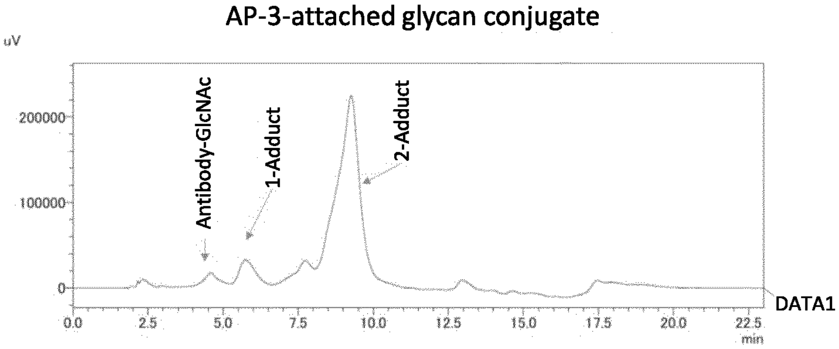


[Fig.14]

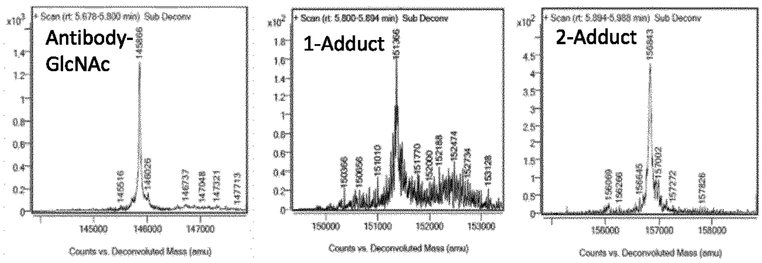


・(1-Adduct)
- (Antibody-GlcNAc) =
5,489 : Caluculated (m/z)
5,500 : Deconvolution data (m/z)

・(2-Adduct)
- (Antibody-GlcNAc) =
10,978 : Caluculated (m/z)
10,977 : Deconvolution data (m/z)

[Fig.15]

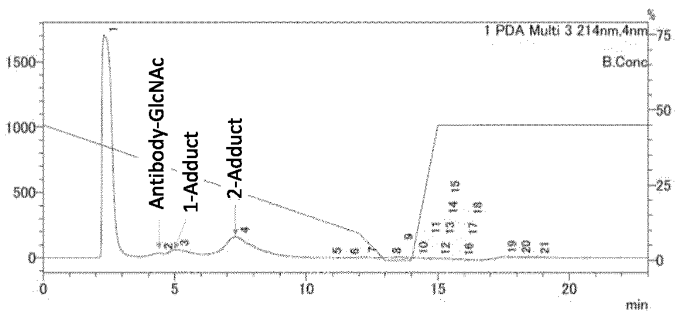


[Fig.16]

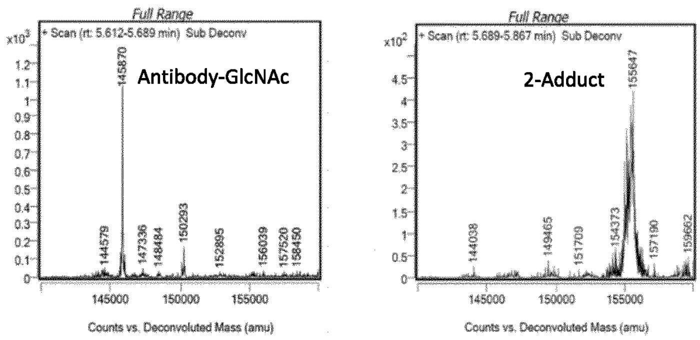


・(2-Adduct)- (Antibody-GlcNAc) =
9,774 : Caluculated (m/z)
9,777 : Deconvolution data (m/z)

[Fig.17]

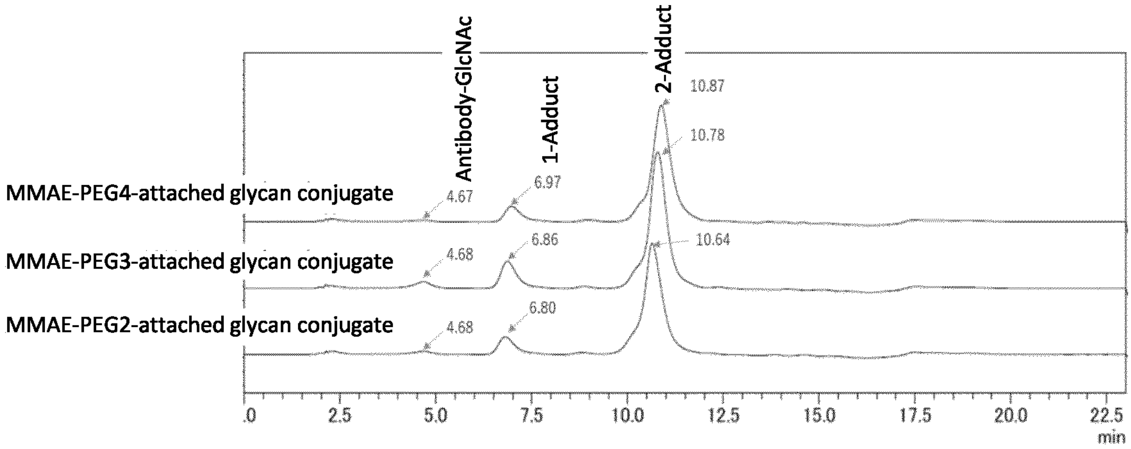


[Fig.18]

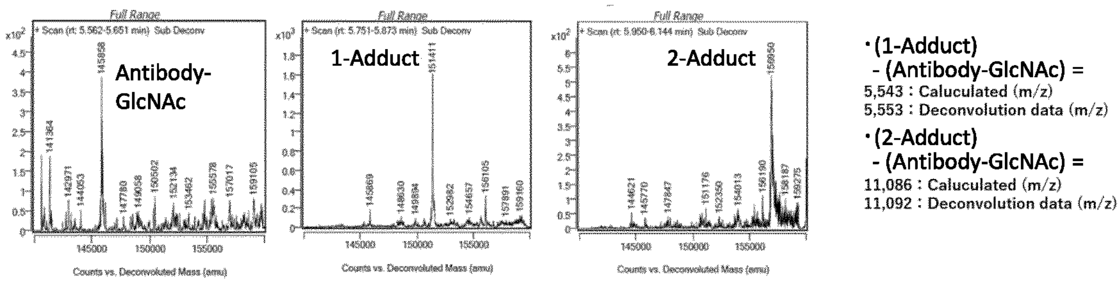


[Fig.19]

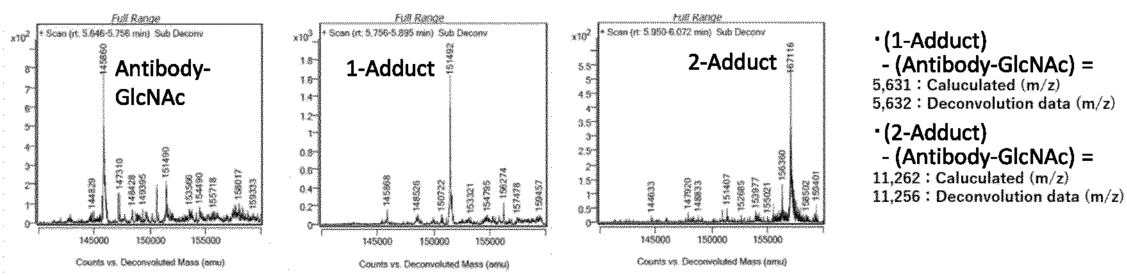

[Fig.20]

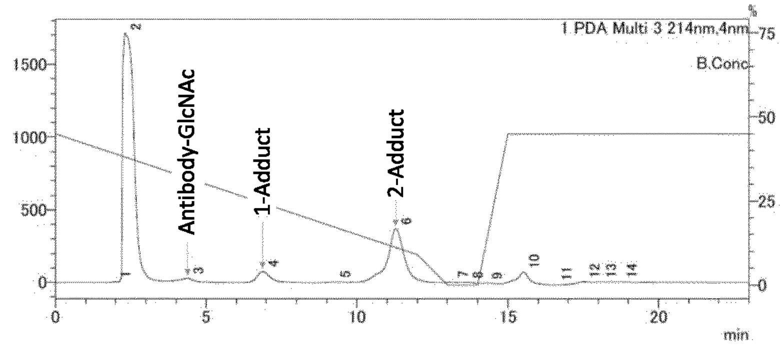


[Fig.21]

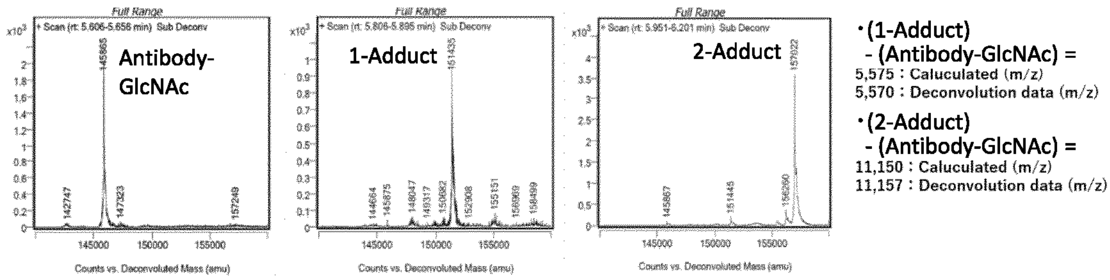


[Fig.22]

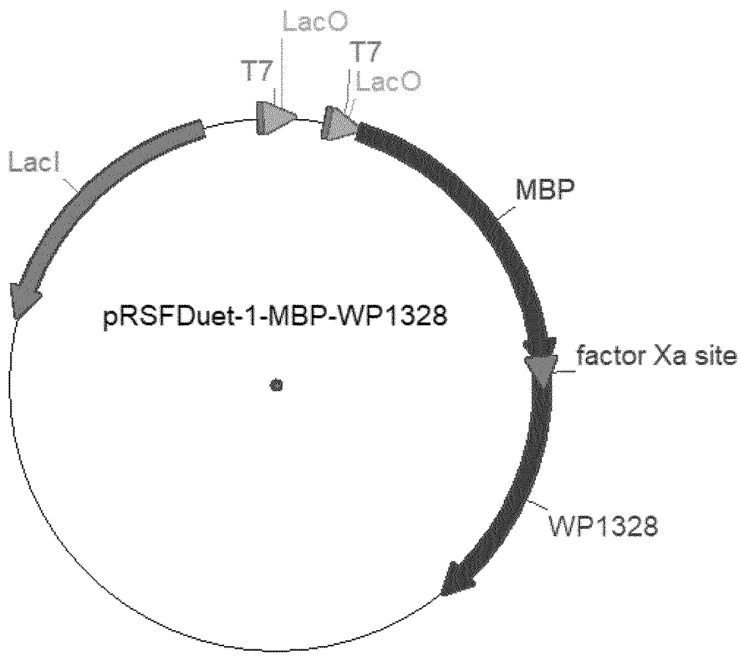

[Fig.23]

## Nucleotide sequence of WP1328 (SEQ ID NO:20)

ATGAAAAAAATTTTTCTGGCCCAGCTGAGCGTGCTGTTTCTGCTGATTCTGGGTGCGTGCAGCAAAA
TGAGCGAAGATCTGGCCCCGGAGACCAGCGATAAAATTACGGCCACCAGTAGTCGCGCGCTGGCGGG
CAACAATGGCGTGTGCATCGCGTACTACATCACCGACGGCCGCAATCCGAGCTTCAAGCTGAAGGAT
ATCCCGGATGGTGTTGACATGGTGATTCTGTTCGGTCTGAAGTACTGGAGTCTGCAAGATACCACCA
AACTGAAACCGGGCACCGATATGATGAGCAGCTTCACGAGCTACAAAGATCTGGACAACCAGATCCG
TAGTCTGCAGAGCCGCGGCATCAAAGTGCTCCAGAACATCGACGACGACGTTAGCTGGCAGAGCAGC
AAGCCGGGCGGCTTTGCGAGTGCCGCCGCGTATGGTGACGCCATCAAGAGCATCGTGATCGATAAGT
GGAAGCTGGACGGCATTAGTCTGCAAGTTGAACACAGCGGCGCGAAACCAAATCCGATCCCGACCTT
TCCGGGTTATGCGGCCACCGGCTATAATGGCTGGTACAGCGGTAGCATGGCCGCGACCCCAGAATTT
CTGAACGTGATCCGCGAGCTCACGAAATACTTCGGTACCACCGCCCCGAACAATAAACAGCTGCAGA
TCGCCAGCGGCATCGATGTGTACAGCTGGAATAAAATTATGGAAAACTTCCGCACCAACTTCAACTA
CATCCAGCTCCAGAGCTACGGTGCGAACGTTAGTCGCACGACGCTGATGATGAACTACGCCACCGGC
ACGAACAAAATCCCGGCGAACAAGATGGTGTTCGGCGCCTACGCGGAAGGTGGTACCAACCAAGCCA
ACGACGTGCTGGTTGCGAAATGGGTTCCGAGCCAAGGCGCCAAAGGCGGCATGATGATTTACACCTA
CAACAGCAACGTGAACTATGCCAACGCCGTTAAGAACGCGGTGAAA

## Amino acid sequence of WP1328 (SEQ ID NO:24)

MKKIFLAQLSVLFLLILGACSKMSEDLAPETSDKITATSSRALAGNNGVC
IAYYITDGRNPSFKLKDIPDGVDMVILFGLKYWSLQDTTKLKPGTDMMSS
FTSYKDLDNQIRSLQSRGIKVLQNIDDDVSWQSSKPGGFASAAAYGDAIK
SIVIDKWKLDGISLQVEHSGAKPNPIPTFPGYAATGYNGWYSGSMAATPE
FLNVIRELTKYFGTTAPNNKQLQIASGIDVYSWNKIMENFRTNFNYIQLQ
SYGANVSRTTLMMNYATGTNKIPANKMVFGAYAEGGTNQANDVLVAKWVP
SQGAKGGMMIYTYNSNVNYANAVKNAVK

[Fig.24]

## Full length nucleotide sequence of pRSFDuet-1-WP1328 (SEQ ID NO:23)

GGGGAATTGTGAGCGGATAACAATTCCCCTGTAGAAATAATTTTGTTTAACTTTAATAAGGAGATATACCATGGGCAGCAGCCATCAC
CATCATCACCACAGCCAGGATCCGAATTCGAGCTCGGCGCGCCTGCAGGTCGACAAGCTTGCGGCCGCATAATGCTTAAGTCGAACAG
AAAGTAATCGTATTGTACACGGCCGCATAATCGAAATTAATACGACTCACTATAGGGGAATTGTGAGCGGATAACAATTCCCCATCTT
AGTATATTAGTTAAGTATAAGAAGGAGATATACATATGATGAAAACTGAAGAAGGTAAACTGGTAATCTGGATTAACGGCGATAAAGG
CTATAACGGTTTGGCTGAAGTCGGTAAGAAATTCGAGAAAGATACCGGAATTAAAGTCACCGTTGAGCATCCGGATAAACTGGAAGAG
AAATTCCCACAGGTTGCGGCAACTGGCGATGGCCCTGACATTATCTTCTGGGCACACGACCGCTTTGGTGGCTACGCTCAATCTGGCC
TGTTGGCTGAAATCACCCCGGACAAAGCGTTCCAGGACAAGCTGTATCCGTTTACCTGGGATGCCGTACGTTACAACGGCAAGCTGAT
TGCTTACCCGATCGCTGTTGAAGCGTTATCGCTGATTTATAACAAAGATCTGCTGCCGAACCCGCCAAAAACCTGGGAAGAGATCCCG
GCGCTGGATAAAGAACTGAAAGCGAAAGGTAAGAGCGCGCTGATGTTCAACCTGCAAGAACCGTACTTCACCTGGCCGCTGATTGCTG
CTGACGGGGGTTATGCGTTCAAGTATGAAAACGGCAAGTACGACATTAAAGACGTGGGCGTGGATAACGCTGGCGCGAAAGCGGGTCT
GACCTTCCTGGTTGACCTGATTAAAAACAAACACATGAATGCAGACACCGATTACTCCATCGCAGAAGCTGCCTTTAATAAAGGCGAA
ACAGCGATGACCATCAACGGCCCGTGGGCATGGTCCAACATCGACACCAGCAAAGTGAATTATGGTGTAACGGTACTGCCGACCTTCA
AGGGTCAACCATCCAAACCGTTCGTTGGCGTGCTGAGCGCAGGTATTAACGCCGCCAGTCCGAACAAAGAGCTGGCAAAAGAGTTCCT
CGAAAACTATCTGCTGACTGATGAAGGTCTGGAAGCGGTTAATAAAGACAAACCGCTGGGTGCCGTAGCGCTGAAGTCTTACGAGGAA
GAGTTGGCGAAAGATCCACGTATTGCCGCCACTATGGAAAACGCCCAGAAAGGTGAAATCATGCCGAACATCCCGCAGATGTCCGCTT
TCTGGTATGCCGTGCGTACTGCGGTGATCAACGCCGCCAGCGGTCGTCAGACTGTCGATGAAGCCCTGAAAGACGCGCAGACTATCGA
GGGAAGGAGTCGCGCGCTGGCGGGCAACAATGGCGTGTGCATCGCGTACTACATCACCGACGGCCGCAATCCGAGCTTCAAGCTGAAG
GATATCCCGGATGGTGTTGACATGGTGATTCTGTTCGGTCTGAAGTACTGGAGTCTGCAAGATACCACCAAACTGAAACCGGGCACCG
ATATGATGAGCAGCTTCACGAGCTACAAAGATCTGGACAACCAGATCCGTAGTCTGCAGAGCCGCGGCATCAAAGTGCTCCAGAACAT
CGACGACGACGTTAGCTGGCAGAGCAGCAAGCCGGGCGGCTTTGCGAGTGCCGCCGCGTATGGTGACGCCATCAAGAGCATCGTGATC
GATAAGTGGAAGCTGGACGGCATTAGTCTGCAAGTTGAACACAGCGGCGCGAAACCAAATCCGATCCCGACCTTTCCGGGTTATGCGG
CCACCGGCTATAATGGCTGGTACAGCGGTAGCATGGCCGCGACCCCAGAATTTCTGAACGTGATCCGCGAGCTCACGAAATACTTCGG
TACCACCGCCCCGAACAATAAACAGCTGCAGATCGCCAGCGGCATCGATGTGTACAGCTGGAATAAAATTATGGAAAACTTCCGCACC
AACTTCAACTACATCCAGCTCCAGAGCTACGGTGCGAACGTTAGTCGCACGACGCTGATGATGAACTACGCCACCGGCACGAACAAAA
TCCCGGCGAACAAGATGGTGTTCGGCGCCTACGCGGAAGGTGGTACCAACCAAGCCAACGACGTGCTGGTTGCGAAATGGGTTCCGAG
CCAAGGCGCCAAAGGCGGCATGATGATTTACACCTACAACAGCAACGTGAACTATGCCAACGCCGTTAAGAACGCGGTGAAATGAAGA
TCTCAATTGGATATCGGCCGGCCACGCGATCGCTGACGTCGGTACCCTCGAGTCTGGTAAAGAAACCGCTGCTGCGAAATTTGAACGC
CAGCACATGGACTCGTCTACTAGCGCAGCTTAATTAACCTAGGCTGCTGCCACCGCTGAGCAATAACTAGCATAACCCCTTGGGGCCT
CTAAACGGGTCTTGAGGGGTTTTTTGCTGAAACCTCAGGCATTTGAGAAGCACACGGTCACACTGCTTCCGGTAGTCAATAAACCGGT
AAACCAGCAATAGACATAAGCGGCTATTTAACGACCCTGCCCTGAACCGACGACAAGCTGACGACCGGGTCTCCGCAAGTGGCACTTT
TCGGGGAAATGTGCGCGGAACCCCTATTTGTTTATTTTTCTAAATACATTCAAATATGTATCCGCTCATGAATTAATTCTTAGAAAAA
CTCATCGAGCATCAAATGAAACTGCAATTTATTCATATCAGGATTATCAATACCATATTTTTGAAAAAGCCGTTTCTGTAATGAAGGA
GAAAACTCACCGAGGCAGTTCCATAGGATGGCAAGATCCTGGTATCGGTCTGCGATTCCGACTCGTCCAACATCAATACAACCTATTA
ATTTCCCCTCGTCAAAAATAAGGTTATCAAGTGAGAAATCACCATGAGTGACGACTGAATCCGGTGAGAATGGCAAAAGTTTATGCAT
TTCTTTCCAGACTTGTTCAACAGGCCAGCCATTACGCTCGTCATCAAAATCACTCGCATCAACCAAACCGTTATTCATTCGTGATTGC
GCCTGAGCGAGACGAAATACGCGGTCGCTGTTAAAAGGACAATTACAAACAGGAATCGAATGCAACCGGCGCAGGAACACTGCCAGCG
CATCAACAATATTTTCACCTGAATCAGGATATTCTTCTAATACCTGGAATGCTGTTTTCCCGGGGATCGCAGTGGTGAGTAACCATGC
ATCATCAGGAGTACGGATAAAATGCTTGATGGTCGGAAGAGGCATAAATTCCGTCAGCCAGTTTAGTCTGACCATCTCATCTGTAACA
TCATTGGCAACGCTACCTTTGCCATGTTTCAGAAACAACTCTGGCGCATCGGGCTTCCCATACAATCGATAGATTGTCGCACCTGATT
GCCCGACATTATCGCGAGCCCATTTATACCCATATAAATCAGCATCCATGTTGGAATTTAATCGCGGCCTAGAGCAAGACGTTTCCCG
TTGAATATGGCTCATACTCTTCCTTTTTCAATATTATTGAAGCATTTATCAGGGTTATTGTCTCATGAGCGGATACATATTTGAATGT
ATTTAGAAAAATAAACAAATAGGCATGCAGCGCTCTTCCGCTTCCTCGCTCACTGACTCGCTACGCTCGGTCGTTCGACTGCGGCGAG
CGGTGTCAGCTCACTCAAAAGCGGTAATACGGTTATCCACAGAATCAGGGGATAAAGCCGGAAAGAACATGTGAGCAAAAAGCAAAGC
ACCGGAAGAAGCCAACGCCGCAGGCGTTTTTCCATAGGCTCCGCCCCCCTGACGAGCATCACAAAAATCGACGCTCAAGCCAGAGGTG
GCGAAACCCGACAGGACTATAAAGATACCAGGCGTTTCCCCCTGGAAGCTCCCTCGTGCGCTCTCCTGTTCCGACCCTGCCGCTTACC
GGATACCTGTCCGCCTTTCTCCCTTCGGGAAGCGTGGCGCTTTCTCATAGCTCACGCTGTTGGTATCTCAGTTCGGTGTAGGTCGTTC
GCTCCAAGCTGGGCTGTGTGCACGAACCCCCCGTTCAGCCCGACCGCTGCGCCTTATCCGGTAACTATCGTCTTGAGTCCAACCCGGT
AAGACACGACTTATCGCCACTGGCAGCAGCCATTGGTAACTGATTTAGAGGACTTTGTCTTGAAGTTATGCACCTGTTAAGGCTAAAC
TGAAAGAACAGATTTTGGTGAGTGCGGTCCTCCAACCCACTTACCTTGGTTCAAAGAGTTGGTAGCTCAGCGAACCTTGAGAAAACCA
CCGTTGGTAGCGGTGGTTTTTCTTTATTTATGAGATGATGAATCAATCGGTCTATCAAGTCAACGAACAGCTATTCCGTTACTCTAGA
TTTCAGTGCAATTTATCTCTTCAAATGTAGCACCTGAAGTCAGCCCCATACGATATAAGTTGTAATTCTCATGTTAGTCATGCCCCGC
GCCCACCGGAAGGAGCTGACTGGGTTGAAGGCTCTCAAGGGCATCGGTCGAGATCCCGGTGCCTAATGAGTGAGCTAACTTACATTAA
TTGCGTTGCGCTCACTGCCCGCTTTCCAGTCGGGAAACCTGTCGTGCCAGCTGCATTAATGAATCGGCCAACGCGCGGGGAGAGGCGG
TTTGCGTATTGGGCGCCAGGGTGGTTTTTCTTTTCACCAGTGAGACGGGCAACAGCTGATTGCCCTTCACCGCCTGGCCCTGAGAGAG
TTGCAGCAAGCGGTCCACGCTGGTTTGCCCCAGCAGGCGAAAATCCTGTTTGATGGTGGTTAACGGCGGGATATAACATGAGCTGTCT
TCGGTATCGTCGTATCCCACTACCGAGATGTCCGCACCAACGCGCAGCCCGGACTCGGTAATGGCGCGCATTGCGCCCAGCGCCATCT
GATCGTTGGCAACCAGCATCGCAGTGGGAACGATGCCCTCATTCAGCATTTGCATGGTTTGTTGAAAACCGGACATGGCACTCCAGTC
GCCTTCCCGTTCCGCTATCGGCTGAATTTGATTGCGAGTGAGATATTTATGCCAGCCAGCCAGACGCAGACGCGCCGAGACAGAACTT
AATGGGCCCGCTAACAGCGCGATTTGCTGGTGACCCAATGCGACCAGATGCTCCACGCCCAGTCGCGTACCGTCTTCATGGGAGAAAA
TAATACTGTTGATGGGTGTCTGGTCAGAGACATCAAGAAATAACGCCGGAACATTAGTGCAGGCAGCTTCCACAGCAATGGCATCCTG
GTCATCCAGCGGATAGTTAATGATCAGCCCACTGACGCGTTGCGCGAGAAGATTGTGCACCGCCGCTTTACAGGCTTCGACGCCGCTT
CGTTCTACCATCGACACCACCACGCTGGCACCCAGTTGATCGGCGCGAGATTTAATCGCCGCGACAATTTGCGACGGCGCGTGCAGGG
CCAGACTGGAGGTGGCAACGCCAATCAGCAACGACTGTTTGCCCGCCAGTTGTTGTGCCACGCGGTTGGGAATGTAATTCAGCTCCGC
CATCGCCGCTTCCACTTTTTCCCGCGTTTTCGCAGAAACGTGGCTGGCCTGGTTCACCACGCGGGAAACGGTCTGATAAGAGACACCG
GCATACTCTGCGACATCGTATAACGTTACTGGTTTCACATTCACCACCCTGAATTGACTCTCTTCCGGGCGCTATCATGCCATACCGC
GAAAGGTTTTGCGCCATTCGATGGTGTCCGGGATCTCGACGCTCTCCCTTATGCGACTCCTGCATTAGGAAATTAATACGACTCACTA
TA

**INTERNATIONAL SEARCH REPORT**

International application No.
**PCT/JP2022/016786**

**A. CLASSIFICATION OF SUBJECT MATTER**

***A61K 47/68***(2017.01)i; ***A61K 39/395***(2006.01)i; ***A61P 35/00***(2006.01)i; ***C12N 9/24***(2006.01)i; ***C12N 15/56***(2006.01)i; ***C12P 19/44***(2006.01)i; *C07K 16/30*(2006.01)n
FI: A61K47/68 ZNA; A61K39/395 C; A61K39/395 L; A61P35/00; C12N9/24; C12N15/56; C12P19/44; C07K16/30

According to International Patent Classification (IPC) or to both national classification and IPC

**B. FIELDS SEARCHED**

Minimum documentation searched (classification system followed by classification symbols)

A61K47/68; A61K39/395; A61P35/00; C12N9/24; C12N15/56; C12P19/44; C07K16/30

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X | WO 2017/010559 A1 (DAIICHI SANKYO COMPANY, LIMITED) 19 January 2017 (2017-01-19) particularly, sequence no. 1, examples | 11 |
| A | | 1-10 |
| Y | GIDDENS, J. P. et al. Endo-F3 Glycosynthase Mutants Enable Chemoenzymatic Synthesis of Core-fucosylated Triantennary Complex Type Glycopeptides and Glycoproteins. J Biol Chem. 2016, 291(17), pages 9356-9370 abstract | 11 |
| A | | 1-10 |
| Y | Accession Number: P36913, entry version 106, UniProtKB/Swiss-Prot [online] (retrieved on 31 May 2022) 02 December 2020, retrieved from the Internet <URL: https://www.uniprot.org/uniprot/P36913.txt?version=106> particularly, sequence | 11 |
| A | | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 May 2022** | **14 June 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/016786**

**C. DOCUMENTS CONSIDERED TO BE RELEVANT**

| Category* | Citation of document, with indication, where appropriate, of the relevant passages | Relevant to claim No. |
|---|---|---|
| Y | Accession Number: A0A1V3U698, entry version 27, UniProtKB/TrEMBL [online] (retrieved on 31 May 2022) 10 February 2021, retrieved from the Internet <URL: https://www.uniprot.org/uniprot/A0A1V3U698.txt?version=27> particularly, sequence | 11 |
| A | | 1-10 |
| A | JP 2019-206491 A (INSTITUTE OF PHYSICAL & CHEMICAL RESEARCH) 05 December 2019 (2019-12-05) entire text | 1-11 |
| A | WO 2020/196475 A1 (DAIICHI SANKYO COMPANY, LIMITED) 01 October 2020 (2020-10-01) entire text | 1-10 |
| A | CN 107778372 A (SHANGHAI INSTITUTE OF MATERIA MEDICA, CHINESE ACADEMY OF SCIENCES) 09 March 2018 (2018-03-09) entire text | 1-11 |
| P, X | JP 2021-145548 A (NOGUCHI INSTITUTE) 27 September 2021 (2021-09-27) particularly, sequence no. 1, examples | 11 |
| P, A | 水野真盛, 他10名, PEG化2糖オキサゾリン誘導体を用いた抗体薬物複合体(ADC)前駆体の合成, 第40回日本糖質学会年会要旨集, 15 October 2021, pages 114, 3B-06, (MIZUNO, Mamoru et al. Synthesis of antibody-drug conjugate (ADC) precursor using PEGylated disaccharide oxazoline derivative. Proceedings of The 40th Annual Meeting of Japanese Society of Carbohydrate Research.) entire text | 1-11 |

Form PCT/ISA/210 (second sheet) (January 2015)

**INTERNATIONAL SEARCH REPORT**

International application No.
**PCT/JP2022/016786**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/016786**

| Patent document cited in search report | Publication date (day/month/year) | Patent family member(s) | Publication date (day/month/year) |
|---|---|---|---|
| WO 2017/010559 A1 | 19 January 2017 | US 2018/0208915 A1<br>particularly, sequence no. 1, examples<br>EP 3323886 A1<br>KR 10-2018-0030536 A<br>CN 108026518 A | |
| JP 2019-206491 A | 05 December 2019 | (Family: none) | |
| WO 2020/196475 A1 | 01 October 2020 | EP 3949987 A1<br>entire text<br>CN 113631190 A<br>KR 10-2021-0141630 A | |
| CN 107778372 A | 09 March 2018 | WO 2018/036403 A1<br>entire text | |
| JP 2021-145548 A | 27 September 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013120066 A1
- WO 2017124084 A1
- US 20120226024 A1
- US 5821337 A
- WO 2004010957 A
- WO 2014145090 A


### Non-patent literature cited in the description


- *Nat. Biotechnol.,* 2005, vol. 23, 1073-1078
- *Cancer Sci.,* 2009, vol. 100, 1566-1572
- *Nat. Biotechnol.,* 2005, vol. 23, 1137-1146
- *Clin. Cancer Res.,* 2001, vol. 7, 1490-1496
- *MAbs,* 2012, vol. 4, 458-465
- *Nat. Biotechnol.,* 2008, vol. 26, 952-932
- *Chem. Soc. Rev.,* 2017, vol. 46, 5128-5146
- *J. Am. Chem. Soc.,* 2012, vol. 134, 12308-12318
- *J. Biol. Chem.,* 2016, vol. 291, 16508-16518
- *J. Biol. Chem.,* 2016, vol. 291, 9356-9370
- *Nat. Protoc.,* 2011, vol. 6, 1183-1191
- *Bioconjug. Chem.,* 2019, vol. 30, 1343-1355
- *Chem, Rev.,* 2013, vol. 113, 4905-4979
- *Method Mol. Biol.,* 2020, vol. 2078, 83-97
- *Chem. Soc. Rev.,* 2019, vol. 48, 4361-4374
- *Protein Cell,* January 2018, vol. 9 (1), 33-46
- *Bioorg. Med. Chem.,* 2012, vol. 20, 571-582
- *J. Control. Release,* 2004, vol. 99, 423-434
- *Bioconjugate Chem.,* 2017, vol. 28, 1906-1915
- **ALTSCHUL et al.** *J. Mol. Biol.,* 1990, vol. 215, 403-410